# EUROPÄISCHE PATENTSCHRIFT

(11) **EP 1 506 289 B1**
(45) Veröffentlichungstag und Bekanntmachung des Hinweises auf die Patenterteilung: **08.08.2012**
(21) Anmeldenummer: 03737958.3
(22) Anmeldetag: 06.05.2003
(51) Int. Cl.: C12N 9/02

(54) **VERFAHREN ZUM ERHÖHEN DES ÖLGEHALTES IN PFLANZEN**
METHODS FOR INCREASING OIL CONTENT IN PLANTS
PROCEDE POUR AUGMENTER LA TENEUR EN HUILE DANS DES VEGETAUX

(30) Priorität: 08.05.2002 DE 10220753; 13.06.2002 DE 10226413
(43) Veröffentlichungstag der Anmeldung: 16.02.2005
(73) Patentinhaber: BASF Plant Science GmbH, 67056 Ludwigshafen (DE)
(72) Erfinder: RENZ, Andreas, 67117 Limburgerhof (DE); BAUER, Jörg, 67063 Ludwigshafen (DE); STITT NIGEL, Marc, 14469 Potsdam (DE); ZRENNER, Rita, Maria, 14476 Golm (DE); GEIGENBERGER, Peter, 14129 Berlin (DE); VIGEOLAS, Helene, 14469 Potsdam (DE)
(74) Vertreter: Bieberbach, Andreas
(86) Internationale Anmeldenummer: PCT/EP2003/004711
(87) Internationale Veröffentlichungsnummer: WO 2003/095655

(56) Entgegenhaltungen:
- WO-A-01/21820
- WO-A-93/07275
- US-A- 6 103 520
- THELEN JAY J ET AL: "Metabolic engineering of fatty acid biosynthesis in plants." METABOLIC ENGINEERING. UNITED STATES JAN 2002, Bd. 4, Nr. 1, Januar 2002 (2002-01), Seiten 12-21, XP002257832 ISSN: 1096-7176

### SEQUENZPROTOKOLL

<110> BASF Plant Science GmbH
<120> Verfahren zum Erhöhen des Ölgehaltes in Pflanzen
<130> NAE 2166/2002
<140>
   <141>
<160> 40
<170> PatentIn Ver. 2.1
<210> 1
   <211> 1176
   <212> DNA
   <213> Saccharomyces cerevisiae
<220>
   <221> CDS
   <222> (1)..(1173)
   <223> coding for G3PDH
<400> 1
   atg tct gct gct gct gat aga tta aac tta act tcc ggc cac ttg aat    48
   Met Ser Ala Ala Ala Asp Arg Leu Asn Leu Thr Ser Gly His Leu Asn
     1               5                  10                  15
   gct ggt aga aag aga agt tcc tct tct gtt tct ttg aag gct gcc gaa    96
   Ala Gly Arg Lys Arg Ser Ser Ser Ser Val Ser Leu Lys Ala Ala Glu
                20                  25                  30
   aag cct ttc aag gtt act gtg att gga tct ggt aac tgg ggt act act   144
   Lys Pro Phe Lys Val Thr Val Ile Gly Ser Gly Asn Trp Gly Thr Thr
            35                  40                  45
   att gcc aag gtg gtt gcc gaa aat tgt aag gga tac cca gaa gtt ttc   192
   Ile Ala Lys Val Val Ala Glu Asn Cys Lys Gly Tyr Pro Glu Val Phe
        50                  55                  60
   gct cca ata gta caa atg tgg gtg ttc gaa gaa gag atc aat ggt gaa   240
   Ala Pro Ile Val Gln Met Trp Val Phe Glu Glu Glu Ile Asn Gly Glu
    65                  70                  75                  80
   aaa ttg act gaa atc ata aat act aga cat caa aac gtg aaa tac ttg   288
   Lys Leu Thr Glu Ile Ile Asn Thr Arg His Gln Asn Val Lys Tyr Leu
                    85                  90                  95
   cct ggc atc act cta ccc gac aat ttg gtt gct aat cca gac ttg att   336
   Pro Gly Ile Thr Leu Pro Asp Asn Leu Val Ala Asn Pro Asp Leu Ile
               100                 105                 110
   gat tca gtc aag gat gtc gac atc atc gtt ttc aac att cca cat caa   384
   Asp Ser Val Lys Asp Val Asp Ile Ile Val Phe Asn Ile Pro His Gln
           115                 120                 125
   ttt ttg ccc cgt atc tgt agc caa ttg aaa ggt cat gtt gat tca cac   432
   Phe Leu Pro Arg Ile Cys Ser Gln Leu Lys Gly His Val Asp Ser His
       130                 135                 140
   gtc aga gct atc tcc tgt cta aag ggt ttt gaa gtt ggt gct aaa ggt   480
   Val Arg Ala Ile Ser Cys Leu Lys Gly Phe Glu Val Gly Ala Lys Gly
   145                 150                 155                 160
   gtc caa ttg cta tcc tct tac atc act gag gaa cta ggt att caa tgt   528
   Val Gln Leu Leu Ser Ser Tyr Ile Thr Glu Glu Leu Gly Ile Gln Cys
                   165                 170                 175
   ggt gct cta tct ggt gct aac att gcc acc gaa gtc gct caa gaa cac     576
   Gly Ala Leu Ser Gly Ala Asn Ile Ala Thr Glu Val Ala Gln Glu His
               180                 185                 190
   tgg tct gaa aca aca gtt gct tac cac att cca aag gat ttc aga ggc     624
   Trp Ser Glu Thr Thr Val Ala Tyr His Ile Pro Lys Asp Phe Arg Gly
           195                 200                 205
   gag ggc aag gac gtc gac cat aag gtt cta aag gcc ttg ttc cac aga     672
   Glu Gly Lys Asp Val Asp His Lys Val Leu Lys Ala Leu Phe His Arg
       210                 215                 220
   cct tac ttc cac gtt agt gtc atc gaa gat gtt gct ggt atc tcc atc     720
   Pro Tyr Phe His Val Ser Val Ile Glu Asp Val Ala Gly Ile Ser Ile
   225                 230                 235                 240
   tgt ggt gct ttg aag aac gtt gtt gcc tta ggt tgt ggt ttc gtc gaa     768
   Cys Gly Ala Leu Lys Asn Val Val Ala Leu Gly Cys Gly Phe Val Glu
                   245                 250                 255
   ggt cta ggc tgg ggt aac aac gct tct gct gcc atc caa aga gtc ggt     816
   Gly Leu Gly Trp Gly Asn Asn Ala Ser Ala Ala Ile Gln Arg Val Gly
               260                 265                 270
   ttg ggt gag atc atc aga ttc ggt caa atg ttt ttc cca gaa tct aga     864
   Leu Gly Glu Ile Ile Arg Phe Gly Gln Met Phe Phe Pro Glu Ser Arg
           275                 280                 285
   gaa gaa aca tac tac caa gag tct gct ggt gtt gct gat ttg atc acc     912
   Glu Glu Thr Tyr Tyr Gln Glu Ser Ala Gly Val Ala Asp Leu Ile Thr
       290                 295                 300
   acc tgc gct ggt ggt aga aac gtc aag gtt gct agg cta atg gct act     960
   Thr Cys Ala Gly Gly Arg Asn Val Lys Val Ala Arg Leu Met Ala Thr
   305                 310                 315                 320
   tct ggt aag gac gcc tgg gaa tgt gaa aag gag ttg ttg aat ggc caa    1008
   Ser Gly Lys Asp Ala Trp Glu Cys Glu Lys Glu Leu Leu Asn Gly Gln
                   325                 330                 335
   tcc gct caa ggt tta att acc tgc aaa gaa gtt cac gaa tgg ttg gaa    1056
   Ser Ala Gln Gly Leu Ile Thr Cys Lys Glu Val His Glu Trp Leu Glu
               340                 345                 350
   aca tgt ggc tct gtc gaa gac ttc cca tta ttt gaa gcc gta tac caa    1104
   Thr Cys Gly Ser Val Glu Asp Phe Pro Leu Phe Glu Ala Val Tyr Gln
           355                 360                 365
   atc gtt tac aac aac tac cca atg aag aac ctg ccg gac atg att gaa    1152
   Ile Val Tyr Asn Asn Tyr Pro Met Lys Asn Leu Pro Asp Met Ile Glu
       370                 375                 380
   gaa tta gat cta cat gaa gat tag                                    1176
   Glu Leu Asp Leu His Glu Asp
   385                 390
<210> 2
   <211> 391
   <212> PRT
   <213> Saccharomyces cerevisiae
<400> 2
   Met Ser Ala Ala Ala Asp Arg Leu Asn Leu Thr Ser Gly His Leu Asn
   1               5                   10                  15
   Ala Gly Arg Lys Arg Ser Ser Ser Ser Val Ser Leu Lys Ala Ala Glu
               20                  25                  30
   Lys Pro Phe Lys Val Thr Val Ile Gly Ser Gly Asn Trp Gly Thr Thr
   35 40 45
   Ile Ala Lys Val Val Ala Glu Asn Cys Lys Gly Tyr Pro Glu Val Phe
   50 55 60
   Ala Pro Ile Val Gln Met Trp Val Phe Glu Glu Glu Ile Asn Gly Glu
   65 70 75 80
   Lys Leu Thr Glu Ile Ile Asn Thr Arg His Gln Asn Val Lys Tyr Leu
   85 90 95
   Pro Gly Ile Thr Leu Pro Asp Asn Leu Val Ala Asn Pro Asp Leu Ile
   100 105 110
   Asp Ser Val Lys Asp Val Asp Ile Ile Val Phe Asn Ile Pro His Gln
   115 120 125
   Phe Leu Pro Arg Ile Cys Ser Gln Leu Lys Gly His Val Asp Ser His
   130 135 140
   Val Arg Ala Ile Ser Cys Leu Lys Gly Phe Glu Val Gly Ala Lys Gly
   145 150 155 160
   Val Gln Leu Leu Ser Ser Tyr Ile Thr Glu Glu Leu Gly Ile Gln Cys
   165 170 175
   Gly Ala Leu Ser Gly Ala Asn Ile Ala Thr Glu Val Ala Gln Glu His
   180 185 190
   Trp Ser Glu Thr Thr Val Ala Tyr His Ile Pro Lys Asp Phe Arg Gly
   195 200 205
   Glu Gly Lys Asp Val Asp His Lys Val Leu Lys Ala Leu Phe His Arg
   210 215 220
   Pro Tyr Phe His Val Ser Val Ile Glu Asp Val Ala Gly Ile Ser Ile
   225 230 235 240
   Cys Gly Ala Leu Lys Asn Val Val Ala Leu Gly Cys Gly Phe Val Glu
   245 250 255
   Gly Leu Gly Trp Gly Asn Asn Ala Ser Ala Ala Ile Gln Arg Val Gly
   260 265 270
   Leu Gly Glu Ile Ile Arg Phe Gly Gln Met Phe Phe Pro Glu Ser Arg
   275 280 285
   Glu Glu Thr Tyr Tyr Gln Glu Ser Ala Gly Val Ala Asp Leu Ile Thr
   290 295 300
   Thr Cys Ala Gly Gly Arg Asn Val Lys Val Ala Arg Leu Met Ala Thr
   305 310 315 320
   Ser Gly Lys Asp Ala Trp Glu Cys Glu Lys Glu Leu Leu Asn Gly Gln
   325 330 335
   Ser Ala Gln Gly Leu Ile Thr Cys Lys Glu Val His Glu Trp Leu Glu
   340 345 350
   Thr Cys Gly Ser Val Glu Asp Phe Pro Leu Phe Glu Ala Val Tyr Gln
   355 360 365
   Ile Val Tyr Asn Asn Tyr Pro Met Lys Asn Leu Pro Asp Met Ile Glu
   370 375 380
   Glu Leu Asp Leu His Glu Asp
   385 390
<210> 3
   <211> 1299
   <212> DNA
   <213> Saccharomyces cerevisiae
<220>
   <221> CDS
   <222> (1)..(1296)
   <223> coding for G3PDH
<220>
   <221> CDS
   <222> (145)..(1296)
   <223> coding for G3PDH (alternative Start codon)
<400> 3
   atg ctt gct gtc aga aga tta aca aga tac aca ttc ctt aag cga acg     48
   Met Leu Ala Val Arg Arg Leu Thr Arg Tyr Thr Phe Leu Lys Arg Thr
    1               5                  10                  15
   cat ccg gtg tta tat act cgt cgt gca tat aaa att ttg cct tca aga     96
   His Pro Val Leu Tyr Thr Arg Arg Ala Tyr Lys Ile Leu Pro Ser Arg
               20                  25                  30
   tct act ttc cta aga aga tca tta tta caa aca caa ctg cac tca aag    144
   Ser Thr Phe Leu Arg Arg Ser Leu Leu Gln Thr Gln Leu His Ser Lys
           35                  40                  45
   atg act gct cat act aat atc aaa cag cac aaa cac tgt cat gag gac    192
   Met Thr Ala His Thr Asn Ile Lys Gln His Lys His Cys His Glu Asp
       50                  55                  60
   cat cct atc aga aga tcg gac tct gcc gtg tca att gta cat ttg aaa    240
   His Pro Ile Arg Arg Ser Asp Ser Ala Val Ser Ile Val His Leu Lys
   65                  70                  75                  80
   cgt gcg ccc ttc aag gtt aca gtg att ggt tct ggt aac tgg ggg acc    288
   Arg Ala Pro Phe Lys Val Thr Val Ile Gly Ser Gly Asn Trp Gly Thr
                   85                  90                  95
   acc atc gcc aaa gtc att gcg gaa aac aca gaa ttg cat tcc cat atc    336
   Thr Ile Ala Lys Val Ile Ala Glu Asn Thr Glu Leu His Ser His Ile
               100                 105                 110
   ttc gag cca gag gtg aga atg tgg gtt ttt gat gaa aag atc ggc gac    384
   Phe Glu Pro Glu Val Arg Met Trp Val Phe Asp Glu Lys Ile Gly Asp
           115                 120                 125
   gaa aat ctg acg gat atc ata aat aca aga cac cag aac gtt aaa tat    432
   Glu Asn Leu Thr Asp Ile Ile Asn Thr Arg His Gln Asn Val Lys Tyr
       130                 135                 140
   cta ccc aat att gac ctg ccc cat aat cta gtg gcc gat cct gat ctt    480
   Leu Pro Asn Ile Asp Leu Pro His Asn Leu Val Ala Asp Pro Asp Leu
   145                 150                 155                 160
   tta cac tcc atc aag ggt gct gac atc ctt gtt ttc aac atc cct cat    528
   Leu His Ser Ile Lys Gly Ala Asp Ile Leu Val Phe Asn Ile Pro His
                   165                 170                 175
   caa ttt tta cca aac ata gtc aaa caa ttg caa ggc cac gtg gcc cct    576
   Gln Phe Leu Pro Asn Ile Val Lys Gln Leu Gln Gly His Val Ala Pro
               180                 185                 190
   cat gta agg gcc atc tcg tgt cta aaa ggg ttc gag ttg ggc tcc aag    624
   His Val Arg Ala Ile Ser Cys Leu Lys Gly Phe Glu Leu Gly Ser Lys
           195                 200                 205
   ggt gtg caa ttg cta tcc tcc tat gtt act gat gag tta gga atc caa     672
   Gly Val Gln Leu Leu Ser Ser Tyr Val Thr Asp Glu Leu Gly Ile Gln
       210                 215                 220
   tgt ggc gca cta tct ggt gca aac ttg gca ccg gaa gtg gcc aag gag     720
   Cys Gly Ala Leu Ser Gly Ala Asn Leu Ala Pro Glu Val Ala Lys Glu
   225                 230                 235                 240
   cat tgg tcc gaa acc acc gtg gct tac caa cta cca aag gat tat caa     768
   His Trp Ser Glu Thr Thr Val Ala Tyr Gln Leu Pro Lys Asp Tyr Gln
                   245                 250                 255
   ggt gat ggc aag gat gta gat cat aag att ttg aaa ttg ctg ttc cac     816
   Gly Asp Gly Lys Asp Val Asp His Lys Ile Leu Lys Leu Leu Phe His
               260                 265                 270
   aga cct tac ttc cac gtc aat gtc atc gat gat gtt gct ggt ata tcc     864
   Arg Pro Tyr Phe His Val Asn Val Ile Asp Asp Val Ala Gly Ile Ser
           275                 280                 285
   att gcc ggt gcc ttg aag aac gtc gtg gca ctt gca tgt ggt ttc gta     912
   Ile Ala Gly Ala Leu Lys Asn Val Val Ala Leu Ala Cys Gly Phe Val
       290                 295                 300
   gaa ggt atg gga tgg ggt aac aat gcc tcc gca gcc att caa agg ctg     960
   Glu Gly Met Gly Trp Gly Asn Asn Ala Ser Ala Ala Ile Gln Arg Leu
   305                 310                 315                 320
   ggt tta ggt gaa att atc aag ttc ggt aga atg ttt ttc cca gaa tcc     1008
   Gly Leu Gly Glu Ile Ile Lys Phe Gly Arg Met Phe Phe Pro Glu Ser
                   325                 330                 335
   aaa gtc gag acc tac tat caa gaa tcc gct ggt gtt gca gat ctg atc     1056
   Lys Val Glu Thr Tyr Tyr Gln Glu Ser Ala Gly Val Ala Asp Leu Ile
               340                 345                 350
   acc acc tgc tca ggc ggt aga aac gtc aag gtt gcc aca tac atg gcc     1104
   Thr Thr Cys Ser Gly Gly Arg Asn Val Lys Val Ala Thr Tyr Met Ala
           355                 360                 365
   aag acc ggt aag tca gcc ttg gaa gca gaa aag gaa ttg ctt aac ggt     1152
   Lys Thr Gly Lys Ser Ala Leu Glu Ala Glu Lys Glu Leu Leu Asn Gly
       370                 375                 380
   caa tcc gcc caa ggg ata atc aca tgc aga gaa gtt cac gag tgg cta     1200
   Gln Ser Ala Gln Gly Ile Ile Thr Cys Arg Glu Val His Glu Trp Leu
   385                 390                 395                 400
   caa aca tgt gag ttg acc caa gaa ttc cca att att cga ggc agt cta     1248
   Gln Thr Cys Glu Leu Thr Gln Glu Phe Pro Ile Ile Arg Gly Ser Leu
                   405                 410                 415
   cca gat agt cta caa caa cgt ccg cat gga aga cct acc gga gat gat     1296
   Pro Asp Ser Leu Gln Gln Arg Pro His Gly Arg Pro Thr Gly Asp Asp
               420                 425                 430
   tga                                                                 1299
<210> 4
   <211> 432
   <212> PRT
   <213> Saccharomyces cerevisiae
<400> 4
   Met Leu Ala Val Arg Arg Leu Thr Arg Tyr Thr Phe Leu Lys Arg Thr
   1               5                   10                  15
   His Pro Val Leu Tyr Thr Arg Arg Ala Tyr Lys Ile Leu Pro Ser Arg
               20                  25                  30
   Ser Thr Phe Leu Arg Arg Ser Leu Leu Gln Thr Gln Leu His Ser Lys
           35                  40                  45
   Met Thr Ala His Thr Asn Ile Lys Gln His Lys His Cys His Glu Asp
       50                  55                  60
   His Pro Ile Arg Arg Ser Asp Ser Ala Val Ser Ile Val His Leu Lys
   65                  70                  75                  80
   Arg Ala Pro Phe Lys Val Thr Val Ile Gly Ser Gly Asn Trp Gly Thr
                   85                  90                  95
   Thr Ile Ala Lys Val Ile Ala Glu Asn Thr Glu Leu His Ser His Ile
               100                 105                 110
   Phe Glu Pro Glu Val Arg Met Trp Val Phe Asp Glu Lys Ile Gly Asp
           115                 120                 125
   Glu Asn Leu Thr Asp Ile Ile Asn Thr Arg His Gln Asn Val Lys Tyr
       130                 135                 140
   Leu Pro Asn Ile Asp Leu Pro His Asn Leu Val Ala Asp Pro Asp Leu
   145                 150                 155                 160
   Leu His Ser Ile Lys Gly Ala Asp Ile Leu Val Phe Asn Ile Pro His
                   165                 170                 175
   Gln Phe Leu Pro Asn Ile Val Lys Gln Leu Gln Gly His Val Ala Pro
               180                 185                 190
   His Val Arg Ala Ile Ser Cys Leu Lys Gly Phe Glu Leu Gly Ser Lys
           195                 200                 205
   Gly Val Gln Leu Leu Ser Ser Tyr Val Thr Asp Glu Leu Gly Ile Gln
       210                 215                 220
   Cys Gly Ala Leu Ser Gly Ala Asn Leu Ala Pro Glu Val Ala Lys Glu
   225                 230                 235                 240
   His Trp Ser Glu Thr Thr Val Ala Tyr Gln Leu Pro Lys Asp Tyr Gln
                   245                 250                 255
   Gly Asp Gly Lys Asp Val Asp His Lys Ile Leu Lys Leu Leu Phe His
               260                 265                 270
   Arg Pro Tyr Phe His Val Asn Val Ile Asp Asp Val Ala Gly Ile Ser
           275                 280                 285
   Ile Ala Gly Ala Leu Lys Asn Val Val Ala Leu Ala Cys Gly Phe Val
       290                 295                 300
   Glu Gly Met Gly Trp Gly Asn Asn Ala Ser Ala Ala Ile Gln Arg Leu
   305                 310                 315                 320
   Gly Leu Gly Glu Ile Ile Lys Phe Gly Arg Met Phe Phe Pro Glu Ser
                   325                 330                 335
   Lys Val Glu Thr Tyr Tyr Gln Glu Ser Ala Gly Val Ala Asp Leu Ile
               340                 345                 350
   Thr Thr Cys Ser Gly Gly Arg Asn Val Lys Val Ala Thr Tyr Met Ala
           355                 360                 365
   Lys Thr Gly Lys Ser Ala Leu Glu Ala Glu Lys Glu Leu Leu Asn Gly
       370                 375                 380
   Gln Ser Ala Gln Gly Ile Ile Thr Cys Arg Glu Val His Glu Trp Leu
   385                 390                 395                 400
   Gln Thr Cys Glu Leu Thr Gln Glu Phe Pro Ile Ile Arg Gly Ser Leu
                   405                 410                 415
   Pro Asp Ser Leu Gln Gln Arg Pro His Gly Arg Pro Thr Gly Asp Asp
               420                 425                 430
<210> 5
   <211> 384
   <212> PRT
   <213> Saccharomyces cerevisiae
<400> 5
   Met Thr Ala His Thr Asn Ile Lys Gln His Lys His Cys His Glu Asp
   1               5                   10                  15
   His Pro Ile Arg Arg Ser Asp Ser Ala Val Ser Ile Val His Leu Lys
               20                  25                  30
   Arg Ala Pro Phe Lys Val Thr Val Ile Gly Ser Gly Asn Trp Gly Thr
           35                  40                  45
   Thr Ile Ala Lys Val Ile Ala Glu Asn Thr Glu Leu His Ser His Ile
       50                  55                  60
   Phe Glu Pro Glu Val Arg Met Trp Val Phe Asp Glu Lys Ile Gly Asp
   65                  70                  75                  80
   Glu Asn Leu Thr Asp Ile Ile Asn Thr Arg His Gln Asn Val Lys Tyr
                   85                  90                  95
   Leu Pro Asn Ile Asp Leu Pro His Asn Leu Val Ala Asp Pro Asp Leu
               100                 105                 110
   Leu His Ser Ile Lys Gly Ala Asp Ile Leu Val Phe Asn Ile Pro His
           115                 120                 125
   Gln Phe Leu Pro Asn Ile Val Lys Gln Leu Gln Gly His Val Ala Pro
       130                 135                 140
   His Val Arg Ala Ile Ser Cys Leu Lys Gly Phe Glu Leu Gly Ser Lys
   145                 150                 155                 160
   Gly Val Gln Leu Leu Ser Ser Tyr Val Thr Asp Glu Leu Gly Ile Gln
                   165                 170                 175
   Cys Gly Ala Leu Ser Gly Ala Asn Leu Ala Pro Glu Val Ala Lys Glu
               180                 185                 190
   His Trp Ser Glu Thr Thr Val Ala Tyr Gln Leu Pro Lys Asp Tyr Gln
           195                 200                 205
   Gly Asp Gly Lys Asp Val Asp His Lys Ile Leu Lys Leu Leu Phe His
       210                 215                 220
   Arg Pro Tyr Phe His Val Asn Val Ile Asp Asp Val Ala Gly Ile Ser
   225                 230                 235                 240
   Ile Ala Gly Ala Leu Lys Asn Val Val Ala Leu Ala Cys Gly Phe Val
                   245                 250                 255
   Glu Gly Met Gly Trp Gly Asn Asn Ala Ser Ala Ala Ile Gln Arg Leu
               260                 265                 270
   Gly Leu Gly Glu Ile Ile Lys Phe Gly Arg Met Phe Phe Pro Glu Ser
           275                 280                 285
   Lys Val Glu Thr Tyr Tyr Gln Glu Ser Ala Gly Val Ala Asp Leu Ile
       290                 295                 300
   Thr Thr Cys Ser Gly Gly Arg Asn Val Lys Val Ala Thr Tyr Met Ala
   305                 310                 315                 320
   Lys Thr Gly Lys Ser Ala Leu Glu Ala Glu Lys Glu Leu Leu Asn Gly
                   325                 330                 335
   Gln Ser Ala Gln Gly Ile Ile Thr Cys Arg Glu Val His Glu Trp Leu
               340                 345                 350
   Gln Thr Cys Glu Leu Thr Gln Glu Phe Pro Ile Ile Arg Gly Ser Leu
           355                 360                 365
   Pro Asp Ser Leu Gln Gln Arg Pro His Gly Arg Pro Thr Gly Asp Asp
       370                 375                 380
<210> 6
   <211> 1122
   <212> DNA
   <213> Schizosaccharomyces pombe
<220>
   <221> CDS
   <222> (1)..(1119)
   <223> coding for G3PDH
<400> 6
   atg act gtg gct gct ttg aac aaa ctc agc gct ctc tcc gga agt att      48
   Met Thr Val Ala Ala Leu Asn Lys Leu Ser Ala Leu Ser Gly Ser Ile
    1               5                  10                  15
   caa aaa tct ttt tca cct aaa ctt att tct gtt ggt atc atc gga tca      96
   Gln Lys Ser Phe Ser Pro Lys Leu Ile Ser Val Gly Ile Ile Gly Ser
               20                  25                  30
   gga aat tgg gga acc gct att gct aaa ata tgt ggt gaa aat gcc aag     144
   Gly Asn Trp Gly Thr Ala Ile Ala Lys Ile Cys Gly Glu Asn Ala Lys
           35                  40                  45
   gct cat cct gat att ttc cat cct caa gta cac atg tgg atg tat gaa     192
   Ala His Pro Asp Ile Phe His Pro Gln Val His Met Trp Met Tyr Glu
       50                  55                  60
   gag aag att caa cat gag gga aaa gag tgc aat ctc acg gaa gtt ttt     240
   Glu Lys Ile Gln His Glu Gly Lys Glu Cys Asn Leu Thr Glu Val Phe
   65                  70                  75                  80
   aac act act cat gaa aac gtt aaa tat ctc aaa ggt atc aaa tgc cct     288
   Asn Thr Thr His Glu Asn Val Lys Tyr Leu Lys Gly Ile Lys Cys Pro
                   85                  90                  95
   tct aac gtc ttc gca aac ccg gac att cgt gat gta ggt tca cgt agc     336
   Ser Asn Val Phe Ala Asn Pro Asp Ile Arg Asp Val Gly Ser Arg Ser
               100                 105                 110
   gac att ctg gta tgg gtt ctc cct cac cag ttc gtt gtg cgt att tgc     384
   Asp Ile Leu Val Trp Val Leu Pro His Gln Phe Val Val Arg Ile Cys
           115                 120                 125
   aat caa ttg aag gga tgc cta aag aag gat gct gtt gca att tca tgc     432
   Asn Gln Leu Lys Gly Cys Leu Lys Lys Asp Ala Val Ala Ile Ser Cys
       130                 135                 140
   atc aaa ggt gta tct gtc acc aag gac cgt gtt cgc ctc ttt tct gat     480
   Ile Lys Gly Val Ser Val Thr Lys Asp Arg Val Arg Leu Phe Ser Asp
   145                 150                 155                 160
   att atc gaa gaa aac acg gga atg tat tgt ggc gtt ctc tct ggc gcc     528
   Ile Ile Glu Glu Asn Thr Gly Met Tyr Cys Gly Val Leu Ser Gly Ala
                   165                 170                 175
   aac att gcc agc gaa gtt gct caa gag aag ttt tgc gaa act act atc     576
   Asn Ile Ala Ser Glu Val Ala Gln Glu Lys Phe Cys Glu Thr Thr Ile
               180                 185                 190
   gga tat ttg cct aat agt tct gtt aat ccc cgc tat act cct aag act     624
   Gly Tyr Leu Pro Asn Ser Ser Val Asn Pro Arg Tyr Thr Pro Lys Thr
           195                 200                 205
   atc caa gct ttg ttt aac cgt ccc tac ttc cgt gtc aac att gtt gag     672
   Ile Gln Ala Leu Phe Asn Arg Pro Tyr Phe Arg Val Asn Ile Val Glu
       210                 215                 220
   gat gtt cct ggt gtt gct ttg ggc ggt gca ctc aag aat atc gtc gct     720
   Asp Val Pro Gly Val Ala Leu Gly Gly Ala Leu Lys Asn Ile Val Ala
   225                 230                 235                 240
   gtc gct gcc ggt att att gat gga ctt gaa ttg gga gat aat acc aaa     768
   Val Ala Ala Gly Ile Ile Asp Gly Leu Glu Leu Gly Asp Asn Thr Lys
                   245                 250                 255
   tct gct gtt atg cgc att ggc ctt ctg gaa atg cag aaa ttc ggc agg     816
   Ser Ala Val Met Arg Ile Gly Leu Leu Glu Met Gln Lys Phe Gly Arg
               260                 265                 270
   atg ttt ttc gat tgt aag cct ctt act atg agc gag gaa tct tgt ggc     864
   Met Phe Phe Asp Cys Lys Pro Leu Thr Met Ser Glu Glu Ser Cys Gly
           275                 280                 285
   ata gcc gat tta att aca act tgc tta ggc ggc cgt aac cac aaa tgc     912
   Ile Ala Asp Leu Ile Thr Thr Cys Leu Gly Gly Arg Asn His Lys Cys
       290                 295                 300
   gct gtg gca ttt gtc aag aca gga aag ccc atg cat gtt gtt gaa caa     960
   Ala Val Ala Phe Val Lys Thr Gly Lys Pro Met His Val Val Glu Gln
   305                 310                 315                 320
   gaa ctt ctt gat ggt cag aag ttg caa ggt gca gct acc gcg aag gag     1008
   Glu Leu Leu Asp Gly Gln Lys Leu Gln Gly Ala Ala Thr Ala Lys Glu
                   325                 330                 335
   gtt tat gag ttc ctt gat aac cag aat aag gta agc gaa ttc cca ttg     1056
   Val Tyr Glu Phe Leu Asp Asn Gln Asn Lys Val Ser Glu Phe Pro Leu
               340                 345                 350
   ttt aca gct gtt tat cgc att gtt tat gag gga ctt cca cct aat aag     1104
   Phe Thr Ala Val Tyr Arg Ile Val Tyr Glu Gly Leu Pro Pro Asn Lys
           355                 360                 365
   ctt ctg gag gct att taa                                             1122
   Leu Leu Glu Ala Ile
       370
<210> 7
   <211> 373
   <212> PRT
   <213> Schizosaccharomyces pombe
<400> 7
   Met Thr Val Ala Ala Leu Asn Lys Leu Ser Ala Leu Ser Gly Ser Ile
   1               5                   10                  15
   Gln Lys Ser Phe Ser Pro Lys Leu Ile Ser Val Gly Ile Ile Gly Ser
               20                  25                  30
   Gly Asn Trp Gly Thr Ala Ile Ala Lys Ile Cys Gly Glu Asn Ala Lys
           35                  40                  45
   Ala His Pro Asp Ile Phe His Pro Gln Val His Met Trp Met Tyr Glu
       50                  55                  60
   Glu Lys Ile Gln His Glu Gly Lys Glu Cys Asn Leu Thr Glu Val Phe
   65                  70                  75                  80
   Asn Thr Thr His Glu Asn Val Lys Tyr Leu Lys Gly Ile Lys Cys Pro
                   85                  90                  95
   Ser Asn Val Phe Ala Asn Pro Asp Ile Arg Asp Val Gly Ser Arg Ser
               100                 105                 110
   Asp Ile Leu Val Trp Val Leu Pro His Gln Phe Val Val Arg Ile Cys
           115                 120                 125
   Asn Gln Leu Lys Gly Cys Leu Lys Lys Asp Ala Val Ala Ile Ser Cys
       130                 135                 140
   Ile Lys Gly Val Ser Val Thr Lys Asp Arg Val Arg Leu Phe Ser Asp
   145                 150                 155                 160
   Ile Ile Glu Glu Asn Thr Gly Met Tyr Cys Gly Val Leu Ser Gly Ala
                   165                 170                 175
   Asn Ile Ala Ser Glu Val Ala Gln Glu Lys Phe Cys Glu Thr Thr Ile
               180                 185                 190
   Gly Tyr Leu Pro Asn Ser Ser Val Asn Pro Arg Tyr Thr Pro Lys Thr
           195                 200                 205
   Ile Gln Ala Leu Phe Asn Arg Pro Tyr Phe Arg Val Asn Ile Val Glu
       210                 215                 220
   Asp Val Pro Gly Val Ala Leu Gly Gly Ala Leu Lys Asn Ile Val Ala
   225                 230                 235                 240
   Val Ala Ala Gly Ile Ile Asp Gly Leu Glu Leu Gly Asp Asn Thr Lys
                   245                 250                 255
   Ser Ala Val Met Arg Ile Gly Leu Leu Glu Met Gln Lys Phe Gly Arg
               260                 265                 270
   Met Phe Phe Asp Cys Lys Pro Leu Thr Met Ser Glu Glu Ser Cys Gly
           275                 280                 285
   Ile Ala Asp Leu Ile Thr Thr Cys Leu Gly Gly Arg Asn His Lys Cys
       290                 295                 300
   Ala Val Ala Phe Val Lys Thr Gly Lys Pro Met His Val Val Glu Gln
   305                 310                 315                 320
   Glu Leu Leu Asp Gly Gln Lys Leu Gln Gly Ala Ala Thr Ala Lys Glu
                   325                 330                 335
   Val Tyr Glu Phe Leu Asp Asn Gln Asn Lys Val Ser Glu Phe Pro Leu
               340                 345                 350
   Phe Thr Ala Val Tyr Arg Ile Val Tyr Glu Gly Leu Pro Pro Asn Lys
           355                 360                 365
   Leu Leu Glu Ala Ile
       370
<210> 8
   <211> 1155
   <212> DNA
   <213> Schizosaccharomyces pombe
<220>
   <221> CDS
   <222> (1)..(1152)
   <223> coding for G3PDH
<400> 8
   atg tct gga tat ggt caa caa ggt gtt tct gct gcc aac atc gac agc    48
   Met Ser Gly Tyr Gly Gln Gln Gly Val Ser Ala Ala Asn Ile Asp Ser
    1               5                  10                  15
   atc cgc ccc aag aaa cgt ttg tca att ggt gta gtt ggc tcc ggt aac    96
   Ile Arg Pro Lys Lys Arg Leu Ser Ile Gly Val Val Gly Ser Gly Asn
               20                  25                  30
   tgg ggt act gcc att gcc aag att tgc ggt gaa aat gcc cgt gcc cac   144
   Trp Gly Thr Ala Ile Ala Lys Ile Cys Gly Glu Asn Ala Arg Ala His
           35                  40                  45
   ggt cac cat ttc aga ggt aag ggg cgc atg tgg gtc ttt gag gag gag   192
   Gly His His Phe Arg Gly Lys Gly Arg Met Trp Val Phe Glu Glu Glu
       50                  55                  60
   att gag tac aag ggt gag aag aga aag ctc acc gaa gta ttc aac gaa      240
   Ile Glu Tyr Lys Gly Glu Lys Arg Lys Leu Thr Glu Val Phe Asn Glu
    65                 70                  75                  80
   gct cac gag aat gtc aaa tac tta ccc ggc atc gaa tgc cct ccc aac      288
   Ala His Glu Asn Val Lys Tyr Leu Pro Gly Ile Glu Cys Pro Pro Asn
                   85                  90                  95
   gtt att gcc gtc ccc gat gtt cgt gag gtc gct aga cgt gcc gac atc      336
   Val Ile Ala Val Pro Asp Val Arg Glu Val Ala Arg Arg Ala Asp Ile
               100                 105                 110
   ctt gtc ttt gtc gtt cct cat caa ttt att gaa cgc gtt tgg cac caa      384
   Leu Val Phe Val Val Pro His Gln Phe Ile Glu Arg Val Trp His Gln
           115                 120                 125
   atg gtc ggt ctc att cgc cct ggt gcc gtt ggt att tcc tgt atc aag      432
   Met Val Gly Leu Ile Arg Pro Gly Ala Val Gly Ile Ser Cys Ile Lys
       130                 135                 140
   ggt gtt gct gtc agc aag gaa ggc tcg ctt tac tct gag gtt atc agc      480
   Gly Val Ala Val Ser Lys Glu Gly Ser Leu Tyr Ser Glu Val Ile Ser
   145                 150                 155                 160
   gag aaa ctc ggt att tac tgt ggt gtt ctt tct ggt gct aac gtt gca      528
   Glu Lys Leu Gly Ile Tyr Cys Gly Val Leu Ser Gly Ala Asn Val Ala
                   165                 170                 175
   aac gaa gtt gcc cgt gag caa ttc tgt gag act act att ggt ttc aac      576
   Asn Glu Val Ala Arg Glu Gln Phe Cys Glu Thr Thr Ile Gly Phe Asn
               180                 185                 190
   cct cct aat gaa gtt gat atc cct cgc gag caa atc gcc gcc gtc tct      624
   Pro Pro Asn Glu Val Asp Ile Pro Arg Glu Gln Ile Ala Ala Val Ser
           195                 200                 205
   gat cgc cct tac ttc tca gtt gtc tcc gtt gac gac gtt gcc ggt gtc      672
   Asp Arg Pro Tyr Phe Ser Val Val Ser Val Asp Asp Val Ala Gly Val
       210                 215                 220
   gcc ttg ggt ggt gct ttg aag aac gta gtt gcc atg gcc gtt ggt ttc      720
   Ala Leu Gly Gly Ala Leu Lys Asn Val Val Ala Met Ala Val Gly Phe
   225                 230                 235                 240
   gct gat ggt ttg gaa tgg ggc ggt aat acc aag gcc gct att atg cgt      768
   Ala Asp Gly Leu Glu Trp Gly Gly Asn Thr Lys Ala Ala Ile Met Arg
                   245                 250                 255
   cgt ggt ttg ttg gag atg caa aag ttt gct act acc ttc ttc gac tct      816
   Arg Gly Leu Leu Glu Met Gln Lys Phe Ala Thr Thr Phe Phe Asp Ser
               260                 265                 270
   gat cct cgt acc atg gtt gag caa tct tgc ggt atc gct gac ttg gtc      864
   Asp Pro Arg Thr Met Val Glu Gln Ser Cys Gly Ile Ala Asp Leu Val
           275                 280                 285
   act tct tgt ttg ggt ggc cgt aac aat cgt tgt gct gaa gca ttt gtc      912
   Thr Ser Cys Leu Gly Gly Arg Asn Asn Arg Cys Ala Glu Ala Phe Val
       290                 295                 300
   aag act ggt aaa tct tta gag acg ctt gaa aaa gag ctc tta ggt ggt      960
   Lys Thr Gly Lys Ser Leu Glu Thr Leu Glu Lys Glu Leu Leu Gly Gly
   305                 310                 315                 320
   caa ctt ctt caa gga gct gcc act tcc aag gat gtt cat gaa ttc ctt      1008
   Gln Leu Leu Gln Gly Ala Ala Thr Ser Lys Asp Val His Glu Phe Leu
                   325                 330                 335
   ctc acc aag gat atg gtc aag gat ttc ccc ttg ttc act gcc gtt tat    1056
   Leu Thr Lys Asp Met Val Lys Asp Phe Pro Leu Phe Thr Ala Val Tyr
               340                 345                 350
   aac att tcc tat gaa gac atg gat ccc aag gat ttg atc atc gtc ctt    1104
   Asn Ile Ser Tyr Glu Asp Met Asp Pro Lys Asp Leu Ile Ile Val Leu
           355                 360                 365
   caa ccc ctt aag gag gac tct gag aac gag ggc ggt act gaa acc gag    1152
   Gln Pro Leu Lys Glu Asp Ser Glu Asn Glu Gly Gly Thr Glu Thr Glu
       370                 375                 380
   taa                                                                1155
<210> 9
   <211> 384
   <212> PRT
   <213> Schizosaccharomyces pombe
<400> 9
   Met Ser Gly Tyr Gly Gln Gln Gly Val Ser Ala Ala Asn Ile Asp Ser
   1               5                   10                  15
   Ile Arg Pro Lys Lys Arg Leu Ser Ile Gly Val Val Gly Ser Gly Asn
               20                  25                  30
   Trp Gly Thr Ala Ile Ala Lys Ile Cys Gly Glu Asn Ala Arg Ala His
           35                  40                  45
   Gly His His Phe Arg Gly Lys Gly Arg Met Trp Val Phe Glu Glu Glu
       50                  55                  60
   Ile Glu Tyr Lys Gly Glu Lys Arg Lys Leu Thr Glu Val Phe Asn Glu
   65                  70                  75                  80
   Ala His Glu Asn Val Lys Tyr Leu Pro Gly Ile Glu Cys Pro Pro Asn
                   85                  90                  95
   Val Ile Ala Val Pro Asp Val Arg Glu Val Ala Arg Arg Ala Asp Ile
               100                 105                 110
   Leu Val Phe Val Val Pro His Gln Phe Ile Glu Arg Val Trp His Gln
           115                 120                 125
   Met Val Gly Leu Ile Arg Pro Gly Ala Val Gly Ile Ser Cys Ile Lys
       130                 135                 140
   Gly Val Ala Val Ser Lys Glu Gly Ser Leu Tyr Ser Glu Val Ile Ser
   145                 150                 155                 160
   Glu Lys Leu Gly Ile Tyr Cys Gly Val Leu Ser Gly Ala Asn Val Ala
                   165                 170                 175
   Asn Glu Val Ala Arg Glu Gln Phe Cys Glu Thr Thr Ile Gly Phe Asn
               180                 185                 190
   Pro Pro Asn Glu Val Asp Ile Pro Arg Glu Gln Ile Ala Ala Val Ser
           195                 200                 205
   Asp Arg Pro Tyr Phe Ser Val Val Ser Val Asp Asp Val Ala Gly Val
       210                 215                 220
   Ala Leu Gly Gly Ala Leu Lys Asn Val Val Ala Met Ala Val Gly Phe
   225                 230                 235                 240
   Ala Asp Gly Leu Glu Trp Gly Gly Asn Thr Lys Ala Ala Ile Met Arg
                   245                 250                 255
   Arg Gly Leu Leu Glu Met Gln Lys Phe Ala Thr Thr Phe Phe Asp Ser
               260                 265                 270
   Asp Pro Arg Thr Met Val Glu Gln Ser Cys Gly Ile Ala Asp Leu Val
           275                 280                 285
   Thr Ser Cys Leu Gly Gly Arg Asn Asn Arg Cys Ala Glu Ala Phe Val
       290                 295                 300
   Lys Thr Gly Lys Ser Leu Glu Thr Leu Glu Lys Glu Leu Leu Gly Gly
   305                 310                 315                 320
   Gln Leu Leu Gln Gly Ala Ala Thr Ser Lys Asp Val His Glu Phe Leu
                   325                 330                 335
   Leu Thr Lys Asp Met Val Lys Asp Phe Pro Leu Phe Thr Ala Val Tyr
               340                 345                 350
   Asn Ile Ser Tyr Glu Asp Met Asp Pro Lys Asp Leu Ile Ile Val Leu
           355                 360                 365
   Gln Pro Leu Lys Glu Asp Ser Glu Asn Glu Gly Gly Thr Glu Thr Glu
       370                 375                 380
<210> 10
   <211> 1197
   <212> DNA
   <213> Yarrowia lipolytica
<220>
   <221> CDS
   <222> (1)..(1194)
   <223> coding for G3PDH
<220>
   <221> CDS
   <222> (40)..(1194)
<400> 10
   atg agc gct cta ctt aga tcg tcc ctg cgt ttt aaa cac atg tcc gcc    48
   Met Ser Ala Leu Leu Arg Ser Ser Leu Arg Phe Lys His Met Ser Ala
    1               5                  10                  15
   gtc aac cgt ctc aca caa cag ctt cga ctg ctg acc gcc tcc gcg cct    96
   Val Asn Arg Leu Thr Gln Gln Leu Arg Leu Leu Thr Ala Ser Ala Pro
               20                  25                  30
   ctc agc gca gcc aac acc gcc ggc aag gct cct ttc aag gtc gcc gtt    144
   Leu Ser Ala Ala Asn Thr Ala Gly Lys Ala Pro Phe Lys Val Ala Val
           35                  40                  45
   gtt ggt tct ggt aac tgg gga acc acc gtc gcc aag att gtc gcc gag    192
   Val Gly Ser Gly Asn Trp Gly Thr Thr Val Ala Lys Ile Val Ala Glu
       50                  55                  60
   aac tgc act gct cac ccc gag ctc ttt gag ccc gag gtt cga gtc tgg    240
   Asn Cys Thr Ala His Pro Glu Leu Phe Glu Pro Glu Val Arg Val Trp
   65                  70                  75                  80
   gtt cga gaa gag aag gtc aac ggc aag aac ctg acc gac att ttc aac    288
   Val Arg Glu Glu Lys Val Asn Gly Lys Asn Leu Thr Asp Ile Phe Asn
                   85                  90                  95
   gct gag cac gag aac gtg cga tac ctc cct aaa atc aaa ctt cct cac    336
   Ala Glu His Glu Asn Val Arg Tyr Leu Pro Lys Ile Lys Leu Pro His
               100                 105                 110
   aac ctg atc gcc gag ccg gat ctg ctc aag gcc gtc gag ggt gcc aac    384
   Asn Leu Ile Ala Glu Pro Asp Leu Leu Lys Ala Val Glu Gly Ala Asn
           115                 120                 125
   atc atc gtc ttc aac ctg ccc cat cag ttc ctg gct ggt gtc tgc aag     432
   Ile Ile Val Phe Asn Leu Pro His Gln Phe Leu Ala Gly Val Cys Lys
       130                 135                 140
   cag ctc aag ggc cac gtc aac ccc aag gct aga gcc atc tcc tgc ctc     480
   Gln Leu Lys Gly His Val Asn Pro Lys Ala Arg Ala Ile Ser Cys Leu
   145                 150                 155                 160
   aag ggt cta gat gtc acc ccc cag ggt gtt tac ctg ctc tcc gac gtt     528
   Lys Gly Leu Asp Val Thr Pro Gln Gly Val Tyr Leu Leu Ser Asp Val
                   165                 170                 175
   atc gag aac gag acc ggt ctc cac tgc ggt gtt ctg tcc ggg gct aac     576
   Ile Glu Asn Glu Thr Gly Leu His Cys Gly Val Leu Ser Gly Ala Asn
               180                 185                 190
   ctc gcc acc gag atc gct ctg gag aag tac tcc gag act acc gtt gct     624
   Leu Ala Thr Glu Ile Ala Leu Glu Lys Tyr Ser Glu Thr Thr Val Ala
           195                 200                 205
   tac aac cga ccc aag gac ttc ttt ggc gag ggt gat gtg acc aac gat     672
   Tyr Asn Arg Pro Lys Asp Phe Phe Gly Glu Gly Asp Val Thr Asn Asp
       210                 215                 220
   gtg ctc aag gct ctg ttc cac cga ccc tac ttc cat gtg cga tgc gtt     720
   Val Leu Lys Ala Leu Phe His Arg Pro Tyr Phe His Val Arg Cys Val
   225                 230                 235                 240
   cag gac gtc gcc ggt gtc tcc atc gga ggt gcc ctt aag aac gtt gtt     768
   Gln Asp Val Ala Gly Val Ser Ile Gly Gly Ala Leu Lys Asn Val Val
                   245                 250                 255
   gcc ctt tgc gcc ggt ttc gtc gag ggc aag aac tgg gga gac aac gcc     816
   Ala Leu Cys Ala Gly Phe Val Glu Gly Lys Asn Trp Gly Asp Asn Ala
               260                 265                 270
   aag gcc gca att atg cga cga ggc atg ctt gag atg atc aac ttc tcc     864
   Lys Ala Ala Ile Met Arg Arg Gly Met Leu Glu Met Ile Asn Phe Ser
           275                 280                 285
   aag cga ttc ttc ccc gaa act gat att aac act ctt aca gtc gag tct     912
   Lys Arg Phe Phe Pro Glu Thr Asp Ile Asn Thr Leu Thr Val Glu Ser
       290                 295                 300
   gcc ggt gtg gcc gat ctc atc acc tcg tgc gct gga ggc cga aac ttc     960
   Ala Gly Val Ala Asp Leu Ile Thr Ser Cys Ala Gly Gly Arg Asn Phe
   305                 310                 315                 320
   aag gtc ggc cga gca ttc gga aag gag agc ggc tcc ggc aag acc atc     1008
   Lys Val Gly Arg Ala Phe Gly Lys Glu Ser Gly Ser Gly Lys Thr Ile
                   325                 330                 335
   cag gac gtg gag aag gag ctt ctc aac ggc cag tcc gcc cag ggc gtc     1056
   Gln Asp Val Glu Lys Glu Leu Leu Asn Gly Gln Ser Ala Gln Gly Val
               340                 345                 350
   atc aca tgt aac gag gtc cac gag ctg ctc aag aac aag aac atg cag     1104
   Ile Thr Cys Asn Glu Val His Glu Leu Leu Lys Asn Lys Asn Met Gln
           355                 360                 365
   aag gac ttc cct ctg ttc gag tcc acc tgg ggc att atc cac ggt gag     1152
   Lys Asp Phe Pro Leu Phe Glu Ser Thr Trp Gly Ile Ile His Gly Glu
       370                 375                 380
   ctc aag att gat gat ctc ccc gag att ctt tac cac gcc aac   tag       1197
   Leu Lys Ile Asp Asp Leu Pro Glu Ile Leu Tyr His Ala Asn
   385                 390                 395
<210> 11
   <211> 398
   <212> PRT
   <213> Yarrowia lipolytica
<400> 11
   Met Ser Ala Leu Leu Arg Ser Ser Leu Arg Phe Lys His Met Ser Ala
   1               5                   10                  15
   Val Asn Arg Leu Thr Gln Gln Leu Arg Leu Leu Thr Ala Ser Ala Pro
               20                  25                  30
   Leu Ser Ala Ala Asn Thr Ala Gly Lys Ala Pro Phe Lys Val Ala Val
           35                  40                  45
   Val Gly Ser Gly Asn Trp Gly Thr Thr Val Ala Lys Ile Val Ala Glu
       50                  55                  60
   Asn Cys Thr Ala His Pro Glu Leu Phe Glu Pro Glu Val Arg Val Trp
   65                  70                  75                  80
   Val Arg Glu Glu Lys Val Asn Gly Lys Asn Leu Thr Asp Ile Phe Asn
                   85                  90                  95
   Ala Glu His Glu Asn Val Arg Tyr Leu Pro Lys Ile Lys Leu Pro His
               100                 105                 110
   Asn Leu Ile Ala Glu Pro Asp Leu Leu Lys Ala Val Glu Gly Ala Asn
           115                 120                 125
   Ile Ile Val Phe Asn Leu Pro His Gln Phe Leu Ala Gly Val Cys Lys
       130                 135                 140
   Gln Leu Lys Gly His Val Asn Pro Lys Ala Arg Ala Ile Ser Cys Leu
   145                 150                 155                 160
   Lys Gly Leu Asp Val Thr Pro Gln Gly Val Tyr Leu Leu Ser Asp Val
                   165                 170                 175
   Ile Glu Asn Glu Thr Gly Leu His Cys Gly Val Leu Ser Gly Ala Asn
               180                 185                 190
   Leu Ala Thr Glu Ile Ala Leu Glu Lys Tyr Ser Glu Thr Thr Val Ala
           195                 200                 205
   Tyr Asn Arg Pro Lys Asp Phe Phe Gly Glu Gly Asp Val Thr Asn Asp
       210                 215                 220
   Val Leu Lys Ala Leu Phe His Arg Pro Tyr Phe His Val Arg Cys Val
   225                 230                 235                 240
   Gln Asp Val Ala Gly Val Ser Ile Gly Gly Ala Leu Lys Asn Val Val
                   245                 250                 255
   Ala Leu Cys Ala Gly Phe Val Glu Gly Lys Asn Trp Gly Asp Asn Ala
               260                 265                 270
   Lys Ala Ala Ile Met Arg Arg Gly Met Leu Glu Met Ile Asn Phe Ser
           275                 280                 285
   Lys Arg Phe Phe Pro Glu Thr Asp Ile Asn Thr Leu Thr Val Glu Ser
       290                 295                 300
   Ala Gly Val Ala Asp Leu Ile Thr Ser Cys Ala Gly Gly Arg Asn Phe
   305                 310                 315                 320
   Lys Val Gly Arg Ala Phe Gly Lys Glu Ser Gly Ser Gly Lys Thr Ile
                   325                 330                 335
   Gln Asp Val Glu Lys Glu Leu Leu Asn Gly Gln Ser Ala Gln Gly Val
               340                 345                 350
   Ile Thr Cys Asn Glu Val His Glu Leu Leu Lys Asn Lys Asn Met Gln
           355                 360                 365
   Lys Asp Phe Pro Leu Phe Glu Ser Thr Trp Gly Ile Ile His Gly Glu
       370                 375                 380
   Leu Lys Ile Asp Asp Leu Pro Glu Ile Leu Tyr His Ala Asn
   385                 390                 395
<210> 12
   <211> 385
   <212> PRT
   <213> Yarrowia lipolytica
<400> 12
   Met Ser Ala Val Asn Arg Leu Thr Gln Gln Leu Arg Leu Leu Thr Ala
   1               5                   10                  15
   Ser Ala Pro Leu Ser Ala Ala Asn Thr Ala Gly Lys Ala Pro Phe Lys
               20                  25                  30
   Val Ala Val Val Gly Ser Gly Asn Trp Gly Thr Thr Val Ala Lys Ile
           35                  40                  45
   Val Ala Glu Asn Cys Thr Ala His Pro Glu Leu Phe Glu Pro Glu Val
       50                  55                  60
   Arg Val Trp Val Arg Glu Glu Lys Val Asn Gly Lys Asn Leu Thr Asp
   65                  70                  75                  80
   Ile Phe Asn Ala Glu His Glu Asn Val Arg Tyr Leu Pro Lys Ile Lys
                   85                  90                  95
   Leu Pro His Asn Leu Ile Ala Glu Pro Asp Leu Leu Lys Ala Val Glu
               100                 105                 110
   Gly Ala Asn Ile Ile Val Phe Asn Leu Pro His Gln Phe Leu Ala Gly
           115                 120                 125
   Val Cys Lys Gln Leu Lys Gly His Val Asn Pro Lys Ala Arg Ala Ile
       130                 135                 140
   Ser Cys Leu Lys Gly Leu Asp Val Thr Pro Gln Gly Val Tyr Leu Leu
   145                 150                 155                 160
   Ser Asp Val Ile Glu Asn Glu Thr Gly Leu His Cys Gly Val Leu Ser
                   165                 170                 175
   Gly Ala Asn Leu Ala Thr Glu Ile Ala Leu Glu Lys Tyr Ser Glu Thr
               180                 185                 190
   Thr Val Ala Tyr Asn Arg Pro Lys Asp Phe Phe Gly Glu Gly Asp Val
           195                 200                 205
   Thr Asn Asp Val Leu Lys Ala Leu Phe His Arg Pro Tyr Phe His Val
       210                 215                 220
   Arg Cys Val Gln Asp Val Ala Gly Val Ser Ile Gly Gly Ala Leu Lys
   225                 230                 235                 240
   Asn Val Val Ala Leu Cys Ala Gly Phe Val Glu Gly Lys Asn Trp Gly
                   245                 250                 255
   Asp Asn Ala Lys Ala Ala Ile Met Arg Arg Gly Met Leu Glu Met Ile
               260                 265                 270
   Asn Phe Ser Lys Arg Phe Phe Pro Glu Thr Asp Ile Asn Thr Leu Thr
           275                 280                 285
   Val Glu Ser Ala Gly Val Ala Asp Leu Ile Thr Ser Cys Ala Gly Gly
       290                 295                 300
   Arg Asn Phe Lys Val Gly Arg Ala Phe Gly Lys Glu Ser Gly Ser Gly
   305                 310                 315                 320
   Lys Thr Ile Gln Asp Val Glu Lys Glu Leu Leu Asn Gly Gln Ser Ala
                   325                 330                 335
   Gln Gly Val Ile Thr Cys Asn Glu Val His Glu Leu Leu Lys Asn Lys
               340                 345                 350
   Asn Met Gln Lys Asp Phe Pro Leu Phe Glu Ser Thr Trp Gly Ile Ile
           355                 360                 365
   His Gly Glu Leu Lys Ile Asp Asp Leu Pro Glu Ile Leu Tyr His Ala
       370                 375                 380
   Asn
   385
<210> 13
   <211> 1206
   <212> DNA
   <213> Zygosaccharomyces rouxii
<220>
   <221> CDS
   <222> (1)..(1203)
   <223> coding for G3PDH
<400> 13
   atg gcc gct act gac aga tta aac caa acc tct gat atc cta tcg caa      48
   Met Ala Ala Thr Asp Arg Leu Asn Gln Thr Ser Asp Ile Leu Ser Gln
   1               5                   10                  15
   tct atg aag aag acc gac tca tca atg tca gtc gtt acc gct gag aat      96
   Ser Met Lys Lys Thr Asp Ser Ser Met Ser Val Val Thr Ala Glu Asn
               20                  25                  30
   cca tac aaa gtt tcc gtc gtc ggc tct ggt aac tgg ggt acc acc atc     144
   Pro Tyr Lys Val Ser Val Val Gly Ser Gly Asn Trp Gly Thr Thr Ile
           35                  40                  45
   gcc aag gtc gtt gcc gaa aac acc aag gaa aag cca gaa ttg ttc caa     192
   Ala Lys Val Val Ala Glu Asn Thr Lys Glu Lys Pro Glu Leu Phe Gln
       50                  55                  60
   gaa cgt gtg gac atg tgg gtg ttt gaa gaa cag atc gac ggt act cca     240
   Glu Arg Val Asp Met Trp Val Phe Glu Glu Gln Ile Asp Gly Thr Pro
   65                  70                  75                  80
   ttg gcc caa atc atc aac acc aag cac cag aac gtg aaa tac ttg cca     288
   Leu Ala Gln Ile Ile Asn Thr Lys His Gln Asn Val Lys Tyr Leu Pro
                   85                  90                  95
   aac atc gac ctt ccg gac aat ttg gtc gct aac cca gac ttg att gcc     336
   Asn Ile Asp Leu Pro Asp Asn Leu Val Ala Asn Pro Asp Leu Ile Ala
               100                 105                 110
   acc acg aag gac gcc gat gtg att gtt ttc aac gtt ccc cat caa ttt     384
   Thr Thr Lys Asp Ala Asp Val Ile Val Phe Asn Val Pro His Gln Phe
           115                 120                 125
   ttg ggc cgt atc gtt gct caa atg aag ggt caa atc aaa cca act gca     432
   Leu Gly Arg Ile Val Ala Gln Met Lys Gly Gln Ile Lys Pro Thr Ala
       130                 135                 140
   cgt gcg gtc tcc tgt cta aag ggt ttc gaa gtt ggt cca aag ggt gtg     480
   Arg Ala Val Ser Cys Leu Lys Gly Phe Glu Val Gly Pro Lys Gly Val
   145                 150                 155                 160
   cag ctt cta tct gac tac gtc act caa gaa ttg ggt atc gaa tgt ggt     528
   Gln Leu Leu Ser Asp Tyr Val Thr Gln Glu Leu Gly Ile Glu Cys Gly
                   165                 170                 175
   gct cta tct ggt gct aac ttg gcc cca gaa gtc gcc aag gaa cac tgg     576
   Ala Leu Ser Gly Ala Asn Leu Ala Pro Glu Val Ala Lys Glu His Trp
               180                 185                 190
   tcc gag acc acc gtc gct tac cac atc cca gac gac ttc aag ggt gac     624
   Ser Glu Thr Thr Val Ala Tyr His Ile Pro Asp Asp Phe Lys Gly Asp
           195                 200                 205
   ggt aag gac atc gac cac cgt gtc ttg aag cag ttg ttc cac aga cca     672
   Gly Lys Asp Ile Asp His Arg Val Leu Lys Gln Leu Phe His Arg Pro
       210                 215                 220
   tac ttc cac gtg aat gtg att gac gat gtt gct ggt atc tcc atc gca     720
   Tyr Phe His Val Asn Val Ile Asp Asp Val Ala Gly Ile Ser Ile Ala
   225                 230                 235                 240
   ggt gca ttg aag aac gtg gtc gcc ttg ggt tgc ggt ttc gtt acc ggt     768
   Gly Ala Leu Lys Asn Val Val Ala Leu Gly Cys Gly Phe Val Thr Gly
                   245                 250                 255
   cta ggt tgg ggt aac aac gcc gcc gcc gcc atc caa cgt gtc ggt ttg     816
   Leu Gly Trp Gly Asn Asn Ala Ala Ala Ala Ile Gln Arg Val Gly Leu
               260                 265                 270
   ggt gaa atc atc aag ttc ggt agg atg ttc ttc cca gaa tcc aag gtg     864
   Gly Glu Ile Ile Lys Phe Gly Arg Met Phe Phe Pro Glu Ser Lys Val
           275                 280                 285
   gag act tac tac caa gaa tcc gca ggt gtt gct gac ttg atc acc acc     912
   Glu Thr Tyr Tyr Gln Glu Ser Ala Gly Val Ala Asp Leu Ile Thr Thr
       290                 295                 300
   tgt tcc ggt ggt aga aac gtc cgt gtt gcc acc gaa atg gcc aag act     960
   Cys Ser Gly Gly Arg Asn Val Arg Val Ala Thr Glu Met Ala Lys Thr
   305                 310                 315                 320
   ggt aag agc ggt gag caa gtc gaa aaa gac atc ttg aac ggt caa tcc     1008
   Gly Lys Ser Gly Glu Gln Val Glu Lys Asp Ile Leu Asn Gly Gln Ser
                   325                 330                 335
   gct caa ggt ttg gtc acc tgt aag gaa gtt cac cag tgg tta gaa tct     1056
   Ala Gln Gly Leu Val Thr Cys Lys Glu Val His Gln Trp Leu Glu Ser
               340                 345                 350
   agt gga aac acc gaa gac ttc cca ttg ttc gag gct gtc tac cag atc     1104
   Ser Gly Asn Thr Glu Asp Phe Pro Leu Phe Glu Ala Val Tyr Gln Ile
           355                 360                 365
   act tac gaa aac gtg ccc atg aag gag ttg cca tct atg atc gaa gaa     1152
   Thr Tyr Glu Asn Val Pro Met Lys Glu Leu Pro Ser Met Ile Glu Glu
       370                 375                 380
   ttg gat atc gat agc aca tcg aag tgc gta ttg agt tac aag atg ggt     1200
   Leu Asp Ile Asp Ser Thr Ser Lys Cys Val Leu Ser Tyr Lys Met Gly
   385                 390                 395                 400
   ctc tag                                                             1206
   Leu
<210> 14
   <211> 401
   <212> PRT
   <213> Zygosaccharomyces rouxii
<400> 14
   Met Ala Ala Thr Asp Arg Leu Asn Gln Thr Ser Asp Ile Leu Ser Gln
   1               5                   10                  15
   Ser Met Lys Lys Thr Asp Ser Ser Met Ser Val Val Thr Ala Glu Asn
               20                  25                  30
   Pro Tyr Lys Val Ser Val Val Gly Ser Gly Asn Trp Gly Thr Thr Ile
           35                  40                  45
   Ala Lys Val Val Ala Glu Asn Thr Lys Glu Lys Pro Glu Leu Phe Gln
       50                  55                  60
   Glu Arg Val Asp Met Trp Val Phe Glu Glu Gln Ile Asp Gly Thr Pro
   65                  70                  75                  80
   Leu Ala Gln Ile Ile Asn Thr Lys His Gln Asn Val Lys Tyr Leu Pro
   85 90 95
   Asn Ile Asp Leu Pro Asp Asn Leu Val Ala Asn Pro Asp Leu Ile Ala
   100 105 110
   Thr Thr Lys Asp Ala Asp Val Ile Val Phe Asn Val Pro His Gln Phe
   115 120 125
   Leu Gly Arg Ile Val Ala Gln Met Lys Gly Gln Ile Lys Pro Thr Ala
   130 135 140
   Arg Ala Val Ser Cys Leu Lys Gly Phe Glu Val Gly Pro Lys Gly Val
   145 150 155 160
   Gln Leu Leu Ser Asp Tyr Val Thr Gln Glu Leu Gly Ile Glu Cys Gly
   165 170 175
   Ala Leu Ser Gly Ala Asn Leu Ala Pro Glu Val Ala Lys Glu His Trp
   180 185 190
   Ser Glu Thr Thr Val Ala Tyr His Ile Pro Asp Asp Phe Lys Gly Asp
   195 200 205
   Gly Lys Asp Ile Asp His Arg Val Leu Lys Gln Leu Phe His Arg Pro
   210 215 220
   Tyr Phe His Val Asn Val Ile Asp Asp Val Ala Gly Ile Ser Ile Ala
   225 230 235 240
   Gly Ala Leu Lys Asn Val Val Ala Leu Gly Cys Gly Phe Val Thr Gly
   245 250 255
   Leu Gly Trp Gly Asn Asn Ala Ala Ala Ala Ile Gln Arg Val Gly Leu
   260 265 270
   Gly Glu Ile Ile Lys Phe Gly Arg Met Phe Phe Pro Glu Ser Lys Val
   275 280 285
   Glu Thr Tyr Tyr Gln Glu Ser Ala Gly Val Ala Asp Leu Ile Thr Thr
   290 295 300
   Cys Ser Gly Gly Arg Asn Val Arg Val Ala Thr Glu Met Ala Lys Thr
   305 310 315 320
   Gly Lys Ser Gly Glu Gln Val Glu Lys Asp Ile Leu Asn Gly Gln Ser
   325 330 335
   Ala Gln Gly Leu Val Thr Cys Lys Glu Val His Gln Trp Leu Glu Ser
   340 345 350
   Ser Gly Asn Thr Glu Asp Phe Pro Leu Phe Glu Ala Val Tyr Gln Ile
   355 360 365
   Thr Tyr Glu Asn Val Pro Met Lys Glu Leu Pro Ser Met Ile Glu Glu
   370 375 380
   Leu Asp Ile Asp Ser Thr Ser Lys Cys Val Leu Ser Tyr Lys Met Gly
   385 390 395 400
   Leu
<210> 15
   <211> 1170
   <212> DNA
   <213> Zygosaccharomyces rouxii
<220>
   <221> CDS
   <222> (1)..(1167)
   <223> coding for G3PDH
<400> 15
   atg gcc gcc act gac aga tta aac caa acc tcc gat atc cta tct cat    48
   Met Ala Ala Thr Asp Arg Leu Asn Gln Thr Ser Asp Ile Leu Ser His
   1               5                   10                  15
   tct atg aag aag act gat acc tca atg tca att gtt acc gct gag aat    96
   Ser Met Lys Lys Thr Asp Thr Ser Met Ser Ile Val Thr Ala Glu Asn
               20                  25                  30
   cct tac aag gtc gct gtt gtc ggt tct ggt aac tgg ggt acc act atc   144
   Pro Tyr Lys Val Ala Val Val Gly Ser Gly Asn Trp Gly Thr Thr Ile
           35                  40                  45
   gct aag gtt gtt gcc gaa aac acc aaa gaa aag cca gag ttg ttc caa   192
   Ala Lys Val Val Ala Glu Asn Thr Lys Glu Lys Pro Glu Leu Phe Gln
       50                  55                  60
   gga cgt gtg gac atg tgg gtt ttc gaa gaa caa atc gat ggt act cca   240
   Gly Arg Val Asp Met Trp Val Phe Glu Glu Gln Ile Asp Gly Thr Pro
   65                  70                  75                  80
   ttg act caa atc atc aac acc aaa cac caa aac gtc aaa tac ctt cca   288
   Leu Thr Gln Ile Ile Asn Thr Lys His Gln Asn Val Lys Tyr Leu Pro
                   85                  90                  95
   aac atc gat ctt ccg ggg aat ttg gtc gct aac cca gat ttg atc tct   336
   Asn Ile Asp Leu Pro Gly Asn Leu Val Ala Asn Pro Asp Leu Ile Ser
               100                 105                 110
   act acc aag gac gct gat gtc atc gtt ttc aac gtt cct cac caa ttt   384
   Thr Thr Lys Asp Ala Asp Val Ile Val Phe Asn Val Pro His Gln Phe
           115                 120                 125
   ttg ggc cgt atc gtt tct caa atg aag ggt caa atc aaa cca gat gct   432
   Leu Gly Arg Ile Val Ser Gln Met Lys Gly Gln Ile Lys Pro Asp Ala
       130                 135                 140
   cgt gcc atc tcc tgt cta aag ggt ttc gaa gtt ggt cca aag ggt gtc   480
   Arg Ala Ile Ser Cys Leu Lys Gly Phe Glu Val Gly Pro Lys Gly Val
   145                 150                 155                 160
   caa cta ctt tct gac tac gtc act caa gaa tta ggt atc caa tgt ggt   528
   Gln Leu Leu Ser Asp Tyr Val Thr Gln Glu Leu Gly Ile Gln Cys Gly
                   165                 170                 175
   gcc cta tct ggt gct aac ttg gct cca gaa gtc gcc aag gaa cac tgg   576
   Ala Leu Ser Gly Ala Asn Leu Ala Pro Glu Val Ala Lys Glu His Trp
               180                 185                 190
   tcc gaa act acc gtc gct tac caa gtc cca gat gac ttc aag ggt gaa   624
   Ser Glu Thr Thr Val Ala Tyr Gln Val Pro Asp Asp Phe Lys Gly Glu
           195                 200                 205
   ggt aaa gat atc gac cac cgt gtc ttg aaa caa ttg ttc cac aga cca   672
   Gly Lys Asp Ile Asp His Arg Val Leu Lys Gln Leu Phe His Arg Pro
       210                 215                 220
   tac ttc cac gtc aat gtg atc gac gat gtt gct ggt att tct atc gca   720
   Tyr Phe His Val Asn Val Ile Asp Asp Val Ala Gly Ile Ser Ile Ala
   225                 230                 235                 240
   ggt gca ttg aag aac gtg gtt gcc ttg ggt tgc ggt ttc gtc acc ggt   768
   Gly Ala Leu Lys Asn Val Val Ala Leu Gly Cys Gly Phe Val Thr Gly
                   245                 250                 255
   cta ggc tgg ggt aac aac gct gcc gcc gcc atc caa cgt gtt ggt ttg     816
   Leu Gly Trp Gly Asn Asn Ala Ala Ala Ala Ile Gln Arg Val Gly Leu
               260                 265                 270
   ggt gaa atc atc aag ttc ggt aga atg ttc ttc cca gaa tcc aag gtg     864
   Gly Glu Ile Ile Lys Phe Gly Arg Met Phe Phe Pro Glu Ser Lys Val
           275                 280                 285
   gaa act tac tac caa gaa tct gca ggt gtt gct gat ttg atc act acc     912
   Glu Thr Tyr Tyr Gln Glu Ser Ala Gly Val Ala Asp Leu Ile Thr Thr
       290                 295                 300
   tgt tcc ggt ggt aga aac gtt cgt gtc gcc act gaa atg gcc aag act     960
   Cys Ser Gly Gly Arg Asn Val Arg Val Ala Thr Glu Met Ala Lys Thr
   305                 310                 315                 320
   ggt aag agc ggt gaa caa gtc gaa aag gac atc ttg aac ggt caa tcc     1008
   Gly Lys Ser Gly Glu Gln Val Glu Lys Asp Ile Leu Asn Gly Gln Ser
                   325                 330                 335
   gct caa ggt ttg att act gct aag gaa gtc cac caa tgg ttg gaa tcc     1056
   Ala Gln Gly Leu Ile Thr Ala Lys Glu Val His Gln Trp Leu Glu Ser
               340                 345                 350
   agc ggt cac acc gaa gaa tac cca ttg ttt gaa gcc gtc tac caa atc     1104
   Ser Gly His Thr Glu Glu Tyr Pro Leu Phe Glu Ala Val Tyr Gln Ile
           355                 360                 365
   act tac gaa aac gtg ccc atg aag gag ttg cca tcc atg atc gaa gaa     1152
   Thr Tyr Glu Asn Val Pro Met Lys Glu Leu Pro Ser Met Ile Glu Glu
       370                 375                 380
   ttg gat atc gta gaa taa                                             1170
   Leu Asp Ile Val Glu
   385
<210> 16
   <211> 389
   <212> PRT
   <213> Zygosaccharomyces rouxii
<400> 16
   Met Ala Ala Thr Asp Arg Leu Asn Gln Thr Ser Asp Ile Leu Ser His
   1 5 10 15
   Ser Met Lys Lys Thr Asp Thr Ser Met Ser Ile Val Thr Ala Glu Asn
   20 25 30
   Pro Tyr Lys Val Ala Val Val Gly Ser Gly Asn Trp Gly Thr Thr Ile
   35 40 45
   Ala Lys Val Val Ala Glu Asn Thr Lys Glu Lys Pro Glu Leu Phe Gln
   50 55 60
   Gly Arg Val Asp Met Trp Val Phe Glu Glu Gln Ile Asp Gly Thr Pro
   65 70 75 80
   Leu Thr Gln Ile Ile Asn Thr Lys His Gln Asn Val Lys Tyr Leu Pro
   85 90 95
   Asn Ile Asp Leu Pro Gly Asn Leu Val Ala Asn Pro Asp Leu Ile Ser
   100 105 110
   Thr Thr Lys Asp Ala Asp Val Ile Val Phe Asn Val Pro His Gln Phe
   115 120 125
   Leu Gly Arg Ile Val Ser Gln Met Lys Gly Gln Ile Lys Pro Asp Ala
   130 135 140
   Arg Ala Ile Ser Cys Leu Lys Gly Phe Glu Val Gly Pro Lys Gly Val
   145 150 155 160
   Gln Leu Leu Ser Asp Tyr Val Thr Gln Glu Leu Gly Ile Gln Cys Gly
   165 170 175
   Ala Leu Ser Gly Ala Asn Leu Ala Pro Glu Val Ala Lys Glu His Trp
   180 185 190
   Ser Glu Thr Thr Val Ala Tyr Gln Val Pro Asp Asp Phe Lys Gly Glu
   195 200 205
   Gly Lys Asp Ile Asp His Arg Val Leu Lys Gln Leu Phe His Arg Pro
   210 215 220
   Tyr Phe His Val Asn Val Ile Asp Asp Val Ala Gly Ile Ser Ile Ala
   225 230 235 240
   Gly Ala Leu Lys Asn Val Val Ala Leu Gly Cys Gly Phe Val Thr Gly
   245 250 255
   Leu Gly Trp Gly Asn Asn Ala Ala Ala Ala Ile Gln Arg Val Gly Leu
   260 265 270
   Gly Glu Ile Ile Lys Phe Gly Arg Met Phe Phe Pro Glu Ser Lys Val
   275 280 285
   Glu Thr Tyr Tyr Gln Glu Ser Ala Gly Val Ala Asp Leu Ile Thr Thr
   290 295 300
   Cys Ser Gly Gly Arg Asn Val Arg Val Ala Thr Glu Met Ala Lys Thr
   305 310 315 320
   Gly Lys Ser Gly Glu Gln Val Glu Lys Asp Ile Leu Asn Gly Gln Ser
   325 330 335
   Ala Gln Gly Leu Ile Thr Ala Lys Glu Val His Gln Trp Leu Glu Ser
   340 345 350
   Ser Gly His Thr Glu Glu Tyr Pro Leu Phe Glu Ala Val Tyr Gln Ile
   355 360 365
   Thr Tyr Glu Asn Val Pro Met Lys Glu Leu Pro Ser Met Ile Glu Glu
   370 375 380
   Leu Asp Ile Val Glu
   385
<210> 17
   <211> 8809
   <212> DNA
   <213> Künstliche Sequenz
<220>
   <223> Beschreibung der künstlichen Sequenz: expression vector pSUN-USP containing Saccharomyces G3PDH
<220>
   <221> misc_feature
   <222> (1017)..(2189)
   <223> coding for G3PDH
<400> 17
   aatattcaaa caaacacata cagcgcgact tatcatggac atacaaatgg acgaacggat 60
   aaaccttttc acgccctttt aaatatccga ttattctaat aaacgctctt ttctcttagg 120
   tttacccgcc aatatatcct gtcaaacact gatagtttaa actgaaggcg ggaaacgaca 180
   atcagatcta gtaggaaaca gctatgacca tgattacgcc aagcttgcat gcctgcaggt 240
   cgactctaga ctagtggatc cgatatcgcc cgggctcgag gtaccgagct cgaattcggc 300
   gcgccgagct cctcgagcaa atttacacat tgccactaaa cgtctaaacc cttgtaattt 360
   gtttttgttt tactatgtgt gttatgtatt tgatttgcga taaattttta tatttggtac 420
   taaatttata acacctttta tgctaacgtt tgccaacact tagcaatttg caagttgatt 480
   aattgattct aaattatttt tgtcttctaa atacatatac taatcaactg gaaatgtaaa 540
   tatttgctaa tatttctact ataggagaat taaagtgagt gaatatggta ccacaaggtt 600
   tggagattta attgttgcaa tgatgcatgg atggcatata caccaaacat tcaataattc 660
   ttgaggataa taatggtacc acacaagatt tgaggtgcat gaacgtcacg tggacaaaag 720
   gtttagtaat ttttcaagac aacaatgtta ccacacacaa gttttgaggt gcatgcatgg 780
   atgccctgtg gaaagtttaa aaatattttg gaaatgattt gcatggaagc catgtgtaaa 840
   accatgacat ccacttggag gatgcaataa tgaagaaaac tacaaattta catgcaacta 900
   gttatgcatg tagtctatat aatgaggatt ttgcaatact ttcattcata cacactcact 960
   aagttttaca cgattataat ttcttcatag ccagcccacc gcggtgggcg gccgccatgt 1020
   ctgctgctgc tgatagatta aacttaactt ccggccactt gaatgctggt agaaagagaa 1080
   gttcctcttc tgtttctttg aaggctgccg aaaagccttt caaggttact gtgattggat 1140
   ctggtaactg gggtactact attgccaagg tggttgccga aaattgtaag ggatacccag 1200
   aagttttcgc tccaatagta caaatgtggg tgttcgaaga agagatcaat ggtgaaaaat 1260
   tgactgaaat cataaatact agacatcaaa acgtgaaata cttgcctggc atcactctac 1320
   ccgacaattt ggttgctaat ccagacttga ttgattcagt caaggatgtc gacatcatcg 1380
   tcttcaacat tccacatcaa tttttgcccc gtatctgtag ccaattgaaa ggtcatgttg 1440
   attcacacgt cagagctatc tcctgtctaa agggttttga agttggtgct aaaggtgtcc 1500
   aattgctatc ctcttacatc actgaggaac taggtattca atgtggtgct ctatctggtg 1560
   ctaacattgc cactgaagtc gctcaagaac actggtctga aacaacagtt gcttaccaca 1620
   ttccaaagga tttcagaggc gagggcaagg acgtcgacca taaggttcta aaggccttgt 1680
   tccacagacc ttacttccac gttagtgtca tcgaagatgt tgctggtatc tccatctgtg 1740
   gtgctttgaa gaacgttgtt gccttaggtt gtggtttcgt cgaaggtcta ggctggggta 1800
   acaacgcttc tgctgccatc caaagagtcg gtttgggtga gatcatcaga ttcggtcaaa 1860
   tgtttttccc agaatctaga gaagaaacat actaccaaga gtctgctggt gttgctgatt 1920
   tgatcaccac ctgcgctggt ggtagaaacg tcaaggttgc taggctaatg gctacttctg 1980
   gtaaggacgc ctgggaatgt gaaaaggagt tgttgaatgg ccaatccgct caaggtttaa 2040
   ttacctgcaa agaagttcac gaatggttgg aaacatgtgg ctctgtcgaa gacttcccat 2100
   tatttgaagc cgtataccaa atcgtttaca acaactaccc aatgaagaac ctgccggaca 2160
   tgattgaaga attagatcta catgaagatt aggcggccgc ctgcagtcta gaaggcctcc 2220
   tgctttaatg agatatgcga gacgcctatg atcgcatgat atttgctttc aattctgttg 2280
   tgcacgttgt aaaaaacctg agcatgtgta gctcagatcc ttaccgccgg tttcggttca 2340
   ttctaatgaa tatatcaccc gttactatcg tatttttatg aataatattc tccgttcaat 2400
   ttactgattg tccgtcgacg aattcactgg ccgtcgtttt acaacgactc agagcttgac 2460
   aggaggcccg atctagtaac atagatgaca ccgcgcgcga taatttatcc tagtttgcgc 2520
   gctatatttt gttttctatc gcgtattaaa tgtataattg cgggactcta atcataaaaa 2580
   cccatctcat aaataacgtc atgcattaca tgttaattat tacatgctta acgtaattca 2640
   acagaaatta tatgataatc atcgcaagac cggcaacagg attcaatctt aagaaacttt 2700
   attgccaaat gtttgaacga tcggggatca tccgggtctg tggcgggaac tccacgaaaa 2760
   tatccgaacg cagcaagatc tagagcttgg gtcccgctca gaagaactcg tcaagaaggc 2820
   gatagaaggc gatgcgctgc gaatcgggag cggcgatacc gtaaagcacg aggaagcggt 2880
   cagcccattc gccgccaagc tcttcagcaa tatcacgggt agccaacgct atgtcctgat 2940
   agcggtccgc cacacccagc cggccacagt cgatgaatcc agaaaagcgg ccattttcca 3000
   ccatgatatt cggcaagcag gcatcgccat gtgtcacgac gagatcctcg ccgtcgggca 3060
   tgcgcgcctt gagcctggcg aacagttcgg ctggcgcgag cccctgatgc tcttcgtcca 3120
   gatcatcctg atcgacaaga ccggcttcca tccgagtacg tgctcgctcg atgcgatgtt 3180
   tcgcttggtg gtcgaatggg caggtagccg gatcaagcgt atgcagccgc cgcattgcat 3240
   cagccatgat ggatactttc tcggcaggag caaggtgaga tgacaggaga tcctgccccg 3300
   gcacttcgcc caatagcagc cagtcccttc ccgcttcagt gacaacgtcg agcacagctg 3360
   cgcaaggaac gcccgtcgtg gccagccacg atagccgcgc tgcctcgtcc tgcagttcat 3420
   tcagggcacc ggacaggtcg gtcttgacaa aaagaaccgg gcgcccctgc gctgacagcc 3480
   ggaacacggc ggcatcagag cagccgattg tctgttgtgc ccagtcatag ccgaatagcc 3540
   tctccaccca agcggccgga gaacctgcgt gcaatccatc ttgttcaatc atgcgaaacg 3600
   atccagatcc ggtgcagatt atttggattg agagtgaata tgagactcta attggatacc 3660
   gaggggaatt tatggaacgt cagtggagca tttttgacaa gaaatatttg ctagctgata 3720
   gtgaccttag gcgacttttg aacgcgcaat aatggtttct gacgtatgtg cttagctcat 3780
   taaactccag aaacccgcgg ctgagtggct ccttcaacgt tgcggttctg tcagttccaa 3840
   acgtaaaacg gcttgtcccg cgtcatcggc gggggtcata acgtgactcc cttaattctc 3900
   cgctcatgat cagattgtcg tttcccgcct tcagtttaaa ctatcagtgt ttgacaggat 3960
   cactgcttgg taataattgt cattagattg tttttatgca tagatgcact cgaaatcagc 4020
   caattttaga caagtatcaa acggatgtta attcagtaca ttaaagacgt ccgcaatgtg 4080
   ttattaagtt gtctaagcgt caatttgttt acaccacaat atatcctgcc accagccagc 4140
   caacagctcc ccgaccggca gctcggcaca aaatcaccac gcgtctaaaa aggtgatgtg 4200
   tatttgagta aaacagcttg cgtcatgcgg tcgctgcgta tatgatgcga tgagtaaata 4260
   aacaaatacg caaggggaac gcatgaaggt tatcgctgta cttaaccaga aaggcgggtc 4320
   aggcaagacg accatcgcaa cccatctagc ccgcgccctg caactcgccg gggccgatgt 4380
   tctgttagtc gattccgatc cccagggcag tgcccgcgat tgggcggccg tgcgggaaga 4440
   tcaaccgcta accgttgtcg gcatcgaccg cccgacgatt gaccgcgacg tgaaggccat 4500
   cggccggcgc gacttcgtag tgatcgacgg agcgccccag gcggcggact tggctgtgtc 4560
   cgcgatcaag gcagccgact tcgtgctgat tccggtgcag ccaagccctt acgacatatg 4620
   ggccaccgcc gacctggtgg agctggttaa gcagcgcatt gaggtcacgg atggaaggct 4680
   acaagcggcc tttgtcgtgt cgcgggcgat caaaggcacg cgcatcggcg gtgaggttgc 4740
   cgaggcgctg gccgggtacg agctgcccat tcttgagtcc cgtatcacgc agcgcgtgag 4800
   ctacccaggc actgccgccg ccggcacaac cgttcttgaa tcagaacccg agggcgacgc 4860
   tgcccgcgag gtccaggcgc tggccgctga aattaaatca aaactcattt gagttaatga 4920
   ggtaaagaga aaatgagcaa aagcacaaac acgctaagtg ccggccgtcc gagcgcacgc 4980
   agcagcaagg ctgcaacgtt ggccagcctg gcagacacgc cagccatgaa gcgggtcaac 5040
   tttcagttgc cggcggagga tcacaccaag ctgaagatgt acgcggtacg ccaaggcaag 5100
   accattaccg agctgctatc tgaatacatc gcgcagctac cagagtaaat gagcaaatga 5160
   ataaatgagt agatgaattt tagcggctaa aggaggcggc atggaaaatc aagaacaacc 5220
   aggcaccgac gccgtggaat gccccatgtg tggaggaacg ggcggttggc caggcgtaag 5280
   cggctgggtt gtctgccggc cctgcaatgg cactggaacc cccaagcccg aggaatcggc 5340
   gtgagcggtc gcaaaccatc cggcccggta caaatcggcg cggcgctggg tgatgacctg 5400
   gtggagaagt tgaaggccgc gcaggccgcc cagcggcaac gcatcgaggc agaagacgcc 5460
   ccggtgaatc gtggcaaggg gccgctgatc gaatccgcaa agaatcccgg caaccgccgg 5520
   cagccggtgc gccgtcgatt aggaagccgc ccaagggcga cgagcaacca gattttttcg 5580
   ttccgatgct ctatgacgtg ggcacccgcg atagtcgcag catcatggac gtggccgttt 5640
   tccgtctgtc gaagcgtgac cgacgagctg gcgaggtgat ccgctacgag cttccagacg 5700
   ggcacgtaga ggtttccgca gggccggccg gcatggccag tgtgtgggat tacgacctgg 5760
   tactgatggc ggtttcccat ctaaccgaat ccatgaaccg ataccgggaa gggaagggag 5820
   acaagcccgg ccgcgtgttc cgtccacacg ttgcggacgt actcaagttc tgccggcgag 5880
   ccgatggcgg aaagcagaaa gacgacctgg tagaaacctg cattcggtta aacaccacgc 5940
   acgttgccat gcagcgtacg aagaaggcca agaacggccg cctggtgacg gtatccgagg 6000
   gtgaagcctt gattagccgc tacaagatcg taaagagcga aaccgggcgg ccggagtaca 6060
   tcgagatcga gctagctgat tggatgtacc gcgagatcac agaaggcaag aacccggacg 6120
   tgctgacggt tcaccccgat tactttttga tcgatcccgg catcggccgt tttctctacc 6180
   gcctggcacg ccgcgccgca ggcaaggcag aagccagatg gttgttcaag acgatctacg 6240
   aacgcagtgg cagcgccgga gagttcaaga agttctgttt caccgtgcgc aagctgatcg 6300
   ggtcaaatga cctgccggag tacgatttga aggaggaggc ggggcaggct ggcccgatcc 6360
   tagtcatgcg ctaccgcaac ctgatcgagg gcgaagcatc cgccggttcc taatgtacgg 6420
   agcagatgct agggcaaatt gccctagcag gggaaaaagg tcgaaaaggt ctctttcctg 6480
   tggatagcac gtacattggg aacccaaagc cgtacattgg gaaccggaac ccgtacattg 6540
   ggaacccaaa gccgtacatt gggaaccggt cacacatgta agtgactgat ataaaagaga 6600
   aaaaaggcga tttttccgcc taaaactctt taaaacttat taaaactctt aaaacccgcc 6660
   tggcctgtgc ataactgtct ggccagcgca cagccgaaga gctgcaaaaa gcgcctaccc 6720
   ttcggtcgct gcgctcccta cgccccgccg cttcgcgtcg gcctatcgcg gcctatgcgg 6780
   tgtgaaatac cgcacagatg cgtaaggaga aaataccgca tcaggcgctc ttccgcttcc 6840
   tcgctcactg actcgctgcg ctcggtcgtt cggctgcggc gagcggtatc agctcactca 6900
   aaggcggtaa tacggttatc cacagaatca ggggataacg caggaaagaa catgtgagca 6960
   aaaggccagc aaaaggccag gaaccgtaaa aaggccgcgt tgctggcgtt tttccatagg 7020
   ctccgccccc ctgacgagca tcacaaaaat cgacgctcaa gtcagaggtg gcgaaacccg 7080
   acaggactat aaagatacca ggcgtttccc cctggaagct ccctcgtgcg ctctcctgtt 7140
   ccgaccctgc cgcttaccgg atacctgtcc gcctttctcc cttcgggaag cgtggcgctt 7200
   tctcatagct cacgctgtag gtatctcagt tcggtgtagg tcgttcgctc caagctgggc 7260
   tgtgtgcacg aaccccccgt tcagcccgac cgctgcgcct tatccggtaa ctatcgtctt 7320
   gagtccaacc cggtaagaca cgacttatcg ccactggcag cagccactgg taacaggatt 7380
   agcagagcga ggtatgtagg cggtgctaca gagttcttga agtggtggcc taactacggc 7440
   tacactagaa ggacagtatt tggtatctgc gctctgctga agccagttac cttcggaaaa 7500
   agagttggta gctcttgatc cggcaaacaa accaccgctg gtagcggtgg tttttttgtt 7560
   tgcaagcagc agattacgcg cagaaaaaaa ggatctcaag aagatccttt gatcttttct 7620
   acggggtctg acgctcagtg gaacgaaaac tcacgttaag ggattttggt catgcatgat 7680
   atatctccca atttgtgtag ggcttattat gcacgcttaa aaataataaa agcagacttg 7740
   acctgatagt ttggctgtga gcaattatgt gcttagtgca tctaacgctt gagttaagcc 7800
   gcgccgcgaa gcggcgtcgg cttgaacgaa tttctagcta gacattattt gccgactacc 7860
   ttggtgatct cgcctttcac gtagtggaca aattcttcca actgatctgc gcgcgaggcc 7920
   aagcgatctt cttcttgtcc aagataagcc tgtctagctt caagtatgac gggctgatac 7980
   tgggccggca ggcgctccat tgcccagtcg gcagcgacat ccttcggcgc gattttgccg 8040
   gttactgcgc tgtaccaaat gcgggacaac gtaagcacta catttcgctc atcgccagcc 8100
   cagtcgggcg gcgagttcca tagcgttaag gtttcattta gcgcctcaaa tagatcctgt 8160
   tcaggaaccg gatcaaagag ttcctccgcc gctggaccta ccaaggcaac gctatgttct 8220
   cttgcttttg tcagcaagat agccagatca atgtcgatcg tggctggctc gaagatacct 8280
   gcaagaatgt cattgcgctg ccattctcca aattgcagtt cgcgcttagc tggataacgc 8340
   cacggaatga tgtcgtcgtg cacaacaatg gtgacttcta cagcgcggag aatctcgctc 8400
   tctccagggg aagccgaagt ttccaaaagg tcgttgatca aagctcgccg cgttgtttca 8460
   tcaagcctta cggtcaccgt aaccagcaaa tcaatatcac tgtgtggctt caggccgcca 8520
   tccactgcgg agccgtacaa atgtacggcc agcaacgtcg gttcgagatg gcgctcgatg 8580
   acgccaacta cctctgatag ttgagtcgat acttcggcga tcaccgcttc ccccatgatg 8640
   tttaactttg ttttagggcg actgccctgc tgcgtaacat cgttgctgct ccataacatc 8700
   aaacatcgac ccacggcgta acgcgcttgc tgcttggatg cccgaggcat agactgtacc 8760
   ccaaaaaaac agtcataaca agccatgaaa accgccactg cgttccatg             8809
<210> 18
   <211> 26
   <212> DNA
   <213> Künstliche Sequenz
<220>
   <223> Beschreibung der künstlichen Sequenz: oligonucleotide primer
<400> 18
   actagtatgt ctgctgctgc tgatag 26
<210> 19
   <211> 26
   <212> DNA
   <213> Künstliche Sequenz
<220>
   <223> Beschreibung der künstlichen Sequenz: oligonucleotide primer
<400> 19
   ctcgagatct tcatgtagat ctaatt 26
<210> 20
   <211> 29
   <212> DNA
   <213> Künstliche Sequenz
<220>
   <223> Beschreibung der künstlichen Sequenz: oligonucleotide primer
<400> 20
   gcggccgcca tgtctgctgc tgctgatag 29
<210> 21
   <211> 28
   <212> DNA
   <213> Künstliche Sequenz
<220>
   <223> Beschreibung der künstlichen Sequenz: oligonucleotide primer
<400> 21
   gcggccgcat cttcatgtag atctaatt 28
<210> 22
   <211> 11
   <212> PRT
   <213> Künstliche Sequenz
<220>
   <223> Beschreibung der künstlichen Sequenz: Yeast G3PDH sequence motive
<220>
   <221> VARIANT
   <222> (8)
   <223> Thr
<400> 22
   Gly Ser Gly Asn Trp Gly Thr Ala Ile Ala Lys
   1               5                   10
<210> 23
   <211> 8
   <212> PRT
   <213> Künstliche Sequenz
<220>
   <223> Beschreibung der künstlichen Sequenz: Yeast G3PDH sequence motive
<220>
   <221> VARIANT
   <222> (2)
   <223> Gln
<400> 23
   His Glu Gln Asn Val Lys Tyr Leu
   1               5
<210> 24
   <211> 12
   <212> PRT
   <213> Künstliche Sequenz
<220>
   <223> Beschreibung der künstlichen Sequenz: Yeast G3PDH sequence motive
<220>
   <221> VARIANT
   <222> (1)
   <223> Asn
<220>
   <221> VARIANT
   <222> (2)
   <223> Val
<220>
   <221> VARIANT
   <222> (3)
   <223> Ile
<220>
   <221> VARIANT
   <222> (5)
   <223> Trp
<220>
   <221> VARIANT
   <222> (6)
   <223> Asn
<220>
   <221> VARIANT
   <222> (7)
   <223> Ile or Val
<220>
   <221> VARIANT
   <222> (12)
   <223> Leu or Ile
<400> 24
   Asp Ile Leu Val Phe Val Leu Pro His Gln Phe Val
    1               5                  10
<210> 25
   <211> 7
   <212> PRT
   <213> Künstliche Sequenz
<220>
   <223> Beschreibung der künstlichen Sequenz: Yeast G3PDH sequence motive
<220>
   <221> VARIANT
   <222> (1)
   <223> Gly
<220>
   <221> VARIANT
   <222> (2)
   <223> Val
<220>
   <221> VARIANT
   <222> (5)
   <223> Ile
<400> 25
   Ala Ile Ser Cys Leu Lys Gly
   1 5
<210> 26
   <211> 14
   <212> PRT
   <213> Künstliche Sequenz
<220>
   <223> Beschreibung der künstlichen Sequenz: Yeast G3PDH sequence motive
<220>
   <221> VARIANT
   <222> (3)
   <223> Ala
<220>
   <221> VARIANT
   <222> (9)
   <223> Ile or Val
<220>
   <221> VARIANT
   <222> (13)
   <223> Ile
<400> 26
   Cys Gly Val Leu Ser Gly Ala Asn Leu Ala Xaa Glu Val Ala
   1 5 10
<210> 27
   <211> 9
   <212> PRT
   <213> Künstliche Sequenz
<220>
   <223> Beschreibung der künstlichen Sequenz: Yeast G3PDH sequence motive
<220>
   <221> VARIANT
   <222> (1)
   <223> Val
<400> 27
   Leu Phe Xaa Arg Pro Tyr Phe Xaa Val
   1 5
<210> 28
   <211> 9
   <212> PRT
   <213> Künstliche Sequenz
<220>
   <223> Beschreibung der künstlichen Sequenz: Yeast G3PDH sequence motive
<220>
   <221> VARIANT
   <222> (2)
   <223> Met
<220>
   <221> VARIANT
   <222> (3)
   <223> Gly
<220>
   <221> VARIANT
   <222> (5)
   <223> Ile
<220>
   <221> VARIANT
   <222> (6)
   <223> Gln
<220>
   <221> VARIANT
   <222> (7)
   <223> Lys or Asn
<220>
   <221> VARIANT
   <222> (9)
   <223> Ser or Ala
<400> 28
   Gly Leu Leu Glu Met Ile Arg Phe Gly
   1 5
<210> 29
   <211> 16
   <212> PRT
   <213> Künstliche Sequenz
<220>
   <223> Beschreibung der künstlichen Sequenz: Yeast G3PDH sequence motive
<220>
   <221> VARIANT
   <222> (13)
   <223> Ile
<400> 29
   Gly Ser Gly Asn Trp Gly Thr Thr Ile Ala Lys Val Val Ala Glu Asn
   1 5 10 15
<210> 30
   <211> 11
   <212> PRT
   <213> Künstliche Sequenz
<220>
   <223> Beschreibung der künstlichen Sequenz: Yeast G3PDH sequence motive
<220>
   <221> VARIANT
   <222> (3)
   <223> Arg
<400> 30
   Asn Thr Lys His Gln Asn Val Lys Tyr Leu Pro
   1 5 10
<210> 31
   <211> 12
   <212> PRT
   <213> Künstliche Sequenz
<220>
   <223> Beschreibung der künstlichen Sequenz: Yeast G3PDH sequence motive
<220>
   <221> VARIANT
   <222> (2)
   <223> Val
<220>
   <221> VARIANT
   <222> (7)
   <223> Val
<400> 31
   Asp Ile Leu Val Phe Asn Ile Pro His Gln Phe Leu
   1 5 10
<210> 32
   <211> 10
   <212> PRT
   <213> Künstliche Sequenz
<220>
   <223> Beschreibung der künstlichen Sequenz: Yeast G3PDH sequence motive
<220>
   <221> VARIANT
   <222> (3)
   <223> Val
<400> 32
   Arg Ala Ile Ser Cys Leu Lys Gly Phe Glu
    1               5                  10
<210> 33
   <211> 14
   <212> PRT
   <213> Künstliche Sequenz
<220>
   <223> Beschreibung der künstlichen Sequenz: Yeast G3PDH sequence motive
<220>
   <221> VARIANT
   <222> (11)
   <223> Thr
<400> 33
   Cys Gly Ala Leu Ser Gly Ala Asn Leu Ala Pro Glu Val Ala
    1               5                  10
<210> 34
   <211> 9
   <212> PRT
   <213> Künstliche Sequenz
<220>
   <223> Beschreibung der künstlichen Sequenz: Yeast G3PDH sequence motive
<400> 34
   Leu Phe His Arg Pro Tyr Phe His Val
    1               5
<210> 35
   <211> 9
   <212> PRT
   <213> Künstliche Sequenz
<220>
   <223> Beschreibung der künstlichen Sequenz: Yeast G3PDH sequence motive
<220>
   <221> VARIANT
   <222> (7)
   <223> Arg
<400> 35
   Gly Leu Gly Glu Ile Ile Lys Phe Gly
   1 5
<210> 36
   <211> 13718
   <212> DNA
   <213> Künstliche Sequenz
<220>
   <223> Beschreibung der künstlichen Sequenz: expression vector pGPTV-gpd1
<220>
   <221> promoter
   <222> (10807)..(11951)
   <223> napin promoter
<220>
   <221> terminator
   <222> (13154)..(13408)
   <223> nos terminator
<220>
   <221> misc_feature
   <222> (11962)..(13137)
   <223> coding for yeast G3PDH (gpd1)
<400> 36
   gatctggcgc cggccagcga gacgagcaag attggccgcc gcccgaaacg atccgacagc 60
   gcgcccagca caggtgcgca ggcaaattgc accaacgcat acagcgccag cagaatgcca 120
   tagtgggcgg tgacgtcgtt cgagtgaacc agatcgcgca ggaggcccgg cagcaccggc 180
   ataatcaggc cgatgccgac agcgtcgagc gcgacagtgc tcagaattac gatcaggggt 240
   atgttgggtt tcacgtctgg cctccggacc agcctccgct ggtccgattg aacgcgcgga 300
   ttctttatca ctgataagtt ggtggacata ttatgtttat cagtgataaa gtgtcaagca 360
   tgacaaagtt gcagccgaat acagtgatcc gtgccgccct ggacctgttg aacgaggtcg 420
   gcgtagacgg tctgacgaca cgcaaactgg cggaacggtt gggggttcag cagccggcgc 480
   tttactggca cttcaggaac aagcgggcgc tgctcgacgc actggccgaa gccatgctgg 540
   cggagaatca tacgcattcg gtgccgagag ccgacgacga ctggcgctca tttctgatcg 600
   ggaatgcccg cagcttcagg caggcgctgc tcgcctaccg cgatggcgcg cgcatccatg 660
   ccggcacgcg accgggcgca ccgcagatgg aaacggccga cgcgcagctt cgcttcctct 720
   gcgaggcggg tttttcggcc ggggacgccg tcaatgcgct gatgacaatc agctacttca 780
   ctgttggggc cgtgcttgag gagcaggccg gcgacagcga tgccggcgag cgcggcggca 840
   ccgttgaaca ggctccgctc tcgccgctgt tgcgggccgc gatagacgcc ttcgacgaag 900
   ccggtccgga cgcagcgttc gagcagggac tcgcggtgat tgtcgatgga ttggcgaaaa 960
   ggaggctcgt tgtcaggaac gttgaaggac cgagaaaggg tgacgattga tcaggaccgc 1020
   tgccggagcg caacccactc actacagcag agccatgtag acaacatccc ctcccccttt 1080
   ccaccgcgtc agacgcccgt agcagcccgc tacgggcttt ttcatgccct gccctagcgt 1140
   ccaagcctca cggccgcgct cggcctctct ggcggccttc tggcgctctt ccgcttcctc 1200
   gctcactgac tcgctgcgct cggtcgttcg gctgcggcga gcggtatcag ctcactcaaa 1260
   ggcggtaata cggttatcca cagaatcagg ggataacgca ggaaagaaca tgtgagcaaa 1320
   aggccagcaa aaggccagga accgtaaaaa ggccgcgttg ctggcgtttt tccataggct 1380
   ccgcccccct gacgagcatc acaaaaatcg acgctcaagt cagaggtggc gaaacccgac 1440
   aggactataa agataccagg cgtttccccc tggaagctcc ctcgtgcgct ctcctgttcc 1500
   gaccctgccg cttaccggat acctgtccgc ctttctccct tcgggaagcg tggcgctttt 1560
   ccgctgcata accctgcttc ggggtcatta tagcgatttt ttcggtatat ccatcctttt 1620
   tcgcacgata tacaggattt tgccaaaggg ttcgtgtaga ctttccttgg tgtatccaac 1680
   ggcgtcagcc gggcaggata ggtgaagtag gcccacccgc gagcgggtgt tccttcttca 1740
   ctgtccctta ttcgcacctg gcggtgctca acgggaatcc tgctctgcga ggctggccgg 1800
   ctaccgccgg cgtaacagat gagggcaagc ggatggctga tgaaaccaag ccaaccagga 1860
   agggcagccc acctatcaag gtgtactgcc ttccagacga acgaagagcg attgaggaaa 1920
   aggcggcggc ggccggcatg agcctgtcgg cctacctgct ggccgtcggc cagggctaca 1980
   aaatcacggg cgtcgtggac tatgagcacg tccgcgagct ggcccgcatc aatggcgacc 2040
   tgggccgcct gggcggcctg ctgaaactct ggctcaccga cgacccgcgc acggcgcggt 2100
   tcggtgatgc cacgatcctc gccctgctgg cgaagatcga agagaagcag gacgagcttg 2160
   gcaaggtcat gatgggcgtg gtccgcccga gggcagagcc atgacttttt tagccgctaa 2220
   aacggccggg gggtgcgcgt gattgccaag cacgtcccca tgcgctccat caagaagagc 2280
   gacttcgcgg agctggtgaa gtacatcacc gacgagcaag gcaagaccga gcgcctttgc 2340
   gacgctcacc gggctggttg ccctcgccgc tgggctggcg gccgtctatg gccctgcaaa 2400
   cgcgccagaa acgccgtcga agccgtgtgc gagacaccgc ggccgccggc gttgtggata 2460
   cctcgcggaa aacttggccc tcactgacag atgaggggcg gacgttgaca cttgaggggc 2520
   cgactcaccc ggcgcggcgt tgacagatga ggggcaggct cgatttcggc cggcgacgtg 2580
   gagctggcca gcctcgcaaa tcggcgaaaa cgcctgattt tacgcgagtt tcccacagat 2640
   gatgtggaca agcctgggga taagtgccct gcggtattga cacttgaggg gcgcgactac 2700
   tgacagatga ggggcgcgat ccttgacact tgaggggcag agtgctgaca gatgaggggc 2760
   gcacctattg acatttgagg ggctgtccac aggcagaaaa tccagcattt gcaagggttt 2820
   ccgcccgttt ttcggccacc gctaacctgt cttttaacct gcttttaaac caatatttat 2880
   aaaccttgtt tttaaccagg gctgcgccct gtgcgcgtga ccgcgcacgc cgaagggggg 2940
   tgccccccct tctcgaaccc tcccggcccg ctaacgcggg cctcccatcc ccccaggggc 3000
   tgcgcccctc ggccgcgaac ggcctcaccc caaaaatggc agcgctggca gtccttgcca 3060
   ttgccgggat cggggcagta acgggatggg cgatcagccc gagcgcgacg cccggaagca 3120
   ttgacgtgcc gcaggtgctg gcatcgacat tcagcgacca ggtgccgggc agtgagggcg 3180
   gcggcctggg tggcggcctg cccttcactt cggccgtcgg ggcattcacg gacttcatgg 3240
   cggggccggc aatttttacc ttgggcattc ttggcatagt ggtcgcgggt gccgtgctcg 3300
   tgttcggggg tgcgataaac ccagcgaacc atttgaggtg ataggtaaga ttataccgag 3360
   gtatgaaaac gagaattgga cctttacaga attactctat gaagcgccat atttaaaaag 3420
   ctaccaagac gaagaggatg aagaggatga ggaggcagat tgccttgaat atattgacaa 3480
   tactgataag ataatatatc ttttatatag aagatatcgc cgtatgtaag gatttcaggg 3540
   ggcaaggcat aggcagcgcg cttatcaata tatctataga atgggcaaag cataaaaact 3600
   tgcatggact aatgcttgaa acccaggaca ataaccttat agcttgtaaa ttctatcata 3660
   attgggtaat gactccaact tattgatagt gttttatgtt cagataatgc ccgatgactt 3720
   tgtcatgcag ctccaccgat tttgagaacg acagcgactt ccgtcccagc cgtgccaggt 3780
   gctgcctcag attcaggtta tgccgctcaa ttcgctgcgt atatcgcttg ctgattacgt 3840
   gcagctttcc cttcaggcgg gattcataca gcggccagcc atccgtcatc catatcacca 3900
   cgtcaaaggg tgacagcagg ctcataagac gccccagcgt cgccatagtg cgttcaccga 3960
   atacgtgcgc aacaaccgtc ttccggagac tgtcatacgc gtaaaacagc cagcgctggc 4020
   gcgatttagc cccgacatag ccccactgtt cgtccatttc cgcgcagacg atgacgtcac 4080
   tgcccggctg tatgcgcgag gttaccgact gcggcctgag ttttttaagt gacgtaaaat 4140
   cgtgttgagg ccaacgccca taatgcgggc tgttgcccgg catccaacgc cattcatggc 4200
   catatcaatg attttctggt gcgtaccggg ttgagaagcg gtgtaagtga actgcagttg 4260
   ccatgtttta cggcagtgag agcagagata gcgctgatgt ccggcggtgc ttttgccgtt 4320
   acgcaccacc ccgtcagtag ctgaacagga gggacagctg atagacacag aagccactgg 4380
   agcacctcaa aaacaccatc atacactaaa tcagtaagtt ggcagcatca cccataattg 4440
   tggtttcaaa atcggctccg tcgatactat gttatacgcc aactttgaaa acaactttga 4500
   aaaagctgtt ttctggtatt taaggtttta gaatgcaagg aacagtgaat tggagttcgt 4560
   cttgttataa ttagcttctt ggggtatctt taaatactgt agaaaagagg aaggaaataa 4620
   taaatggcta aaatgagaat atcaccggaa ttgaaaaaac tgatcgaaaa ataccgctgc 4680
   gtaaaagata cggaaggaat gtctcctgct aaggtatata agctggtggg agaaaatgaa 4740
   aacctatatt taaaaatgac ggacagccgg tataaaggga ccacctatga tgtggaacgg 4800
   gaaaaggaca tgatgctatg gctggaagga aagctgcctg ttccaaaggt cctgcacttt 4860
   gaacggcatg atggctggag caatctgctc atgagtgagg ccgatggcgt cctttgctcg 4920
   gaagagtatg aagatgaaca aagccctgaa aagattatcg agctgtatgc ggagtgcatc 4980
   aggctctttc actccatcga catatcggat tgtccctata cgaatagctt agacagccgc 5040
   ttagccgaat tggattactt actgaataac gatctggccg atgtggattg cgaaaactgg 5100
   gaagaagaca ctccatttaa agatccgcgc gagctgtatg attttttaaa gacggaaaag 5160
   cccgaagagg aacttgtctt ttcccacggc gacctgggag acagcaacat ctttgtgaaa 5220
   gatggcaaag taagtggctt tattgatctt gggagaagcg gcagggcgga caagtggtat 5280
   gacattgcct tctgcgtccg gtcgatcagg gaggatatcg gggaagaaca gtatgtcgag 5340
   ctattttttg acttactggg gatcaagcct gattgggaga aaataaaata ttatatttta 5400
   ctggatgaat tgttttagta cctagatgtg gcgcaacgat gccggcgaca agcaggagcg 5460
   caccgacttc ttccgcatca agtgttttgg ctctcaggcc gaggcccacg gcaagtattt 5520
   gggcaagggg tcgctggtat tcgtgcaggg caagattcgg aataccaagt acgagaagga 5580
   cggccagacg gtctacggga ccgacttcat tgccgataag gtggattatc tggacaccaa 5640
   ggcaccaggc gggtcaaatc aggaataagg gcacattgcc ccggcgtgag tcggggcaat 5700
   cccgcaagga gggtgaatga atcggacgtt tgaccggaag gcatacaggc aagaactgat 5760
   cgacgcgggg ttttccgccg aggatgccga aaccatcgca agccgcaccg tcatgcgtgc 5820
   gccccgcgaa accttccagt ccgtcggctc gatggtccag caagctacgg ccaagatcga 5880
   gcgcgacagc gtgcaactgg ctccccctgc cctgcccgcg ccatcggccg ccgtggagcg 5940
   ttcgcgtcgt ctcgaacagg aggcggcagg tttggcgaag tcgatgacca tcgacacgcg 6000
   aggaactatg acgaccaaga agcgaaaaac cgccggcgag gacctggcaa aacaggtcag 6060
   cgaggccaag caggccgcgt tgctgaaaca cacgaagcag cagatcaagg aaatgcagct 6120
   ttccttgttc gatattgcgc cgtggccgga cacgatgcga gcgatgccaa acgacacggc 6180
   ccgctctgcc ctgttcacca cgcgcaacaa gaaaatcccg cgcgaggcgc tgcaaaacaa 6240
   ggtcattttc cacgtcaaca aggacgtgaa gatcacctac accggcgtcg agctgcgggc 6300
   cgacgatgac gaactggtgt ggcagcaggt gttggagtac gcgaagcgca cccctatcgg 6360
   cgagccgatc accttcacgt tctacgagct ttgccaggac ctgggctggt cgatcaatgg 6420
   ccggtattac acgaaggccg aggaatgcct gtcgcgccta caggcgacgg cgatgggctt 6480
   cacgtccgac cgcgttgggc acctggaatc ggtgtcgctg ctgcaccgct tccgcgtcct 6540
   ggaccgtggc aagaaaacgt cccgttgcca ggtcctgatc gacgaggaaa tcgtcgtgct 6600
   gtttgctggc gaccactaca cgaaattcat atgggagaag taccgcaagc tgtcgccgac 6660
   ggcccgacgg atgttcgact atttcagctc gcaccgggag ccgtacccgc tcaagctgga 6720
   aaccttccgc ctcatgtgcg gatcggattc cacccgcgtg aagaagtggc gcgagcaggt 6780
   cggcgaagcc tgcgaagagt tgcgaggcag cggcctggtg gaacacgcct gggtcaatga 6840
   tgacctggtg cattgcaaac gctagggcct tgtggggtca gttccggctg ggggttcagc 6900
   agccagcgct ttactggcat ttcaggaaca agcgggcact gctcgacgca cttgcttcgc 6960
   tcagtatcgc tcgggacgca cggcgcgctc tacgaactgc cgataaacag aggattaaaa 7020
   ttgacaattg tgattaaggc tcagattcga cggcttggag cggccgacgt gcaggatttc 7080
   cgcgagatcc gattgtcggc cctgaagaaa gctccagaga tgttcgggtc cgtttacgag 7140
   cacgaggaga aaaagcccat ggaggcgttc gctgaacggt tgcgagatgc cgtggcattc 7200
   ggcgcctaca tcgacggcga gatcattggg ctgtcggtct tcaaacagga ggacggcccc 7260
   aaggacgctc acaaggcgca tctgtccggc gttttcgtgg agcccgaaca gcgaggccga 7320
   ggggtcgccg gtatgctgct gcgggcgttg ccggcgggtt tattgctcgt gatgatcgtc 7380
   cgacagattc caacgggaat ctggtggatg cgcatcttca tcctcggcgc acttaatatt 7440
   tcgctattct ggagcttgtt gtttatttcg gtctaccgcc tgccgggcgg ggtcgcggcg 7500
   acggtaggcg ctgtgcagcc gctgatggtc gtgttcatct ctgccgctct gctaggtagc 7560
   ccgatacgat tgatggcggt cctgggggct atttgcggaa ctgcgggcgt ggcgctgttg 7620
   gtgttgacac caaacgcagc gctagatcct gtcggcgtcg cagcgggcct ggcgggggcg 7680
   gtttccatgg cgttcggaac cgtgctgacc cgcaagtggc aacctcccgt gcctctgctc 7740
   acctttaccg cctggcaact ggcggccgga ggacttctgc tcgttccagt agctttagtg 7800
   tttgatccgc caatcccgat gcctacagga accaatgttc tcggcctggc gtggctcggc 7860
   ctgatcggag cgggtttaac ctacttcctt tggttccggg ggatctcgcg actcgaacct 7920
   acagttgttt ccttactggg ctttctcagc cccagatctg gggtcgatca gccggggatg 7980
   catcaggccg acagtcggaa cttcgggtcc ccgacctgta ccattcggtg agcaatggat 8040
   aggggagttg atatcgtcaa cgttcacttc taaagaaata gcgccactca gcttcctcag 8100
   cggctttatc cagcgatttc ctattatgtc ggcatagttc tcaagatcga cagcctgtca 8160
   cggttaagcg agaaatgaat aagaaggctg ataattcgga tctctgcgag ggagatgata 8220
   tttgatcaca ggcagcaacg ctctgtcatc gttacaatca acatgctacc ctccgcgaga 8280
   tcatccgtgt ttcaaacccg gcagcttagt tgccgttctt ccgaatagca tcggtaacat 8340
   gagcaaagtc tgccgcctta caacggctct cccgctgacg ccgtcccgga ctgatgggct 8400
   gcctgtatcg agtggtgatt ttgtgccgag ctgccggtcg gggagctgtt ggctggctgg 8460
   tggcaggata tattgtggtg taaacaaatt gacgcttaga caacttaata acacattgcg 8520
   gacgttttta atgtactggg gtggtttttc ttttcaccag tgagacgggc aacagctgat 8580
   tgcccttcac cgcctggccc tgagagagtt gcagcaagcg gtccacgctg gtttgcccca 8640
   gcaggcgaaa atcctgtttg atggtggttc cgaaatcggc aaaatccctt ataaatcaaa 8700
   agaatagccc gagatagggt tgagtgttgt tccagtttgg aacaagagtc cactattaaa 8760
   gaacgtggac tccaacgtca aagggcgaaa aaccgtctat cagggcgatg gcccactacg 8820
   tgaaccatca cccaaatcaa gttttttggg gtcgaggtgc cgtaaagcac taaatcggaa 8880
   ccctaaaggg agcccccgat ttagagcttg acggggaaag ccggcgaacg tggcgagaaa 8940
   ggaagggaag aaagcgaaag gagcgggcgc cattcaggct gcgcaactgt tgggaagggc 9000
   gatcggtgcg ggcctcttcg ctattacgcc agctggcgaa agggggatgt gctgcaaggc 9060
   gattaagttg ggtaacgcca gggttttccc agtcacgacg ttgtaaaacg acggccagtg 9120
   aattaattcc catcttgaaa gaaatatagt ttaaatattt attgataaaa taacaagtca 9180
   ggtattatag tccaagcaaa aacataaatt tattgatgca agtttaaatt cagaaatatt 9240
   tcaataactg attatatcag ctggtacatt gccgtagatg aaagactgag tgcgatatta 9300
   tgtgtaatac ataaattgat gatatagcta gcttagctca tcgggggatc cgtcgaagct 9360
   agcttgggtc ccgctcagaa gaactcgtca agaaggcgat agaaggcgat gcgctgcgaa 9420
   tcgggagcgg cgataccgta aagcacgagg aagcggtcag cccattcgcc gccaagctct 9480
   tcagcaatat cacgggtagc caacgctatg tcctgatagc ggtccgccac acccagccgg 9540
   ccacagtcga tgaatccaga aaagcggcca ttttccacca tgatattcgg caagcaggca 9600
   tcgccatggg tcacgacgag atcctcgccg tcgggcatgc gcgccttgag cctggcgaac 9660
   agttcggctg gcgcgagccc ctgatgctct tcgtccagat catcctgatc gacaagaccg 9720
   gcttccatcc gagtacgtgc tcgctcgatg cgatgtttcg cttggtggtc gaatgggcag 9780
   gtagccggat caagcgtatg cagccgccgc attgcatcag ccatgatgga tactttctcg 9840
   gcaggagcaa ggtgagatga caggagatcc tgccccggca cttcgcccaa tagcagccag 9900
   tcccttcccg cttcagtgac aacgtcgagc acagctgcgc aaggaacgcc cgtcgtggcc 9960
   agccacgata gccgcgctgc ctcgtcctgc agttcattca gggcaccgga caggtcggtc 10020
   ttgacaaaaa gaaccgggcg cccctgcgct gacagccgga acacggcggc atcagagcag 10080
   ccgattgtct gttgtgccca gtcatagccg aatagcctct ccacccaagc ggccggagaa 10140
   cctgcgtgca atccatcttg ttcaatccaa gctcccatgg gccctcgact agagtcgaga 10200
   tctggattga gagtgaatat gagactctaa ttggataccg aggggaattt atggaacgtc 10260
   agtggagcat ttttgacaag aaatatttgc tagctgatag tgaccttagg cgacttttga 10320
   acgcgcaata atggtttctg acgtatgtgc ttagctcatt aaactccaga aacccgcggc 10380
   tgagtggctc cttcaacgtt gcggttctgt cagttccaaa cgtaaaacgg cttgtcccgc 10440
   gtcatcggcg gggtcataac cgtgactccc ttaattctcc gctcatgatc ttgatcccct 10500
   gcgccatcag atccttggcg gcaagaaagc catccagttt actttgcagg gcttcccaac 10560
   cttaccagag ggcgccccag ctggcaattc cggttcgctt gctgtccata aaaccgccca 10620
   gtctagctat cgccatgtaa gcccactgca agctacctgc tttctctttg cgcttgcgtt 10680
   ttcccttgtc cagatagccc agtagctgac attcatccgg ggtcagcacc gtttctgcgg 10740
   actggctttc tacgtgttcc gcttccttta gcagcccttg cgccctgagt gcttgcggca 10800
   gcgtgaagct ttcttcatcg gtgattgatt cctttaaaga cttatgtttc ttatcttgct 10860
   tctgaggcaa gtattcagtt accagttacc acttatattc tggactttct gactgcatcc 10920
   tcatttttcc aacattttaa atttcactat tggctgaatg cttcttcttt gaggaagaaa 10980
   caattcagat ggcagaaatg tatcaaccaa tgcatatata caaatgtacc tcttgttctc 11040
   aaaacatcta tcggatggtt ccatttgctt tgtcatccaa ttagtgacta ctttatatta 11100
   ttcactcctc tttattacta ttttcatgcg aggttgccat gtacattata tttgtaagga 11160
   ttgacgctat tgagcgtttt tcttcaattt tctttatttt agacatgggt atgaaatgtg 11220
   tgttagagtt gggttgaatg agatatacgt tcaagtgaat ggcataccgt tctcgagtaa 11280
   ggatgaccta cccattcttg agacaaatgt tacattttag tatcagagta aaatgtgtac 11340
   ctataactca aattcgattg acatgtatcc attcaacata aaattaaacc agcctgcacc 11400
   tgcatccaca tttcaagtat tttcaaaccg ttcggctcct atccaccggg tgtaacaaga 11460
   cggattccga atttggaaga ttttgactca aattcccaat ttatattgac cgtgactaaa 11520
   tcaactttaa cttctataat tctgattaag ctcccaattt atattcccaa cggcactacc 11580
   tccaaaattt atagactctc atcccctttt aaaccaactt agtaaacgtt ttttttttta 11640
   attttatgaa gttaagtttt taccttgttt ttaaaaagaa tcgttcataa gatgccatgc 11700
   cagaacatta gctacacgtt acacatagca tgcagccgcg gagaattgtt tttcttcgcc 11760
   acttgtcact cccttcaaac acctaagagc ttctctctca cagcacacac atacaatcac 11820
   atgcgtgcat gcattattac acgtgatcgc catgcaaatc tcctttatag cctataaatt 11880
   aactcatccg cttcactctt tactcaaacc aaaactcatc aatacaaaca agattaaaaa 11940
   catacacgag gatccactag tatgtctgct gctgctgata gattaaactt aacttccggc 12000
   cacttgaatg ctggtagaaa gagaagttcc tcttctgttt ctttgaaggc tgccgaaaag 12060
   cctttcaagg ttactgtgat tggatctggt aactggggta ctactattgc caaggtggtt 12120
   gccgaaaatt gtaagggata cccagaagtt ttcgctccaa tagtacaaat gtgggtgttc 12180
   gaagaagaga tcaatggtga aaaattgact gaaatcataa atactagaca tcaaaacgtg 12240
   aaatacttgc ctggcatcac tctacccgac aatttggttg ctaatccaga cttgattgat 12300
   tcagtcaagg atgtcgacat catcgtcttc aacattccac atcaattttt gccccgtatc 12360
   tgtagccaat tgaaaggtca tgttgattca cacgtcagag ctatctcctg tctaaagggt 12420
   tttgaagttg gtgctaaagg tgtccaattg ctatcctctt acatcactga ggaactaggt 12480
   attcaatgtg gtgctctatc tggtgctaac attgccactg aagtcgctca agaacactgg 12540
   tctgaaacaa cagttgctta ccacattcca aaggatttca gaggcgaggg caaggacgtc 12600
   gaccataagg ttctaaaggc cttgttccac agaccttact tccacgttag tgtcatcgaa 12660
   gatgttgctg gtatctccat ctgtggtgct ttgaagaacg ttgttgcctt aggttgtggt 12720
   ttcgtcgaag gtctaggctg gggtaacaac gcttctgctg ccatccaaag agtcggtttg 12780
   ggtgagatca tcagattcgg tcaaatgttt ttcccagaat ctagagaaga aacatactac 12840
   caagagtctg ctggtgttgc tgatttgatc accacctgcg ctggtggtag aaacgtcaag 12900
   gttgctaggc taatggctac ttctggtaag gacgcctggg aatgtgaaaa ggagttgttg 12960
   aatggccaat ccgctcaagg tttaattacc tgcaaagaag ttcacgaatg gttggaaaca 13020
   tgtggctctg tcgaagactt cccattattt gaagccgtat accaaatcgt ttacaacaac 13080
   tacccaatga agaacctgcc ggacatgatt gaagaattag atctacatga agattagctc 13140
   gacgaatttc cccgatcgtt caaacatttg gcaataaagt ttcttaagat tgaatcctgt 13200
   tgccggtctt gcgatgatta tcatataatt tctgttgaat tacgttaagc atgtaataat 13260
   taacatgtaa tgcatgacgt tatttatgag atgggttttt atgattagag tcccgcaatt 13320
   atacatttaa tacgcgatag aaaacaaaat atagcgcgca aactaggata aattatcgcg 13380
   cgcggtgtca tctatgttac tagatcggga attcagatcg gctgagtggc tccttcaacg 13440
   ttgcggntct gtcagtncca aacgtaaaac gggttggtcc gcggnatcgg gcgggggccc 13500
   ttaaccgtgn actnccntna ttnctccggc ttcantgnnn agaattggnc ntttccccgn 13560
   cntcagttta aactatcagg tgtttgacag gatatatttg gcgggtaaac ctaaganaaa 13620
   agagcgttta ttagaataat cggatattta aaagggccgn gaaaaggttt atcccttccg 13680
   tccatttgta tgngcatgcc naccaccagg gttcccca                         13718
<210> 37
   <211> 1254
   <212> DNA
   <213> Emericella nidulans
<220>
   <221> CDS
   <222> (1)..(1251)
   <223> coding for G3PDH
<400> 37
   atg ggc tct ctt gga ccg tat aag caa aag cac aag gtg act gtg gtg     48
   Met Gly Ser Leu Gly Pro Tyr Lys Gln Lys His Lys Val Thr Val Val
   1               5                   10                  15
   gga tcg ggt aac tgg ggc acc gct ata gcc aaa atc gtc gcc gag aat     96
   Gly Ser Gly Asn Trp Gly Thr Ala Ile Ala Lys Ile Val Ala Glu Asn
               20                  25                  30
   act gcc agc aac cct gcg gtc ttt gag aag gat gtt cag atg tgg gtt     144
   Thr Ala Ser Asn Pro Ala Val Phe Glu Lys Asp Val Gln Met Trp Val
           35                  40                  45
   ttc gag gaa aag gtc gag att ccg aaa tcg tcg aag cat tat gat cct     192
   Phe Glu Glu Lys Val Glu Ile Pro Lys Ser Ser Lys His Tyr Asp Pro
       50                  55                  60
   gcc tct tct ctt tgc cag ggc ccg cag aat ctg aca gat att atc aac     240
   Ala Ser Ser Leu Cys Gln Gly Pro Gln Asn Leu Thr Asp Ile Ile Asn
   65                  70                  75                  80
   cat acc cat gag aat atc aag tac ctc ccc gga att acc ctt ccg gaa     288
   His Thr His Glu Asn Ile Lys Tyr Leu Pro Gly Ile Thr Leu Pro Glu
                   85                  90                  95
   aac ttg att gcc aat cca tcg cta gtc gac gcg gtt caa gac agc act     336
   Asn Leu Ile Ala Asn Pro Ser Leu Val Asp Ala Val Gln Asp Ser Thr
               100                 105                 110
   atc ctc gtc ttc aac cta ccc cat caa ttc atc atc aat att tgt gaa     384
   Ile Leu Val Phe Asn Leu Pro His Gln Phe Ile Ile Asn Ile Cys Glu
           115                 120                 125
   cag atc aag ggc aag att gtc cca tac gcg cgt gga att tct tgc ata     432
   Gln Ile Lys Gly Lys Ile Val Pro Tyr Ala Arg Gly Ile Ser Cys Ile
       130                 135                 140
   aag ggc gtg gat gtg aat gag gaa gga gtc cac ctg ttt tcc gaa aca     480
   Lys Gly Val Asp Val Asn Glu Glu Gly Val His Leu Phe Ser Glu Thr
   145                 150                 155                 160
   att gga aag att ctc ggg atc tac tgt ggc gcc ctg tcc ggt gcc aac     528
   Ile Gly Lys Ile Leu Gly Ile Tyr Cys Gly Ala Leu Ser Gly Ala Asn
                   165                 170                 175
   atc gcg aat gag gtc gcc cag gaa aag tgg tcc gag tct agc att ggt     576
   Ile Ala Asn Glu Val Ala Gln Glu Lys Trp Ser Glu Ser Ser Ile Gly
               180                 185                 190
   tat gat cca ccg cat ttt gac tct aaa gcc cct tct cct ccc aac cga     624
   Tyr Asp Pro Pro His Phe Asp Ser Lys Ala Pro Ser Pro Pro Asn Arg
           195                 200                 205
   tcc cct tcc gca tcg act gac aat atc ctg cac ttc gag cac aaa gac     672
   Ser Pro Ser Ala Ser Thr Asp Asn Ile Leu His Phe Glu His Lys Asp
       210                 215                 220
   gtt tcg ggt caa ctt tcg cgg gta aag cta cag gct cta cct tcc gaa     720
   Val Ser Gly Gln Leu Ser Arg Val Lys Leu Gln Ala Leu Pro Ser Glu
   225                 230                 235                 240
   ttt cct ccc atc gac cat gcc ctt ctc aag tcg cta ttc cac cgt cct     768
   Phe Pro Pro Ile Asp His Ala Leu Leu Lys Ser Leu Phe His Arg Pro
                   245                 250                 255
   tac ttc cat att ggt gtg gta agt gac gtc gca ggt gtt tcg tta gga     816
   Tyr Phe His Ile Gly Val Val Ser Asp Val Ala Gly Val Ser Leu Gly
               260                 265                 270
   ggt gcc ctt aag aat gtc gtt gct gtc gcg gca ggg tgg gtt gtg ggc     864
   Gly Ala Leu Lys Asn Val Val Ala Val Ala Ala Gly Trp Val Val Gly
           275                 280                 285
   aaa gga tgg gga gac aat gcg aag gct gca att atg cga gtt ggg ctt     912
   Lys Gly Trp Gly Asp Asn Ala Lys Ala Ala Ile Met Arg Val Gly Leu
       290                 295                 300
   ttg gaa atg gtg aag ttc ggc gaa cag ttt ttc ggt gct acc atc aac     960
   Leu Glu Met Val Lys Phe Gly Glu Gln Phe Phe Gly Ala Thr Ile Asn
   305                 310                 315                 320
   act cgc acc ttc act gaa gaa agt gct ggt gtt gcc gat cta atc acg    1008
   Thr Arg Thr Phe Thr Glu Glu Ser Ala Gly Val Ala Asp Leu Ile Thr
                   325                 330                 335
   agt tgc agt ggc gga cga aac ttc cgc tgc gca aag ctt agc att gaa    1056
   Ser Cys Ser Gly Gly Arg Asn Phe Arg Cys Ala Lys Leu Ser Ile Glu
               340                 345                 350
   aga aac cag ccg att gag aaa atc gag gag aca gag ttg aac ggc cag    1104
   Arg Asn Gln Pro Ile Glu Lys Ile Glu Glu Thr Glu Leu Asn Gly Gln
           355                 360                 365
   aag ctg caa ggc act ttg act gca gtc gaa gtc aac agt ttc ttg aaa    1152
   Lys Leu Gln Gly Thr Leu Thr Ala Val Glu Val Asn Ser Phe Leu Lys
       370                 375                 380
   aag caa ggt tta gaa gaa gag ttc cca ttg ttt act gca gtc tac cga    1200
   Lys Gln Gly Leu Glu Glu Glu Phe Pro Leu Phe Thr Ala Val Tyr Arg
   385                 390                 395                 400
   gtt ctt caa ggc acc atg tct gtg gac gag att cct tct ttc att gag    1248
   Val Leu Gln Gly Thr Met Ser Val Asp Glu Ile Pro Ser Phe Ile Glu
                   405                 410                 415
   cgg taa                                                            1254
   Arg
<210> 38
   <211> 417
   <212> PRT
   <213> Emericella nidulans
<400> 38
   Met Gly Ser Leu Gly Pro Tyr Lys Gln Lys His Lys Val Thr Val Val
   1               5                   10                  15
   Gly Ser Gly Asn Trp Gly Thr Ala Ile Ala Lys Ile Val Ala Glu Asn
               20                  25                  30
   Thr Ala Ser Asn Pro Ala Val Phe Glu Lys Asp Val Gln Met Trp Val
           35                  40                  45
   Phe Glu Glu Lys Val Glu Ile Pro Lys Ser Ser Lys His Tyr Asp Pro
       50                  55                  60
   Ala Ser Ser Leu Cys Gln Gly Pro Gln Asn Leu Thr Asp Ile Ile Asn
   65                  70                  75                  80
   His Thr His Glu Asn Ile Lys Tyr Leu Pro Gly Ile Thr Leu Pro Glu
                   85                  90                  95
   Asn Leu Ile Ala Asn Pro Ser Leu Val Asp Ala Val Gln Asp Ser Thr
               100                 105                 110
   Ile Leu Val Phe Asn Leu Pro His Gln Phe Ile Ile Asn Ile Cys Glu
           115                 120                 125
   Gln Ile Lys Gly Lys Ile Val Pro Tyr Ala Arg Gly Ile Ser Cys Ile
       130                 135                 140
   Lys Gly Val Asp Val Asn Glu Glu Gly Val His Leu Phe Ser Glu Thr
   145                 150                 155                 160
   Ile Gly Lys Ile Leu Gly Ile Tyr Cys Gly Ala Leu Ser Gly Ala Asn
                   165                 170                 175
   Ile Ala Asn Glu Val Ala Gln Glu Lys Trp Ser Glu Ser Ser Ile Gly
               180                 185                 190
   Tyr Asp Pro Pro His Phe Asp Ser Lys Ala Pro Ser Pro Pro Asn Arg
           195                 200                 205
   Ser Pro Ser Ala Ser Thr Asp Asn Ile Leu His Phe Glu His Lys Asp
       210                 215                 220
   Val Ser Gly Gln Leu Ser Arg Val Lys Leu Gln Ala Leu Pro Ser Glu
   225                 230                 235                 240
   Phe Pro Pro Ile Asp His Ala Leu Leu Lys Ser Leu Phe His Arg Pro
                   245                 250                 255
   Tyr Phe His Ile Gly Val Val Ser Asp Val Ala Gly Val Ser Leu Gly
               260                 265                 270
   Gly Ala Leu Lys Asn Val Val Ala Val Ala Ala Gly Trp Val Val Gly
           275                 280                 285
   Lys Gly Trp Gly Asp Asn Ala Lys Ala Ala Ile Met Arg Val Gly Leu
       290                 295                 300
   Leu Glu Met Val Lys Phe Gly Glu Gln Phe Phe Gly Ala Thr Ile Asn
   305                 310                 315                 320
   Thr Arg Thr Phe Thr Glu Glu Ser Ala Gly Val Ala Asp Leu Ile Thr
                   325                 330                 335
   Ser Cys Ser Gly Gly Arg Asn Phe Arg Cys Ala Lys Leu Ser Ile Glu
               340                 345                 350
   Arg Asn Gln Pro Ile Glu Lys Ile Glu Glu Thr Glu Leu Asn Gly Gln
           355                 360                 365
   Lys Leu Gln Gly Thr Leu Thr Ala Val Glu Val Asn Ser Phe Leu Lys
       370                 375                 380
   Lys Gln Gly Leu Glu Glu Glu Phe Pro Leu Phe Thr Ala Val Tyr Arg
   385                 390                 395                 400
   Val Leu Gln Gly Thr Met Ser Val Asp Glu Ile Pro Ser Phe Ile Glu
                   405                 410                 415
   Arg
<210> 39
   <211> 999
   <212> DNA
   <213> Debaryomyces hansenii
<220>
   <221> CDS
   <222> (1)..(996)
   <223> coding for G3PDH (partial)
<400> 39
   gga tct ggt aac tgg ggt act gct gtt gct aag atc gta tct gaa aac     48
   Gly Ser Gly Asn Trp Gly Thr Ala Val Ala Lys Ile Val Ser Glu Asn
    1               5                  10                  15
   acg gct gaa aaa cca gaa gtg ttc gaa aag caa gtg aac atg tgg gtt     96
   Thr Ala Glu Lys Pro Glu Val Phe Glu Lys Gln Val Asn Met Trp Val
               20                  25                  30
   ttt gaa gaa gaa gtt gac gga caa aag ttg act gaa atc atc aac gcc     144
   Phe Glu Glu Glu Val Asp Gly Gln Lys Leu Thr Glu Ile Ile Asn Ala
           35                  40                  45
   aaa cac gaa aac gtt aag tac ttg cca gaa gtc aag ttg ccg gaa aac     192
   Lys His Glu Asn Val Lys Tyr Leu Pro Glu Val Lys Leu Pro Glu Asn
       50                  55                  60
   ttg gtt gca aac cca gac gtt gtt gac act gtc aag gat gca gac tta     240
   Leu Val Ala Asn Pro Asp Val Val Asp Thr Val Lys Asp Ala Asp Leu
   65                  70                  75                  80
   tta att ttt aac att cca cat caa ttc tta cca aga gtg tgt aag caa     288
   Leu Ile Phe Asn Ile Pro His Gln Phe Leu Pro Arg Val Cys Lys Gln
                    85                  90                  95
   ttg gtt ggc cat gtc aag cca tct gcc aga gcc atc tcc tgt ttg aag     336
   Leu Val Gly His Val Lys Pro Ser Ala Arg Ala Ile Ser Cys Leu Lys
               100                 105                 110
   ggt ttg gaa gtt ggc cca gaa ggt tgt aag ttg tta tcg caa tct atc     384
   Gly Leu Glu Val Gly Pro Glu Gly Cys Lys Leu Leu Ser Gln Ser Ile
           115                 120                 125
   aac gat act tta ggt gtc cac tgt ggt gtc tta tct ggt gcc aac att     432
   Asn Asp Thr Leu Gly Val His Cys Gly Val Leu Ser Gly Ala Asn Ile
       130                 135                 140
   gcc aac gaa gtt gcc aga gaa aga tgg tct gaa acc acc att gcc tac     480
   Ala Asn Glu Val Ala Arg Glu Arg Trp Ser Glu Thr Thr Ile Ala Tyr
   145                 150                 155                 160
   aac att cca gaa gat ttc aga ggt aag ggt aga gat atc gac gaa tac     528
   Asn Ile Pro Glu Asp Phe Arg Gly Lys Gly Arg Asp Ile Asp Glu Tyr
                   165                 170                 175
   gtc tta aag caa tta ttc cac aga acc tac ttc cat gtc aga gtc atc     576
   Val Leu Lys Gln Leu Phe His Arg Thr Tyr Phe His Val Arg Val Ile
               180                 185                 190
   aac gac atc ata ggt gct tct ttc gct ggt gct ttg aag aat gtt gtt     624
   Asn Asp Ile Ile Gly Ala Ser Phe Ala Gly Ala Leu Lys Asn Val Val
           195                 200                 205
   gcc tgt gct gtt ggt ttc gtt atc ggt gcc ggc tgg ggt gac aac gct     672
   Ala Cys Ala Val Gly Phe Val Ile Gly Ala Gly Trp Gly Asp Asn Ala
       210                 215                 220
   aag gcc gct atc atg aga atc ggt atc aga gaa atc atc cac ttt gcc     720
   Lys Ala Ala Ile Met Arg Ile Gly Ile Arg Glu Ile Ile His Phe Ala
   225                 230                 235                 240
   tct tac tac caa aag ttc ggt gtc aag ggt cca gct cca gaa tcc act     768
   Ser Tyr Tyr Gln Lys Phe Gly Val Lys Gly Pro Ala Pro Glu Ser Thr
                   245                 250                 255
   act ttc act gag gaa tct gcc ggt gtc gct gac tta atc acc act tgt     816
   Thr Phe Thr Glu Glu Ser Ala Gly Val Ala Asp Leu Ile Thr Thr Cys
               260                 265                 270
   tcc ggt ggt aga aat gtc aag gtt gct aga tac atg att gaa aac aac     864
   Ser Gly Gly Arg Asn Val Lys Val Ala Arg Tyr Met Ile Glu Asn Asn
           275                 280                 285
   gtt gac gct tgg gaa gcc gaa aag att gtc tta aag ggt caa tct tct     912
   Val Asp Ala Trp Glu Ala Glu Lys Ile Val Leu Lys Gly Gln Ser Ser
       290                 295                 300
   caa ggt atc tta act gcc aag gaa gtc cac gaa ttg tta act aac tac     960
   Gln Gly Ile Leu Thr Ala Lys Glu Val His Glu Leu Leu Thr Asn Tyr
   305                 310                 315                 320
   aac tta tcg aat gaa ttc cca tta ttt gaa gcc gta tac                 999
   Asn Leu Ser Asn Glu Phe Pro Leu Phe Glu Ala Val
                   325                 330
<210> 40
   <211> 332
   <212> PRT
   <213> Debaryomyces hansenii
<400> 40
   Gly Ser Gly Asn Trp Gly Thr Ala Val Ala Lys Ile Val Ser Glu Asn
    1               5                  10                  15
   Thr Ala Glu Lys Pro Glu Val Phe Glu Lys Gln Val Asn Met Trp Val
               20                  25                  30
   Phe Glu Glu Glu Val Asp Gly Gln Lys Leu Thr Glu Ile Ile Asn Ala
           35                  40                  45
   Lys His Glu Asn Val Lys Tyr Leu Pro Glu Val Lys Leu Pro Glu Asn
       50                  55                  60
   Leu Val Ala Asn Pro Asp Val Val Asp Thr Val Lys Asp Ala Asp Leu
   65                  70                  75                  80
   Leu Ile Phe Asn Ile Pro His Gln Phe Leu Pro Arg Val Cys Lys Gln
                   85                  90                  95
   Leu Val Gly His Val Lys Pro Ser Ala Arg Ala Ile Ser Cys Leu Lys
               100                 105                 110
   Gly Leu Glu Val Gly Pro Glu Gly Cys Lys Leu Leu Ser Gln Ser Ile
           115                 120                 125
   Asn Asp Thr Leu Gly Val His Cys Gly Val Leu Ser Gly Ala Asn Ile
       130                 135                 140
   Ala Asn Glu Val Ala Arg Glu Arg Trp Ser Glu Thr Thr Ile Ala Tyr
   145                 150                 155                 160
   Asn Ile Pro Glu Asp Phe Arg Gly Lys Gly Arg Asp Ile Asp Glu Tyr
                   165                 170                 175
   Val Leu Lys Gln Leu Phe His Arg Thr Tyr Phe His Val Arg Val Ile
               180                 185                 190
   Asn Asp Ile Ile Gly Ala Ser Phe Ala Gly Ala Leu Lys Asn Val Val
           195                 200                 205
   Ala Cys Ala Val Gly Phe Val Ile Gly Ala Gly Trp Gly Asp Asn Ala
       210                 215                 220
   Lys Ala Ala Ile Met Arg Ile Gly Ile Arg Glu Ile Ile His Phe Ala
   225                 230                 235                 240
   Ser Tyr Tyr Gln Lys Phe Gly Val Lys Gly Pro Ala Pro Glu Ser Thr
                   245                 250                 255
   Thr Phe Thr Glu Glu Ser Ala Gly Val Ala Asp Leu Ile Thr Thr Cys
               260                 265                 270
   Ser Gly Gly Arg Asn Val Lys Val Ala Arg Tyr Met Ile Glu Asn Asn
           275                 280                 285
   Val Asp Ala Trp Glu Ala Glu Lys Ile Val Leu Lys Gly Gln Ser Ser
       290                 295                 300
   Gln Gly Ile Leu Thr Ala Lys Glu Val His Glu Leu Leu Thr Asn Tyr
   305                 310                 315                 320
   Asn Leu Ser Asn Glu Phe Pro Leu Phe Glu Ala Val
                   325                 330

## Beschreibung

Die Erfindung betrifft Verfahren zum Erhöhen des Ölgehaltes in Pflanzen, bevorzugt in pflanzlichen Samen, durch Expression von Glycerol-3-phosphatdehydrogenasen (G3PDH) aus Hefen, bevorzugt aus Saccharomyces cerevisiae. Die Erfindung betrifft ferner Expressionskonstrukte zur Expression von Hefe G3PDH in Pflanzen, bevorzugt in pflanzlichen Samen, transgene Pflanzen exprimierend Hefe G3PDH, sowie die Verwendung besagter transgener Pflanzen zur Herstellung von zur Herstellung von Ölen, Fetten, freien Fettsäuren oder Derivaten der vorgenannten.

Die Erhöhung des Ölgehalts in Pflanzen und insbesondere in Pflanzensamen ist für die klassische wie für die modernen Pflanzenzüchtung und insbesondere die pflanzliche Biotechnologie von großem Intressen. Bedingt durch den steigenden Verbrauch von Pflanzenölen für Ernährung bzw. industrielle Anwendungen sind Möglichkeiten zur Steigerung bzw. Modifikation von Pflanzenölen zunehmend Gegenstand aktueller Forschung (z.B. Töpfer et al. (1995) Science 268:681-686). Ziel ist dabei insbesondere die Erhöhung des Gehaltes an Fettsäuren in Samenölen.

Auch die aus den pflanzlichen Ölen erhältlichen Fettsäuren sind von besonderem Interesse. Sie kommen beispielsweise als Grundstoffe für Weichmacher, Schmierstoffe, Tenside, Kosmetika usw. zum Einsatz oder werden in der Lebens- und Futtermittelindustrie als wertvolle Grundstoffe eingesetzt. So ist beispielsweise die Bereitstellung von Rapsölen mit Fettsäuren mittlerer Kettenlänge von besonderem Interesse, da diese besonderes in der Tensidherstellung begehrt sind.

Durch die gezielte Modulation pflanzlicher Stoffwechselwege mittels gentechnischer Verfahren kann der pflanzliche Metabolismus in einer Weise vorteilhaft verändert werden, die durch klassische Züchtungsmethoden nur über langwierige Schritte bzw. überhaupt nicht zu erreichen wären. So werden ungewöhnliche Fettsäuren, beispielsweise bestimmte polyungesättigte Fettsäuren, nur in bestimmten Pflanzen bzw. überhaupt nicht in Pflanzen synthetisiert und können deshalb nur durch Expression des entsprechenden Enzyms in transgenen Pflanzen hergestellt werden (z.B. Millar et al. (2000) Trends Plant Sci 5:95-101).

Triacylgylceride und andere Lipide werden aus Fettsäuren synthetisiert. Die Fettsäure- und Triacylglyceridbiosynthese lassen sich aufgrund der Kompartimentierung als getrennte Biosynthesewege, jedoch im Hinblick auf das Endprodukt, als ein Biosyntheseweg ansehen. Die Lipidsynthese kann dabei in zwei Teilmechanismen unterteilt werden, einen quasi "prokaryotischen" und einen quasi "eukaryotischen" (Browse et al. (1986) Biochemical J 235:25-31; Ohlrogge & Browse (1995) Plant Cell 7:957-970). Der prokaryotische Mechanismus ist in den Plastiden lokalisiert und umfasst die Biosynthese der freien Fettsäuren, die in das Cytosol exportiert werden, wo sie als Fettsäureacyl-CoA-Ester in den eukaryotischen Mechanismus eingehen und mit Glycerin-3-phosphat (G3P) zu Phosphatidsäure (PA) verestert werden. PA ist der Ausgangspunkt für die Synthese von neutralen und polaren Lipiden. Die neutralen Lipide werden dabei über den Kennedy-Weg am Endoplasmatischen Reticulum synthetisiert (Voelker (1996) Genetic Engineering, Setlow (ed.) 18:111-113; Shankline & Cahoon (1998) Annu Rev Plant Physiol Plant Mol Biol 49:611-649; Frentzen (1998) Lipids 100:161-166). Neben der Biosynthese von Triacylglyceriden dient G3P auch der Synthese von Glycerol (z.B. zur Osmoregulation und gegen Kältestress).

Das für die Synthese wesentliche G3P wird dabei durch Reduktion von Dihydroxyacetonphosphat (DHAP) mittels der Glycerin-3-Phosphat-Dehydrogenase (G3PDH), auch als Dihydroxyacetonphosphatreduktase bezeichnet, synthetisiert. Dabei fungiert in der Regel NADH als reduzierendes Cosubstrat (EC 1.1.1.8). Eine weitere Klasse von Glycerin-3-Phosphat-Dehydrogenasen (EC 1.1.99.5) verwendet FAD als Cosubstrat. Die Enzyme dieser Klasse katalysieren die Reaktion von DHAP zu G3P. In eukaryontischen Zellen sind die beiden Enzymklassen in unterschiedlichen Kompartimenten verteilt, wobei die NADabhängigen cytosolisch und die FAD-abhängigen mitochondriell lokalisiert sind (für Saccharomyces cerevisiae siehe z.B. Larsson et al., 1998, Yeast 14:347-357). EP-A 0 353 049 beschreibt eine NAD-unabhängige G3PDH aus Bacillus sp. Auch in Saccharomyces cerevisiae wurde eine NAD-unabhängige G3PDH identifiziert (Miyata K, Nagahisa M (1969) Plant Cell Physiol 10(3):635-643).

G3PDH ist ein essentielles Enzym in Prokaryoten und Eukaryoten, das neben der Funktion in der Lipidbiosynthese auch für die Aufrechterhaltung des zellulären Redoxstatus durch den Einfluss auf das NAD+/NADH Verhältnis mitverantwortlich ist. Die Deletion des GPD2 Gens in Saccharomyces cerevisae (eine von zwei Isoformen der G3PDH in dieser Hefe) hat ein vermindertes Wachstum unter anaeroben Bedingungen zur Folge. Darüber hinaus scheint die G3PDH eine Rolle in der Stressantwort der Hefe v.a. gegen osmotischen Stress zu spielen. Die Deletion des GPD1 Gens bedingt in Saccharomyces cerevisae eine Hypersensitivität gegen Salz.

Sequenzen für G3PDHs wurden beschrieben für Insekten (Drosophila melanogaster, Drosophila virilis), Pflanzen (Arabidopsis thaliana, Cuphea lanceolata), Säugern (Homo sapiens, Mus musculus, Sus scrofa, Rattus norvegicus), Fischen (Salmo salar, Osmerus mordax), Vögeln (Ovis aries), Amphibien (Xenopus laevis), Nematoden (Caenorhabditis elegans), Algen und Bakterien.

Pflanzliche Zellen weisen mindestens zwei Isoformen der G3PDH auf, eine cytoplasmatische und eine plastidäre (Gee RW et al. (1988) Plant Physiol 86:98-103; Gee RW et al. (1988) Plant Physiol 87:379-383). Die enzymatische Aktivität der Glycerin-3-Phosphat-Dehydrogenase wurde bei Pflanzen erstmals in Kartoffelknollen festgestellt (Santora GT et al. (1979) Arch Biochem Biophys 196:403-411). Weitere zytosolisch und plastidär lokalisierte G3PDH Aktivitäten wurden in anderen Pflanzen wie Erbse, Mais oder Soja detektiert (Gee RW et al. (1988) PLANT PHYSIOL 86(1): 98-103). Beschrieben sind ferner G3PDHs aus Algen wie z.B. zwei plastidäre und einer zytosolische G3PDH-Isoform aus Dunaliella tertiolecta (Gee R et al.(1993) Plant Physiol 103(1):243-249; Gee R et al. (1989) PLANT PHYSIOL 91(1):345-351). Für die pflanzliche G3PDH aus Cuphea lanceolata wurde vorgeschlagen, durch Überexpression in Pflanzen eine Erhöhung des Ölgehaltes oder eine Verschiebung im Fettsäuremuster zu erreichen (WO 95/06733). Entsprechende Effekte konnten jedoch nicht belegt werden.

Bakterielle G3PDHs und ihre Funktion sind beschrieben (Hsu und Fox (1970) J Bacteriol 103:410-416; Bell (1974) J Bacterial 117:1065-1076).

WO 01/21820 beschreibt die heterologe Expression einer mutierten E. coli G3PDH für erhöhte Stresstoleranz und Änderung der Fettsäurezusammensetzung in Speicherölen. Die mutierte E.coli G3PDH (gpsA2FR) weist einen einzelnen Aminosäureaustausch auf, der eine verringerte Inhibition durch G3P bedingt. Die heterologe Expression der gpsA2FR Mutante führt zu Glycerolipiden mit einem erhöhten Anteil an C16-Fettsäuren und einem einhergehenden verminderten Anteil an C18-Fettsäuren. Die Veränderungen im Fettsäuremuster sind relativ gering: Ein Anstieg von 2 bis 5 % an 16:0 Fettsäuren und 1,5 bis 3,5 % bei 16:3 Fettsäuren, sowie eine Verminderung an 18:2 und 18:3 Fettsäuren um 2 bis 5 % wurde beobachtet. Der Gesamtgehalt als Glycerolipiden blieb unbeeinflusst.

Beschrieben sind ferner G3PDH aus Hefen (Ascomyceten) wie
a) Schizosaccharomyces pombe (Pidoux AL et al. (1990) Nucleic Acids Res 18 (23): 7145; GenBank Acc.-No.: X56162; Ohmiya R et al. (1995) Mol Microbiol 18(5):963-73; GenBank Acc.-No.: D50796, D50797),
b) Yarrowia lipolytica (GenBank Acc.-No.: AJ250328)
c) Zygosaccharomyces rouxii (Iwaki T et al. Yeast (2001) 18(8):737-44; GenBank Acc.-No: AB047394, AB047395, AB047397) oder
d) Saccharomyces cerevisiae (Albertyn J et al. (1994) Mol Cell Biol 14(6):4135-44; Albertyn J et al. (1992) FEBS LETT 308(2):130-132; Merkel JR et al. (1982) Anal Biochem 122 (1):180-185; Wang HT et al. (1994) J Bacteriol. 176(22):7091-5; Eriksson P et al. (1995) Mol Microbiol. 17(1):95-107; GenBank Acc.-No.: U04621, X76859, Z35169).
e) Emericella nidulans (GenBank Acc.-No.: AF228340)
f) Debaryomyces hansenii (GenBank Acc.-No.: AF210060)

Es stellte sich daher die Aufgabe alternative Verfahren zur Erhöhung des Ölgehaltes in Pflanzen bereitzustellen. Diese Aufgabe wird durch die vorliegende Erfindung gelöst.

Ein erster Gegenstand der Erfindung umfasst ein Verfahren zum Erhöhen des Gesamtölgehalt in einem pflanzlichen Organismus oder einem Gewebe, Organ, Teil oder Zelle desselben, umfassend
a) transgene Expression einer Glycerol-3-phosphat Dehydrogenase aus einer Hefe in besagtem pflanzlichen Organismus oder einem Gewebe, Organ, Teil oder Zelle desselben und
b) Auswahl von pflanzlichen Organismen, bei denen - im Unterschied oder Vergleich zur Ausgangsorganismus - der Gesamtölgehalt in dem besagten pflanzlichen Organismus oder einem Gewebe, Organ, Teil oder Zelle desselben erhöht ist.

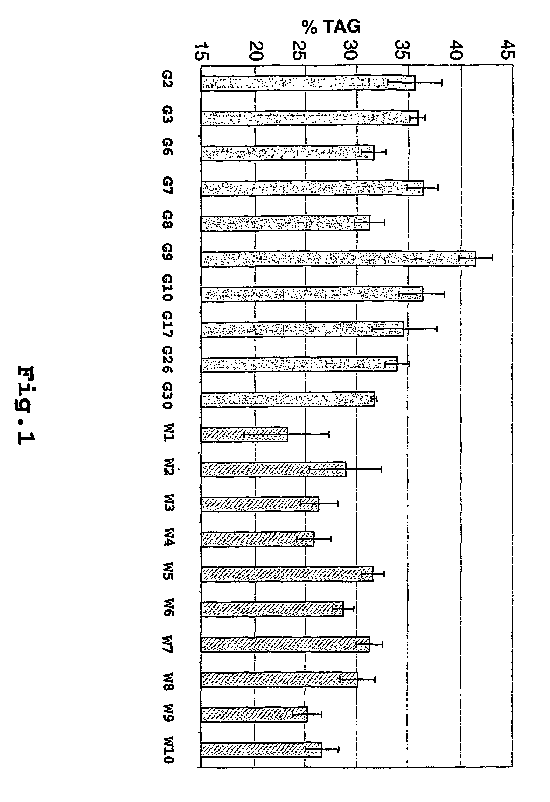

Überraschenderweise konnte festgestellt werden, dass die samenspezifische heterologe Expression des Hefeproteins Gpd1p (G3PDH aus Saccharomyces cerevisiae; SEQ ID NO: 2) in Arabidopsis-Samen zu einer signifikanten Erhöhung des Gehalts an Triacylglyceriden (Speicheröle) führt. Der Ölgehalt wurde dabei um etwa 22 %, in einer transgenen Linie sogar um 41 %, verglichen mit Wildtyp-Kontrollpflanzen gesteigert (siehe Fig. 1). Die transgene Expression der Glycerol 3-phosphat Dehydrogenase aus Hefe zeigte keine nachteiligen Effekte auf das Wachstum oder andere Eigenschaften der transformierten Pflanzen.

Da G3PDH ein Schlüsselenzym der Biosynthese in allen pflanzlichen Organismen ist, kann das erfindungsgemäße Verfahren im Prinzip auf alle Pflanzenarten - neben der als Modelpflanze eingesetzten Art *Arabidopsis thaliana -* angewendet werden. Bevorzugt wird das erfindungsgemäße Verfahren auf Ölpflanzen angewendet, die bereits natürlicherweise einen hohen Ölgehalt aufweisen und/oder zu industriellen Gewinnung von Ölen verwendet werden.

"Pflanzlicher Organismus oder Gewebe, Organ, Teil, Zelle oder Vermehrungsgut desselben" meint allgemein jeden ein oder mehrzelligen Organismus oder ein Zelle, Gewebe, Teile oder Vermehrungsgut (wie Samen oder Früchte) desselben, der zur Photosynthese befähigt ist. Eingeschlossen sind im Rahmen der Erfindung alle Gattungen und Arten höherer und niederer Pflanzen des Pflanzenreiches. Einjährige, mehrjährige, monocotyledone und dicotyledone Pflanzen sind bevorzugt. Eingeschlossen sind reife Pflanze, Saatgut, Sprosse und Keimlinge, sowie davon abgeleitete Teile, Vermehrungsgut (zum Beispiel Knollen, Samen oder Früchte) und Kulturen, zum Beispiel Zell- oder Kalluskulturen.

"Pflanze" im Rahmen der Erfindung meint alle Gattungen und Arten höherer und niederer Pflanzen des Pflanzenreiches. Eingeschlossen unter dem Begriff sind die reifen Pflanzen, Saatgut, Sprossen und Keimlinge, sowie davon abgeleitete Teile, Vermehrungsgut, Pflanzenorgane, Gewebe, Protoplasten, Kallus und andere Kulturen, zum Beispiel Zellkulturen, sowie alle anderen Arten von Gruppierungen von Pflanzenzellen zu funktionellen oder strukturellen Einheiten. Reife Pflanzen meint Pflanzen zu jedem beliebigen Entwicklungsstadium jenseits des Keimlings. Keimling meint eine junge, unreife Pflanze in einem frühen Entwicklungsstadium.

"Pflanze" umfasst alle einjährigen und mehrjährige, monokotyledonen und dikotyledonen Pflanzen und schließt beispielhaft jedoch nicht einschränkend solche der Gattungen Cucurbita, Rosa, Vitis, Juglans, Fragaria, Lotus, Medicago, Onobrychis, Trifolium, Trigonella, Vigna, Citrus, Linum, Geranium, Manihot, Daucus, Arabidopsis, Brassica, Raphanus, Sinapis, Atropa, Capsicum, Datura, Hyoscyamus, Lycopersicon, Nicotiana, Solarium, Petunia, Digitalis, Majorana, Cichorium, Helianthus, Lactuca, Bromus, Asparagus, Antirrhinum, Heterocallis, Nemesis, Pelargonium, Panieum, Pennisetum, Ranunculus, Senecio, Salpiglossis, Cucumis, Browaalia, Glycine, Pisum, Phaseolus, Lolium, Oryza, Zea, Avena, Hordeum, Secale, Triticum, Sorghum, Picea und Populus ein.

Bevorzugt sind Pflanzen nachfolgender Pflanzenfamilien: Amaranthaceae, Asteraceae, Brassicaceae, Carophyllaceae, Chenopodiaceae, Compositae, Cruciferae, Cucurbitaceae, Labiatae, Leguminosae, Papilionoideae, Liliaceae, Linaceae, Malvaceae, Rosaceae, Rubiaceae, Saxifragaceae, Scrophulariaceae, Solanacea, Sterculiaceae, Tetragoniacea, Theaceae, Umbelliferae.

Bevorzugte monokotyle Pflanzen sind insbesondere ausgewählt aus den monokotylen Kulturpflanzen, wie zum Beispiel der Familie der Gramineae wie Reis, Mais, Weizen oder andere Getreidearten wie Gerste, Hirse, Roggen, Triticale oder Hafer sowie dem Zuckerrohr sowie alle Arten von Gräsern.

Die Erfindung wird ganz besonders bevorzugt aus dikotyledone pflanzliche Organismen angewendet. Bevorzugte dikotyle Pflanzen sind insbesondere ausgewählt aus den dikotylen Kulturpflanzen, wie zum Beispiel
- Asteraceae wie Sonnenblume, Tagetes oder Calendula und andere mehr,
- Compositae, besonders die Gattung Lactuca, ganz besonders die Art sativa (Salat) und andere mehr,
- Cruciferae, besonders die Gattung Brassica, ganz besonders die Arten napus (Raps), campestris (Rübe), oleracea cv Tastie (Kohl), oleracea cv Snowball Y (Blumenkohl) und oleracea cv Emperor (Broccoli) und weitere Kohlarten; und der Gattung Arabidopsis, ganz besonders die Art thaliana sowie Kresse oder Canola und andere mehr,
- Cucurbitaceae wie Melone, Kürbis oder Zucchini und andere mehr,
- Leguminosae besonders die Gattung Glycine, ganz besonders die Art max (Sojabohne) Soja sowie Alfalfa, Erbse, Bohnengewächsen oder Erdnuss und andere mehr
- Rubiaceae, bevorzugt der Unterklasse Lamiidae wie beispielsweise Coffea arabica oder Coffea liberica (Kaffeestrauch) und andere mehr,
- Solanaceae besonders die Gattung Lycopersicon, ganz besonders die Art esculentum (Tomate), die Gattung Solanum, ganz besonders die Art tuberosum (Kartoffel) und melongena (Aubergine) und die Gattung Capsicum, ganz besonders die Art annum (Pfeffer) sowie Tabak oder Paprika und andere mehr,
- Sterculiaceae, bevorzugt der Unterklasse Dilleniidae wie beispielsweise Theobroma cacao (Kakaostrauch) und andere mehr,
- Theaceae, bevorzugt der Unterklasse Dilleniidae wie beispielsweise Camellia sinensis oder Thea sinensis (Teestrauch) und andere mehr,
- Umbelliferae, besonders die Gattung Daucus (ganz besonders die Art carota (Karrotte)) und Apium (ganz besonders die Art graveolens dulce (Selarie)) und andere mehr;
sowie Lein, Baumwolle, Hanf, Flachs, Gurke, Spinat, Möhre, Zuckerrübe und den verschiedenen Baum-, Nuss- und Weinarten, insbesondere Banane und Kiwi.

Umfasst sind ferner Schmuckpflanzen, Nutz- oder Zierbäume, Blumen, Schnittblumen, Sträucher oder Rasen. Beispielhaft aber nicht einschränkend seien zu nennen Angiospermen, Bryophyten wie zum Beispiel Hepaticae (Leberblümchen) und Musci (Moose); Pteridophyten wie Farne, Schachtelhalm und Lycopoden; Gymnospermen wie Koniferen, Cycaden, Ginkgo und Gnetalen, die Familien der Rosaceae wie Rose, Ericaceae wie Rhododendrons und Azaleen, Euphorbiaceae wie Weihnachtssterne und Kroton, Caryophyllaceae wie Nelken, Solanaceae wie Petunien, Gesneriaceae wie das Usambaraveilchen, Balsaminaceae wie das Springkraut, Orchidaceae wie Orchideen, Iridaceae wie Gladiolen, Iris, Freesie und Krokus, Compositae wie Ringelblume, Geraniaceae wie Geranien, Liliaceae wie der Drachenbaum, Moraceae wie Ficus, Araceae wie Philodendron und andere mehr.

Pflanzliche Organismen im Sinne der Erfindung sind weiterhin weitere photosynthetisch aktive befähigte Organismen, wie zum Beispiel Algen, Cyanobakterien sowie Moose. Bevorzugte Algen sind Grünalgen, wie beispielsweise Algen der Gattung Haematococcus, Phaedactylum tricornatum, Volvox oder Dunaliella. Insbesondere bevorzugt ist Synechocystis.

Am meisten bevorzugt sind Ölpflanzen. Ölpflanzen meint Pflanzen, die bereits natürlicherweise einen hohen Ölgehalt aufweisen und/oder zu industriellen Gewinnung von Ölen verwendet werden. Diese Pflanzen können einen hohen Ölgehalt und/oder aber eine besondere, industriell interessante Fettsäurezusammensetzung aufweisen. Bevorzugt sind Pflanzen, die einen Lipidanteil von mindestens 1 Gew.-% aufweisen. Ölpflanzen umfassen beispielhaft: Borago officinalis (Borretsch); Brassica Arten wie *B. campestris, B. napus, B. rapa* (Senf, Raps oder Rübsel); *Cannabis sativa* (Hanf); Curthamus *tinctorius* (Färberdiestel); Cocos *nucifera* (Kokosnuss); Crambe abyssinica (Krambe); Cuphea Arten (Cuphea Arten liefern fettsäuren von mittlerer Kettenlänge insbesondere für industrielle Anwendungen); *Elaeis guinensis* (Afrikanische Ölpalme); *Ekeis oleiferu* (Amerikanische Ölpalme); *Glycine max* (Sojabohne); *Gossypium hirsitum* (Amerikanische Baumwolle); *Gossypium barbadense* (Ägyptische Baumwolle); *Gossypium herbaceum* (Asiatische Baumwolle); *Helianthus annus* (Sonnenblume); *Linum usitatissimum* (Lein oder Flachs); *Oenethem biennis* (Nachtkerze); Ozea *europea* (Olive); Oryza *sativa* (Rice); *Ricinus communis* (Castor); Sesamum *indicum* (Sesam); *Triticum* Arten (Weizen); Zea *maize* (Mais) sowie verschiedene Nussarten wie beispielsweise Walnuss oder Mandel.

"Gesamtölgehalt" meint die Summe aller Öle, bevorzugt die Summe die Triacylglyceride.

"Öle" umfasst neutrale und/oder polare Lipiden und Mischungen derselben. Beispielhaft jedoch nicht einschränkend seien die in Tabelle 1 aufgeführten zu nennen.

**Tab. 1: Pflanzliche Lipidklassen**

| | |
|---|---|
| Neutrale Lipide | Triacylglycerol (TAG) |
| | Diacylglycerol (DAG) |
| | Monoacylglycerol (MAG) |
| | |
| Polare Lipide | Monogalactosyldiacylglycerol (MGDG) |
| | Digalactosyldiacylglycerol (DGDG) |
| | Phosphatidylglycerol (PG) |
| | Phosphatidylcholine (PC) |
| | Phosphatidylethanolamine (PE) |
| | Phosphatidylinositol (PI) |
| | Phosphatidylserin (PS) |
| | Sulfoquinovosyldiacylglycerol |

Neutrale Lipide meint bevorzugt Triacylglyceride. Sowohl die neutralen als auch die polaren Lipide können ein breites Spektrum an verschiedenen Fettsäuren enthalten. Beispielhaft jedoch nicht einschränkend seien die in Tabelle 2 aufgeführten Fettsäuren zu nennen.

**Tab. 2: Übersicht über verschiedene Fettsäuren (Auswahl) ¹ Kettenlänge:Anzahl der Doppelbindungen * nicht natürlicherweise in Pflanzen vorkommend**

| Nomenklatur¹ | Name |
|---|---|
| 16:0 | Palmitinsäure |
| 16:1 | Palmitoleinsäure |
| 16:3 | Roughaninsäure |
| 18:0 | Stearinsäure |
| 18:1 | Ölsäure |
| 18:2 | Linolsäure |
| 18:3 | Linolensäure |
| γ-18:3 | Gamma-Linolensäure* |
| 20:0 | Arachidinsäure |
| 22:6 | Docosahexanonsäure (DHA) * |
| 20:2 | Eicosadienonsäure |
| 20:4 | Arachidonsäure (AA) * |
| 20:5 | Eicosapentaenosäure (EPA) * |
| 22:1 | Erucasäure |

Öle meint bevorzugt Samenöle.

"Erhöhung" des Gesamtölgehaltes meint die Steigerung des Gehaltes an Ölen in einer Pflanze oder einem Teil, Gewebe oder Organ derselben, bevorzugt in den Samenorganen der Pflanze. Dabei ist der Ölgehalt im Vergleich zu einer nicht dem erfindungsgemäßen Verfahren unterworfenen, aber ansonsten unveränderten Ausgangspflanze unter ansonsten gleichen Rahmenbedingungen um mindestens 5 %, bevorzugt mindestens 10 %, besonders bevorzugt mindestens 15 %, ganz besonders bevorzugt mindestens 20 %, am meisten bevorzugt mindestens 25 % erhöht. Rahmenbedingungen meint dabei alle für die Keimung, Kultivierung oder Wachstum der Pflanze relevanten Bedingungen wir Boden-, Klima- oder Lichtverhältnisse, Düngung, Bewässerung, Pflanzenschutzmaßnahmen usw.

"Glycerol 3-phosphat Dehydrogenase aus Hefe" (infolge "Hefe G3PDH") meint allgemein all solche Enzyme, die in der Lage sind, Dihydroxyacetonphosphat (DHAP) zu Glycerol-3-phosphat (G3P) - bevorzugt unter Verwendung eines Cosubstrates wie NADH - umzusetzen und natürlicherweise in einer Hefe exprimiert werden.

Hefen meint eine die Gruppe einzelliger Pilze mit ausgeprägter Zellwand und Bildung von Pseudomyzel (im Ggs. zu Schimmelpilzen). Ihre Vermehrung erfolgt vegetativ durch Sprossung und/oder Spaltung (Schizosaccharomyces bzw. Saccharomycodes).

Umfasst sind sogenannte unechte Hefen, und zwar bevorzugt die Familien Cryptococcaceae, Sporobolomycetaceae mit den Gattungen Cryptococcus, Torulopsis, Pityrosporum, Brettanomyces, Candida, Kloeckera, Trigonopsis, Trichosporon, Rhodotorula bzw. Sporobolomyces und Bullera, sowie echte Hefen (Hefen mit auch geschlechtlicher Vermehrung; Ascus), und zwar bevorzugt die Familien Endo- u. Saccharomycetaceae, mit den Gattungen Saccharo-, Debaro-, Lipomyces, Hansenula, Endomycopsis, Pichia, Hanseniaspora. Am meisten bevorzugt sind die Arten Saccharomyces cerevisiae, Pichia pastoris, Hansenula polymorpha, Schizosaccharomyces pombe, Kluyveromyces lactis, Zygosaccharomyces rouxii, Yarrowia lipolitica, Emericella nidulans, Aspergillus nidulans, Debaryomyces hansenii und Torulaspora hansenii..

Hefe G3PDH meint insbesondere Polypeptide die als "wesentlichen Eigenschaften" nachfolgende Eigenschaften aufweisen:
a) die Umsetzung von Dihydroxyacetonphosphat zu Glycerin-3-phosphat unter Verwendung von NADH als Cosubstrat (EC 1.1.1.8), und
b) eine Peptidsequenz umfassend mindestens ein Sequenzmotiv ausgewählt aus der Gruppe von Sequenzmotiven bestehend aus
   i) GSGNWGT(A/T)IAK (SEQ ID NO: 22)
   ii) CG(V/A)LSGAN(L/I/V)AXE(V/I)A (SEQ ID NO: 26)
   iii) (L/V)FXRPYFXV (SEQ ID NO: 27)
      bevorzugt ist das Sequenzmotiv ausgewählt aus der Gruppe bestehend aus
   iv) GSGNWGTTIAKV(V/I)AEN (SEQ ID NO: 29)
   v) NT (K/R) HQNVKYLP (SEQ ID NO: 30)
   vi) D(I/V)LVFN(I/V)PHQFL (SEQ ID NO: 31)
   vii) RA(I/V)SCLKGFE (SEQ ID NO: 32)
   viii) CGALSGANLA(P/T)EVA (SEQ ID NO: 33)
   ix) LFHRPYFHV (SEQ ID NO: 34)
   x) GLGEII(K/R)FG (SEQ ID NO: 35)
      besonders bevorzugt enthält die Peptidsequenz mindestens 2 oder 3, ganz besonders bevorzugt mindestens 4 oder 5, am meisten bevorzugt alle der Sequenzmotive ausgewählt aus der Gruppe der Sequenzmotive i), ii) und iii) oder ausgewählt aus der Gruppe der Sequenzmotive iv), v), vi), vii), viii), ix) und xiv). (Angaben in Klammern meinen alternativ mögliche Aminosäuren an dieser Position; z.B. mein (V/I), dass an dieser Position Valin oder Isoleucin möglich ist). Weiterhin kann eine Hefe G3PDH optional - zusätzlich zu mindestens einem der oben genannten Sequenzmotive i) bis x) - weitere Sequenzmotive umfassen ausgewählt aus der Gruppe bestehend aus
   xi) H(E/Q)NVKYL (SEQ ID NO: 23)
   xii) (D/N) (I/V) (L/I) V (F/W) (V/N) (L/I/V) PHQF(V/L/I) (SEQ ID NO: 24)
   xiii) (A/G) (I/V) SC (L/I) KG (SEQ ID NO: 25)
   xiv)G(L/M)(L/G)E(M/I)(I/Q)(R/K/N)F(G/S/A) (SEQ ID NO: 28)

Am meisten bevorzugt meint Hefe G3PDH das Hefeproteins Gpd1p gemäß SEQ ID NO: 2, sowie funktionelle Äquivalente als auch funktionell äquivalente Teile der vorgenannten.

Funktionelle Äquivalente meint insbesondere natürliche oder künstliche Mutationen des Hefeproteins Gpd1p gemäß SEQ ID NO: 2 sowie homologe Polypeptide aus anderen Hefen, die die gleichen wesentlichen Eigenschaften einer Hefe G3PDH, entsprechend oben gegebener Definition, aufweisen. Mutationen umfassen Substitutionen, Additionen, Deletionen, Inversion oder Insertionen eines oder mehrerer Aminosäurereste. Insbesondere Bevorzugt sind die Polypeptide beschrieben durch SEQ ID NO: 4, 5, 7, 9, 11, 12, 14, 16, 38 oder 40.

Die zu den im Rahmen dieser Erfindung vorteilhaft einzusetzenden Hefe G3PDH können durch Datenbanksuche oder Durchmustern von Gen - oder cDNA-Bibliotheken - unter Verwendung der beispielhaft aufgeführten Hefe G3PDH-Sequenz gemäß SEQ ID NO: 2 oder der für dieses kodierenden Nukleinsäuresequenz gemäß SEQ ID NO: 1 als Suchsequenz bzw. Sonde - leicht aufgefunden werden.

Bevorzugt haben besagte funktionelle Äquivalente eine Homologie von mindestens 80 %, bevorzugt mindestens 90 % zu dem Protein mit der SEQ ID NO: 2.

Unter Homologie zwischen zwei Polypeptiden wird die Identität der Aminosäuresequenz über die gesamte Sequenzlänge verstanden, die durch Vergleich mit Hilfe des Programmalgorithmus GAP (Wisconsin Package Version 10.0, University of Wisconsin, Genetics Computer Group (GCG), Madison, USA) unter Einstellung folgender Parameter berechnet wird:
Gap Weight: 8 Length Weight: 2
Average Match: 2,912 Average Mismatch: -2,003

Beispielhaft wird unter einer Sequenz, die eine Homologie von mindestens 80 % auf Proteinbasis mit der Sequenz SEQ ID NO: 2 aufweist, eine Sequenz verstanden, die bei einem Vergleich mit der Sequenz SEQ ID NO: 2 nach obigem Programmalgorithmus mit obigem Parametersatz eine Homologie von mindestens 80 % aufweist. Funktionelle Äquivalente umfasst auch solche Proteine, die durch Nukleinsäuresequenzen kodiert werden, die eine Homologie von mindestens 60 %, besonders bevorzugt mindestens 70 %, besonders bevorzugt mindestens 80 %, am meisten bevorzugt mindestens 90 % zu der Nukleinsäuresequenz mit der SEQ ID NO: 1 haben.

Unter Homologie zwischen zwei Nukleinsäuresequenzen wird die Identität der beiden Nukleinsäuresequezen über die gesamte Sequenzlänge verstanden, die durch Vergleich mit Hilfe des Programmalgorithmus GAP (Wisconsin Package Version 10.0, University of Wisconsin, Genetics Computer Group (GCG), Madison, USA; Altschul et al. (1997) Nucleic Acids Res. 25:3389ff) unter Einstellung folgender Parameter berechnet wird:
Gap Weight: 50 Length Weight: 3
Average Match: 10 Average Mismatch:0

Beispielhaft wird unter einer Sequenz, die eine Homologie von mindestens 80 % auf Nukleinsäurebasis mit der Sequenz SEQ ID NO: 1 aufweist, eine Sequenz verstanden, die bei einem Vergleich mit der Sequenz SEQ ID NO: 1 nach obigem Programmalgorithmus mit obigem Parametersatz eine Homologie von mindestens 80 % aufweist.

Funktionelle Äquivalente umfasst auch solche Proteine, die durch Nukleinsäuresequenzen kodiert werden, die unter Standardbedingungen mit einer der durch SEQ ID NO: 1 beschriebenen Nukleinsäuresequenz, der zu dieser komplementären Nukleinsäuresequenz oder Teilen der vorgenannten hybridisieren und die wesentlichen Eigenschaften einer Hefe G3PDH aufweisen.

"Standardhybridisierungsbedingungen" ist breit zu verstehen, meint aber bevorzugt stringent Hybridisierungsbedingungen. Solche Hybridisierungsbedingungen sind unter anderem bei Sambrook J, Fritsch EF, Maniatis T et al., in Molecular Cloning (A Laboratory Manual), 2. Auflage, Cold Spring Harbor Laboratory Press, 1989, Seiten 9.31-9.57) oder in Current Protocols in Molecular Biology, John Wiley & Sons, N.Y. (1989), 6.3.1-6.3.6. beschrieben. Beispielhaft können die Bedingungen während des Waschschrittes ausgewählt sein aus dem Bereich von Bedingungen mit hoher Stringenz (mit ungefähr 0,2X SSC bei 50°C bevorzugt bei 65°C) (20X SSC: 0,3 M Natriumcitrat, 3 M NaCl, pH 7,0). Während der Hybridisierung können auch denaturierende Agenzien wie zum Beispiel Formamid oder SDS eingesetzt werden. In Gegenwart von 50 % Formamid wird die Hybridisierung bevorzugt bei 42°C ausgeführt.

Ein weiterer Gegenstand der Erfindung betrifft transgene Expressionskonstrukte, die eine transgene Expression einer Hefe G3PDH, in einem pflanzlichen Organismus oder einem Gewebe, Organ, Teil Zellen oder Vermehrungsmaterial des besagten pflanzlichen Organismus gewährleisten können.

Für die Hefe G3PDH gilt dabei die oben genannte Definition, besonders bevorzugt ist die transgene Expression einer Hefe G3PDH beschrieben durch die Sequenz mit der SEQ ID NO: 2.

In besagten transgenen Expressionskonstrukten steht ein Nukleinsäuremolekül kodierend für eine Hefe G3PDH bevorzugt in funktioneller Verknüpfung mit mindestens einem genetischen Kontrollelement (beispielsweise einem Promotor), das eine Expression in einem pflanzlichen Organismus oder einem Gewebe, Organ, Teil, Zelle oder Vermehrungsmaterial desselben gewährleistet.

Insbesondere bevorzugt sind transgene Expressionskassetten, wobei die Nukleinsäuresequenz kodierend für eine Glycerol-3-phosphat Dehydrogenase beschrieben ist durch
a) eine Sequenz mit der SEQ ID NO: 1, 3, 6, 8, 10, 13, 15, 37 oder 39 oder
b) eine Sequenz, die sich entsprechend dem degenerierten genetischen Code von einer Sequenz mit der SEQ ID NO: 1, 3, 6, 8, 10, 13,15, 37 oder 39 ableitet, oder
c) eine Sequenz, die eine Identität von mindestens 60 % zu der Sequenz mit der SEQ ID NO: 1 aufweist.

Unter einer funktionellen Verknüpfung versteht man zum Beispiel die sequentielle Anordnung eines Promotors mit der zu exprimierenden Nukleinsäuresequenz kodierend für eine Hefe G3PDH (zum Beispiel der Sequenz gemäß SEQ ID NO: 1) und ggf. weiterer regulativer Elemente wie zum Beispiel einem Terminator derart, dass jedes der regulativen Elemente seine Funktion bei der transgenen Expression der Nukleinsäuresequenz erfüllen kann. Dazu ist nicht unbedingt eine direkte Verknüpfung im chemischen Sinne erforderlich. Genetische Kontrollsequenzen, wie zum Beispiel Enhancer-Sequenzen, können ihre Funktion auch von weiter entfernten Positionen oder gar von anderen DNA-Molekülen aus auf die Zielsequenz ausüben. Bevorzugt sind Anordnungen, in denen die transgen zu exprimierende Nukleinsäuresequenz hinter der als Promoter fungierenden Sequenz positioniert wird, so dass beide Sequenzen kovalent miteinander verbunden sind. Bevorzugt ist dabei der Abstand zwischen der Promotorsequenz und der transgen zu exprimierende Nukleinsäuresequenz geringer als 200 Basenpaare, besonders bevorzugt kleiner als 100 Basenpaare, ganz besonders bevorzugt kleiner als 50 Basenpaare.

Die Herstellung einer funktionellen Verknüpfung als auch die Herstellung einer transgenen Expressionskassette kann mittels gängiger Rekombinations- und Klonierungstechniken realisiert werden, wie sie beispielsweise in Maniatis T, Fritsch EF und Sambrook J (1989) Molecular Cloning: A Laboratory Manual, Cold Spring Harbor Laboratory, Cold Spring Harbor (NY), in Silhavy TJ, Berman ML und Enquist LW (1984) Experiments with Gene Fusions, Cold Spring Harbor Laboratory, Cold Spring Harbor (NY), in Ausubel FM et al. (1987) Current Protocols in Molecular Biology, Greene Publishing Assoc. and Wiley Interscience und bei Gelvin et al. (1990) In: Plant Molecular Biology Manual beschrieben sind. Zwischen beide Sequenzen können aber auch weitere Sequenzen positioniert werden, die zum Beispiel die Funktion eines Linkers mit bestimmten Restriktionsenzymschnittstellen oder eines Signalpeptides haben. Auch kann die Insertion von Sequenzen zur Expression von Fusionsproteinen führen. Bevorzugt kann die Expressionskassette, bestehend aus einer Verknüpfung von Promoter und zu exprimierender Nukleinsäuresequenz, integriert in einem Vektor vorliegen und durch zum Beispiel Transformation in ein pflanzliches Genom insertiert werden.

Unter einer transgenen Expressionskassette sind aber auch solche Konstruktionen zu verstehen, bei denen die Nukleinsäuresequenz kodierend für eine Hefe G3PDH so hinter einen endogenen, pflanzlichen Promotor platziert wird, das dieser die Expression der Hefe G3PDH bewirkt.

Bevorzugt werden in den transgenen Expressionskassetten Promotoren eingesetzt, die in einem pflanzlichen Organismus oder einem Gewebe, Organ, Teil, Zelle oder Vermehrungsmaterial desselben funktionell sind. In pflanzlichen Organismen funktionelle Promotoren meint grundsätzlich jeden Promotor, der die Expression von Genen, insbesondere Fremdgenen, in Pflanzen oder Pflanzenteilen, -zellen, -geweben, -kulturen steuern kann. Dabei kann die Expression beispielsweise konstitutiv, induzierbar oder entwicklungsabhängig sein.

### Bevorzugt sind:

### a) Konstitutive Promotoren

"Konstitutive" Promotoren meint solche Promotoren, die eine Expression in zahlreichen, bevorzugt allen, Geweben über einen größeren Zeitraum der Pflanzenentwicklung, bevorzugt zu allen Zeitpunkten der Pflanzenentwicklung, gewährleisten (Benfey et al.(1989) EMBO J 8:2195-2202). Vorzugsweise verwendet man insbesondere einen pflanzlichen Promotor oder einen Promotor, der einem Pflanzenvirus entstammt. Insbesondere bevorzugt ist der Promotor des 35S-Transkriptes des CaMV Blumenkohlmosaikvirus (Franck et al. (1980) Cell 21:285-294; Odell et al. (1985) Nature 313:810-812; Shewmaker et al. (1985) Virology 140:281-288; Gardner et al. (1986) Plant Mol Biol 6:221- 228) oder der 19S CaMV Promotor (US 5,352,605; WO 84/02913; Benfey et al. (1989) EMBO J 8:2195-2202). Ein weiterer geeigneter konstitutiver Promotor ist der "Rubisco small subunit (SSU)"-Promotor (US 4,962,028), der LeguminB-Promotor (GenBank Acc.-Nr. X03677), der Promotor der Nopalinsynthase aus Agrobacterium, der TR-Doppelpromotor, der OCS (Octopin Synthase) Promotor aus Agrobacterium, der Ubiquitin Promotor (Holtorf S et al. (1995) Plant Mol Biol 29:637-649), den Ubiquitin 1 Promotor (Christensen et al. (1992) Plant Mol Biol 18:675-689; Bruce et al. (1989) Proc Natl Acad Sci USA 86:9692-9696), den Smas Promotor, den Cinnamylalkoholdehydrogenase-Promotor (US 5,683,439), die Promotoren der vakuolärer ATPase Untereinheiten, den Promotor des Nitrilase-1 Gens aus Arabidopsis thaliana (GenBank Acc.-No.: U38846, Nukleotide 3862 bis 5325 oder alternativ 5342) oder der Promotor eines prolinreichen Proteins aus Weizen (WO 91/13991), sowie weitere Promotoren von Genen, deren konstitutive Expression in Pflanzen dem Fachmann bekannt ist. Besonders bevorzugt sind der CaMV 35S-Promotor und der Nitrilase-1 Promotor aus Arabidopsis thaliana.

### b) Gewebespezifische Promotoren

Bevorzugt sind ferner Promotoren mit Spezifitäten für Samen, wie zum Beispiel der Promotor des Phaseolins (US 5,504,200; Bustos MM et al. (1989) Plant Cell 1(9):839-53), des 2S Albumingens (Joseffson LG et al. (1987) J Biol Chem 262:12196- 12201), des Legumins (Shirsat A et al. (1989) Mol Gen Genet 215(2):326-331), des USP (unknown seed protein; Bäumlein H et al. (1991) Mol Gen Genet 225(3):459-67), des Napin Gens (US 5,608,152; Stalberg K et al. (1996) L Planta 199:515-519), des Saccharosebindeproteins (WO 00/26388) oder der Legumin B4-Promotor (LeB4; Bäumlein H et al. (1991) Mol Gen Genet 225: 121-128; Baeumlein et al. (1992) Plant Journal 2(2):233-9; Fiedler U et al. (1995) Biotechnology (NY) 13(10):1090f), der Oleosin-Promoter aus Arabidopsis (WO 98/45461), der Bce4-Promoter aus Brassica (WO 91/13980).

Weitere geeignete samenspezifische Promotoren sind die der Gene kodierend für das "High Molecular Weight Glutenin" (HMWG), Gliadin, Verzweigungsenzym, ADP Glucose Pyrophosphatase (AGPase) oder die Stärkesynthase. Bevorzugt sind ferner Promotoren, die eine samenspezifische Expression in Monokotyledonen wie Mais, Gerste, Weizen, Roggen, Reis etc. erlauben. Vorteilhaft eingesetzt werden können der Promoter des lpt2 oder 1pt1-Gen (WO 95/15389, WO 95/23230) oder die Promotoren beschrieben in WO 99/16890 (Promotoren des Hordein-Gens, des Glutelin-Gens, des Oryzin-Gens, des Prolamin-Gens, des Gliadin-Gens, des Glutelin-Gens, des Zein-Gens, des Kasirin-Gens oder des Secalin-Gens).

### c) Chemisch induzierbare Promotoren

Die Expressionskassetten können auch einen chemisch induzierbaren Promotor enthalten (Übersichtsartikel: Gatz et al. (1997) Annu Rev Plant Physiol Plant Mol Biol 48:89-108), durch den die Expression des exogenen Gens in der Pflanze zu einem bestimmten Zeitpunkt gesteuert werden kann. Derartige Promotoren, wie z.B. der PRP1 Promotor (Ward et al. (1993) Plant Mol Biol 22:361-366), durch Salicylsäure induzierbarer Promotor (WO 95/19443), ein durch Benzolsulfonamidinduzierbarer Promotor (EP 0 388 186), ein durch Tetrazyklininduzierbarer Promotor (Gatz et al. (1992) Plant J 2:397-404), ein durch Abscisinsäure induzierbarer Promotor (EP 0 335 528) bzw. ein durch Ethanol- oder Cyclohexanoninduzierbarer Promotor (WO 93/21334) können ebenfalls verwendet werden. Ferner geeignet ist der Promotor des Glutathione-S-transferase Isoform II Gens (GST-II-27), der durch exogen applizierte Safener wie z.B. N,N-Diallyl-2,2-dichloroacetamid aktiviert werden kann (W0 93/01294) und in zahlreichen Geweben von sowohl Monokotyledonen als auch Dikotyledonen funktionell ist.

Besonders bevorzugt sind konstitutive sowie ganz besonders bevorzugt samenspezifische Promotoren, insbesondere der Napin - und der USP-Promotor.

Es können ferner weitere Promotoren funktionell mit der zu exprimierenden Nukleinsäuresequenz verknüpft sein, die eine Expression in weiteren Pflanzengeweben oder in anderen Organismen, wie zum Beispiel E.coli Bakterien ermöglichen. Als Pflanzen Promotoren kommen im Prinzip alle oben beschriebenen Promotoren in Frage.

Die in den erfindungsgemäßen transgenen Expressionskassetten oder transgenen Vektoren enthaltenen Nukleinsäuresequenzen können mit weiteren genetischen Kontrollsequenzen neben einem Promoter funktionell verknüpft sein. Der Begriff der genetischen Kontrollsequenzen ist breit zu verstehen und meint all solche Sequenzen, die einen Einfluss auf das Zustandekommen oder die Funktion der erfindungsgemäßen Expressionskassette haben. Genetische Kontrollsequenzen modifizieren zum Beispiel die Transkription und Translation in prokaryotischen oder eukaryotischen Organismen. Vorzugsweise umfassen die erfindungsgemäßen transgenen Expressionskassetten 5'-stromaufwärts von der jeweiligen transgen zu exprimierenden Nukleinsäuresequenz einen pflanzenspezifischen Promoter und 3'-stromabwärts eine Terminatorsequenz als zusätzliche genetische Kontrollsequenz, sowie gegebenenfalls weitere übliche regulative Elemente, und zwar jeweils funktionell verknüpft mit der transgen zu exprimierenden Nukleinsäuresequenz.

Genetische Kontrollsequenzen umfassen auch weitere Promotoren, Promotorelemente oder Minimalpromotoren, die die expressionsteuernden Eigenschaften modifizieren können. So kann durch genetische Kontrollsequenzen zum Beispiel die gewebespezifische Expression zusätzlich abhängig von bestimmten Stressfaktoren erfolgen. Entsprechende Elemente sind zum Beispiel für Wasserstress, Abscisinsäure (Lam E und Chua NH, J Biol Chem 1991; 266(26): 17131 -17135) und Hitzestress (Schoffl F et al. (1989) Mol Gen Genetics 217(2-3):246-53) beschrieben.

Weitere vorteilhafte Kontrollsequenzen sind beispielsweise in den gram-positiven Promotoren amy und SPO2, in den Hefe- oder Pilzpromotoren ADC1, MFa, AC, P-60, CYC1, GAPDH, TEF, rp28, ADH.

Prinzipiell können alle natürlichen Promotoren mit ihren Regulationssequenzen wie die oben genannten für das erfindungsgemäße Verfahren verwendet werden. Darüberhinaus können auch synthetische Promotoren vorteilhaft verwendet werden.

Genetische Kontrollsequenzen umfassen ferner auch die 5'-untranslatierte Regionen, Introns oder nichtkodierende 3'-Region von Genen wie beipielsweise das Actin-1 Intron, oder die Adh1-S Introns 1, 2 und 6 (allgemein: The Maize Handbook, Chapter 116, Freeling and Walbot, Eds., Springer, New York (1994)). Es ist gezeigt worden, dass diese eine signifikante Funktionen bei der Regulation der Genexpression spielen können. So wurde gezeigt, dass 5'-untranslatierte Sequenzen die transiente Expression heterologer Gene verstärken können. Beispielhaft für Translationsverstärker sei die 5'-Leadersequenz aus dem Tabak-Mosaik-Virus zu nennen (Gallie et al. (1987) Nucl Acids Res 15:8693-8711) und dergleichen. Sie können ferner die Gewebsspezifität fördern (Rouster J et al. (1998) Plant J 15:435-440).

Die transgene Expressionskassette kann vorteilhafterweise eine oder mehrere sogenannte "enhancer Sequenzen" funktionell verknüpft mit dem Promoter enthalten, die eine erhöhte transgene Expression der Nukleinsäuresequenz ermöglichen. Auch am 3'-Ende der transgen zu exprimierenden Nukleinsäuresequenzen können zusätzliche vorteilhafte Sequenzen inseriert werden, wie weitere regulatorische Elemente oder Terminatoren. Die transgen zu exprimierenden Nukleinsäuresequenzen können in einer oder mehreren Kopien im Genkonstrukt enthalten sein.

Als Kontrollsequenzen geeignete Polyadenylierungssignale sind pflanzliche Polyadenylierungssignale, vorzugsweise solche, die im wesentlichen T-DNA Polyadenylierungssignale aus *Agrobacterium tumefaciens,* insbesondere des Gens 3 der. T-DNA (Octopin Synthase) des Ti-Plasmids pTiACHS entsprechen (Gielen et al. (1984) EMBO J 3:835 ff) oder funktionelle Äquivalente davon. Beispiele für besonders geeignete Terminatorsequenzen sind der OCS (Octopin-Synthase)-Terminator und der NOS (Nopalin-Synthase)-Terminator.

Als Kontrollsequenzen sind weiterhin solche zu verstehen, die eine homologe Rekombination bzw. Insertion in das Genom eines Wirtsorganismus ermöglichen oder die Entfernung aus dem Genom erlauben. Bei der homologen Rekombination kann zum Beispiel die kodierende Sequenz eines bestimmten endogenen Gens gegen die für eine dsRNA kodierende Sequenz gezielt ausgetauscht werden. Methoden wie die cre/lox-Technologie erlauben eine gewebespezifische, unter Umständen induzierbare Entfernung der Expressionskassette aus dem Genom des Wirtsorganismus (Sauer B (1998) Methods. 14(4):381-92). Hier werden bestimmte flankierende Sequenzen dem Zielgen angefügt (lox-Sequenzen), die später eine Entfernung mittels der cre-Rekombinase ermöglichen.

Eine transgene Expressionskassetten und die von ihr abgeleiteten transgenen Vektoren können weitere Funktionselemente enthalten. Der Begriff Funktionselement ist breit zu verstehen und meint all solche Elemente, die einen Einfluss auf Herstellung, Vermehrung oder Funktion der erfindungsgemäßen Expressionskassetten, Vektoren oder transgenen Organismen haben. Beispielhaft aber nicht einschränkend seien zu nennen:
a) Selektionsmarker, die eine Resistenz gegen einen Metabolismusinhibitor wie 2-Desoxyglucose-6-phosphat (WO 98/45456), Antibiotika oder Biozide, bevorzugt Herbizide, wie zum Beispiel Kanamycin, G 418, Bleomycin, Hygromycin, oder Phosphinotricin etc. verleihen. Besonders bevorzugte Selektionsmarker sind solche die eine Resistenz gegen Herbizide verleihen. Beispielhaft seien genannt: DNA Sequenzen, die für Phosphinothricinacetyltransferasen (PAT) kodieren und Glutaminsynthaseinhibitoren inaktivieren (bar und pat Gen), 5-Enolpyruvylshikimat-3-phosphatsynthasegene (EPSP Synthasegene), die eine Resistenz gegen Glyphosat® (N-(phosphonomethyl)glycin) verleihen, das für das Glyphosat® degradierende Enzyme kodierende gox Gen (Glyphosatoxidoreduktase), das deh Gen (kodierend für eine Dehalogenase, die Dalapon inaktiviert), Sulfonylurea- und Imidazolinon inaktivierende Acetolactatsynthasen sowie bxn Gene, die für Bromoxynil degradierende Nitrilaseenzyme kodieren, das aasa-Gen, das eine Resistenz gegen das Antibiotikum Apectinomycin verleih, das Streptomycinphosphotransferase (SPT) Gen, das eine Resistenz gegen Streptomycin gewährt, das Neomycinphosphotransferas (NPTII) Gen, das eine Resistenz gegen Kanamycin oder Geneticidin verleiht, das Hygromycinphosphotransferase (HPT) Gen, das eine Resistenz gegen Hygromycin vermittelt, das Acetolactatsynthas Gen (ALS), das eine Resistenz gegen Sulfonylharnstoff-Herbizide verleiht (z.B. mutierte ALS-Varianten mit z.B. der S4 und/oder Hra Mutation).
b) Reportergene, die für leicht quantifizierbare Proteine kodieren und über Eigenfarbe oder Enzymaktivität eine Bewertung der Transformationseffizienz oder des Expressionsortes oder -zeitpunktes gewährleisten. Ganz besonders bevorzugt sind dabei Reporter-Proteine (Schenborn E, Groskreutz D. Mol Biotechnol. 1999; 13(1):29-44) wie das "green fluorescence protein" (GFP) (Sheen et al.(1995) Plant Journal 8(5):777-784), die Chloramphenicoltransferase, eine Luziferase (Ow et al. (1986) Science 234:856-859), das Aequorin-Gen (Prasher et al. (1985) Biochem Biophys Res Commun 126(3):1259-1268), die β-Galactosidase, ganz besonders bevorzugt ist die β-Glucuronidase (Jefferson et al. (1987) EMBO J 6:3901-3907).
c) Replikationsursprünge, die eine Vermehrung der erfindungsgemäßen Expressionskassetten oder Vektoren in zum Beispiel E.coli gewährleisten. Beispielhaft seien genannt ORI (origin of DNA replication), der pBR322 ori oder der P15A ori (Sambrook et al.: Molecular Cloning. A Laboratory Manual, 2nd ed. Cold Spring Harbor Laboratory Press, Cold Spring Harbor, NY, 1989).
d) Elemente, die für eine Agrobakterium vermittelte Pflanzentransformation erforderlich sind, wie zum Beispiel die rechte oder linke Begrenzung der T-DNA oder die vir-Region.

Zur Selektion erfolgreich homolog rekombinierter oder auch transformierter Zellen ist es in der Regel erforderlich, einen selektionierbaren Marker zusätzlich einzuführen, der den erfolgreich rekombinierten Zellen eine Resistenz gegen ein Biozid (zum Beispiel ein Herbizid), einen Metabolismusinhibitor wie 2-Desoxyglucose-6-phosphat (WO 98/45456) oder ein Antibiotikum verleiht. Der Selektionsmarker erlaubt die Selektion der transformierten Zellen von untransformierten (McCormick et al. (1986) Plant Cell Reports 5:81-84).

Zusätzlich kann besagte transgene Expressionskassette oder Vektoren weitere Nukleinsäuresequenzen enthalten, die nicht für eine Hefe G3PDH kodieren, und deren transgene Expression zu einer zusätzlichen Steigerung der Fettsäure-Biosynthese führt (infolge proOIL). Diese zusätzlich transgen exprimierte proOIL Nukleinsäuresequenz kann beispielhaft aber nicht einschränkend ausgewählt sein aus Nukleinsäuren kodierend für Acetyl-CoA-Carboxylase (ACCase), Glycerol-3-Phosphat Acyltransferase (GPAT), Lysophosphatidat-Acyltransferase (LPAT), Diacylglycerol-Acyltransferase (DAGAT) und Phospholipid:diacylglycerol-acyltransferase (PDAT). Entsprechende Sequenzen sind dem Fachmann bekannt und aus Datenbanken oder entsprechenden cDNA-Banken der jeweiligen Pflanzen leicht zugänglich.

Die Einführung einer erfindungsgemäßen Expressionskassette in einen Organismus oder Zellen, Geweben, Organe, Teile bzw. Samen desselben (bevorzugt in Pflanzen bzw. pflanzliche Zellen, Gewebe, Organe, Teile oder Samen), kann vorteilhaft unter Verwendung von Vektoren realisiert werden, in denen die transgenen Expressionskassetten enthalten sind. Ein weiterer Gegenstand der Erfindung betrifft daher besagte transgene Vektoren, die eine transgene Expressionskassette für eine Hefe G3PDH umfassen.

Vektoren können beispielhaft Plasmide, Cosmide, Phagen, Viren oder auch Agrobacterien sein. Die Expressionskassette kann in den Vektor (bevorzugt ein Plasmidvektor) über eine geeignete Restriktionsschnittstelle eingeführt werden. Der entstandene Vektor wird zunächst in E.coli eingeführt. Korrekt transformierte E.coli werden selektioniert, gezüchtet und der rekombinante Vektor mit dem Fachmann geläufigen Methoden gewonnen. Restriktionsanalyse und Sequenzierung können dazu dienen, den Klonierungsschritt zu prüfen. Bevorzugt sind solche Vektoren, die eine stabile Integration der Expressionskassette in das Wirtsgenom ermöglichen.

Ein weiterer Gegenstand der Erfindung betrifft transgene pflanzliche Organismen oder Gewebe, Organ, Teil, Zellen oder Vermehrungsgut desselben, die eine Hefe G3PDH entsprechend oben gegebener Definition, eine transgene Expressionskassette für eine Hefe G3PDH oder einen transgenen Vektor umfassend eine solche Expressionskassette enthalten.

Die Herstellung eines entsprechenden transgenen pflanzlichen Organismus erfolgt z.B. mittels Transformation oder Transfektion mittels der entsprechenden Proteine oder Nukleinsäuren. Die Herstellung eines transformierten Organismus (bzw. einer transformierten Zelle oder Gewebes) erfordert, dass die entsprechende DNA (z.B. der Expressionsvektor), RNA oder Protein in die entsprechende Wirtszelle eingebracht wird. Für diesen Vorgang, der als Transformation (oder Transduktion bzw. Transfektion) bezeichnet wird, steht eine Vielzahl von Methoden zur Verfügung (Keown et al. (1990) Methods in Enzymology 185:527-537). So kann die DNA oder RNA beispielhaft direkt durch Mikroinjektion oder durch Bombardierung mit DNA-beschichteten Mikropartikeln eingeführt werden. Auch kann die Zelle chemisch, zum Beispiel mit Polyethylenglycol, permeabilisiert werden, so dass die DNA durch Diffusion in die Zelle gelangen kann. Die DNA kann auch durch Protoplastenfusion mit anderen DNA-enthaltenden Einheiten wie Minicells, Zellen, Lysosomen oder Liposomen erfolgen. Elektroporation ist eine weitere geeignete Methode zur Einführung von DNA, bei der die Zellen reversibel durch einen elektrischen Impuls permeabilisert werden. Möglich sind ferner das Quellen von Pflanzenteilen in DNA-Lösungen sowie Pollen- oder Pollenschlauchtransformation. Entsprechende Verfahren sind beschrieben (beispielsweise bei Bilang et al. (1991) Gene 100:247-250; Scheid et al. (1991) Mol Gen Genet 228:104-112; Guerche et al. (1987) Plant Science 52:111-116; Neuhause et al. (1987) Theor Appl Genet 75:30-36; Klein et al. (1987) Nature 327:70-73; Howell et al. (1980) Science 208:1265; Horsch et al. (1985) Science 227:1229-1231; DeBlock et al. (1989) Plant Physiology 91:694-701; Methods for Plant Molecular Biology (Weissbach and Weissbach, eds.) Academic Press Inc. (1988); and Methods in Plant Molecular Biology (Schuler and Zielinski, eds.) Academic Press Inc. (1989)).

Bei Pflanzen werden dabei die beschriebenen Methoden zur Transformation und Regeneration von Pflanzen aus Pflanzengeweben oder Pflanzenzellen zur transienten oder stabilen Transformation genutzt. Geeignete Methoden sind vor allem die Protoplastentransformation durch Polyethylenglykol-induzierte DNA-Aufnahme, das biolistische Verfahren mit der Genkanone, die sogenannte "particle bombardment" Methode, die Elektroporation, die Inkubation trockener Embryonen in DNA-haltiger Lösung und die Mikroinjektion.

Neben diesen "direkten" Transformationstechniken kann eine Transformation auch durch bakterielle Infektion mittels Agrobacterium tumefaciens oder Agrobacterium rhizogenes und Übertragung von entsprechenden rekombinanten Ti-Plasmiden oder Ri-Plasmiden durch oder durch Infektion mit transgenen Pflanzenviren durchgeführt werden. Die Agrobacterium-vermittelte Transformation ist am besten für dicotyledone Pflanzenzellen geeignet. Die Verfahren sind beispielsweise beschrieben bei Horsch RB et al. (1985) Science 225: 1229f.

Werden Agrobacterien verwendet, so ist die Expressionskassette in spezielle Plasmide zu integrieren, entweder in einen Zwischenvektor (englisch: shuttle or intermediate vector) oder einen binären Vektor. Wird ein Ti oder Ri Plasmid zur Transformation verwendet werden soll, ist zumindest die rechte Begrenzung, meistens jedoch die rechte und die linke Begrenzung der Ti oder Ri Plasmid T-DNA als flankierende Region mit der einzuführenden Expressionskassette verbunden.

Bevorzugt werden binäre Vektoren verwendet. Binäre Vektoren können sowohl in E.coli als auch in Agrobacterium replizieren. Sie enthalten in der Regel ein Selektionsmarkergen und einen Linker oder Polylinker flankiert von der rechten und linken T-DNA Begrenzungssequenz. Sie können direkt in Agrobacterium transformiert werden (Holsters et al. (1978) Mol Gen Genet 163:181-187). Das Selektionsmarkergen erlaubt eine Selektion transformierter Agrobakteria und ist zum Beispiel das nptII Gen, das eine Resistenz gegen Kanamycin verleiht. Das in diesem Fall als Wirtsorganismus fungierende Agrobacterium sollte bereits ein Plasmid mit der vir-Region enthalten. Diese ist für die Übertragung der T-DNA auf die pflanzliche Zelle erforderlich. Ein so transformiertes Agrobacterium kann zur Transformation pflanzlicher Zellen verwendet werden. Die Verwendung von T-DNA zur Transformation pflanzlicher Zellen ist intensiv untersucht und beschrieben (EP 120 516; Hoekema, In: The Binary Plant Vector System, Offsetdrukkerij Kanters B.V., Alblasserdam, Chapter V; An et al. (1985) EMBO J 4:277-287). Verschiedene binäre Vektoren sind bekannt und teilweise kommerziell erhältlich wie zum Beispiel pBI101.2 oder pBIN19 (Clontech Laboratories, Inc. USA).

Weitere zur Expression in Pflanzen geeignet Promotoren sind beschrieben (Rogers et al. (1987) Meth in Enzymol 153:253-277; Schardl et al. (1987) Gene 61:1-11; Berger et al. (1989) Proc Natl Acad Sci USA 86:8402-8406).

Direkte Transformationstechniken eignen sich für jeden Organismus und Zelltyp. Im Falle von Injektion oder Elektroporation von DNA bzw. RNA in pflanzliche Zellen sind keine besonderen Anforderungen an das verwendete Plasmid gestellt. Einfache Plasmide wie die der pUC-Reihe können verwendet werden. Sollen vollständige Pflanzen aus den transformierten Zellen regeneriert werden, so ist er erforderlich, das sich auf dem Plasmid ein zusätzliches selektionierbares Markergen befindet.

Stabil transformierte Zellen d.h. solche, die die eingeführte DNA integriert in die DNA der Wirtszelle enthalten, können von untransformierten selektioniert werden, wenn ein selektionierbarer Marker Bestandteil der eingeführten DNA ist. Als Marker kann beispielhaft jedes Gen fungieren, dass eine Resistenz gegen Antibiotika oder Herbizide (wie Kanamycin, G 418, Bleomycin, Hygromycin oder Phosphinotricin etc.) zu verleihen vermag (s.o.). Transformierte Zellen, die ein solches Markergen exprimieren, sind in der Lage, in der Gegenwart von Konzentrationen eines entsprechenden Antibiotikums oder Herbizides zu überleben, die einen untransformierten Wildtyp abtöten. Beispiel sind oben genannt und umfassen bevorzugt das bar Gen, dass Resistenz gegen das Herbizid Phosphinotricin verleiht (Rathore KS et al. (1993) Plant Mol Biol 21(5):871-884), das nptII Gen, dass Resistenz gegen Kanamycin verleiht, das hpt Gen, das Resistenz gegen Hygromycin verleiht, oder das EPSP-Gen, das Resistenz gegen das Herbizid Glyphosat verleiht. Der Selektionsmarker erlaubt die Selektion von transformierten Zellen von untransformierten (McCormick et al. (1986) Plant Cell Reports 5:81-84). Die erhaltenen Pflanzen können in üblicher Weise gezüchtet und gekreuzt werden. Zwei oder mehr Generationen sollten kultiviert werden, um sicherzustellen, dass die genomische Integration stabil und vererblich ist.

Die oben genannten Verfahren sind beispielsweise beschrieben in Jenes B et al. (1993) Techniques for Gene Transfer, in: Transgenic Plants, Vol. 1, Engineering and Utilization, herausgegeben von SD Kung und R Wu, Academic Press, S.128-143 sowie in Potrykus (1991) Annu Rev Plant Physiol Plant Molec Biol 42:205-225). Vorzugsweise wird das zu exprimierende Konstrukt in einen Vektor kloniert, der geeignet ist, Agrobacterium tumefaciens zu transformieren, beispielsweise pBin19 (Bevan et al. (1984) Nucl Acids Res 12:8711f).

Sobald eine transformierte Pflanzenzelle hergestellt wurde, kann eine vollständige Pflanze unter Verwendung von dem Fachmann bekannten Verfahren erhalten werden. Hierbei geht man beispielhaft von Kalluskulturen aus. Aus diesen noch undifferenzierten Zellmassen kann die Bildung von Spross und Wurzel in bekannter Weise induziert werden. Die erhaltenen Sprösslinge können ausgepflanzt und gezüchtet werden.

Dem Fachmann sind auch Verfahren bekannt, um aus Pflanzenzellen, Pflanzenteile und ganze Pflanzen zu regenerieren. Beispielsweise werden hierzu Verfahren beschrieben von Fennell et al. (1992) Plant Cell Rep. 11: 567-570; Stoeger et al (1995) Plant Cell Rep. 14:273-278; Jahne et al. (1994) Theor Appl Genet 89:525-533 verwendet.

"Transgen" meint bezüglich zum Beispiel einer Hefe G3PDH Nukleinsäuresequenz, einer Expressionskassette oder einem Vektor enthaltend besagte G3PDH Nukleinsäuresequenz oder einem Organismus transformiert mit besagter Nukleinsäuresequenz, Expressionskassette oder Vektor alle solche durch gentechnische Methoden zustandegekommene Konstruktionen, in denen sich entweder
a) die Nukleinsäuresequenz kodierend für eine Hefe G3PDH, oder
b) eine mit besagter Nukleinsäuresequenz unter a) funktionell verknüpfte genetische Kontrollsequenz, zum Beispiel ein in pflanzlichen Organismen funktioneller Promotor, oder
c) (a) und (b)
sich nicht in ihrer natürlichen, genetischen Umgebung befinden oder durch gentechnische Methoden modifiziert wurden, wobei die Modifikation beispielhaft eine Substitution, Addition, Deletion, Inversion oder Insertion eines oder mehrerer Nukleotidreste sein kann. Natürliche genetische Umgebung meint den natürlichen chromosomalen Locus in dem Herkunftsorganismus oder das Vorliegen in einer genomischen Bibliothek. Im Fall einer genomischen Bibliothek ist die natürliche, genetische Umgebung der Nukleinsäuresequenz bevorzugt zumindest noch teilweise erhalten. Die Umgebung flankiert die Nukleinsäuresequenz zumindest an einer Seite und hat eine Sequenzlänge von mindestens 50 bp, bevorzugt mindestens 500 bp, besonders bevorzugt mindestens 1000 bp, ganz besonders bevorzugt mindestens 5000 bp. Eine natürlich vorkommende Expressionskassette - beispielsweise die natürlich vorkommende Kombination des Promotors eines Gens kodierend für eine Hefe G3PDH mit dem entsprechenden Hefe G3PDH-Gen wird zu einer transgenen Expressionskassette, wenn diese durch nichtnatürliche, synthetische ("künstliche") Verfahren wie beispielsweise einer Mutagenisierung geändert wird. Entsprechende Verfahren sind beschrieben (US 5,565,350; WO 00/15815; siehe auch oben).

Als transgene Organismen bevorzugte Wirts- oder Ausgangsorganismen sind vor allem Pflanzen gemäß der oben genannten Definition. Eingeschlossen sind im Rahmen der Erfindung alle Gattungen und Arten höherer und niedrigerer Pflanzen des Pflanzenreiches, insbesondere Pflanzen, die für die Gewinnung von Ölen verwendet werden wie beispielsweise Raps, Sonnenblume, Sesam, Färberdistel, Ölbaum, Soja, Mais, Weizen und Nussarten. Eingeschlossen sind ferner die reifen Pflanzen, Saatgut, Sprossen und Keimlinge, sowie davon abgeleitete Teile, Vermehrungsgut und Kulturen, zum Beispiel Zellkulturen. Reife Pflanzen meint Pflanzen zu jedem beliebigen Entwicklungsstadium jenseits des Keimlings. Keimling meint eine junge, unreife Pflanze in einem frühen Entwicklungsstadium.

Die Herstellung der transgenen Organismen kann mit den oben beschriebenen Verfahren zur Transformation oder Transfektion von Organismen realisiert werden.

Ein weiterer Gegenstand der Erfindung betrifft die Verwendung der erfindungsgemäßen, transgenen Organismen und der von ihnen abgeleitete Zellen, Zellkulturen, Teile - wie zum Beispiel bei transgenen pflanzlichen Organismen Wurzeln, Blätter etc.-, und transgenes Vermehrungsgut wie Saaten oder Früchte, zur Herstellung von Nahrungs- oder Futtermitteln, Pharmazeutika oder Feinchemikalien, insbesondere von Ölen, Fetten, Fettsäuren oder Derivaten der vorgenannten.

### Sequenzen

1. SEQ ID NO: 1
   Nukleinsäuresequenz kodierend für Saccharomyces cerevisiae G3PDH (Gpd1p)
2. SEQ ID NO: 2
   Proteinsequenz kodierend für Saccharomyces cerevisiae G3PDH (Gpd1p)
3. SEQ ID NO: 3
   Nukleinsäuresequenz kodierend für Saccharomyces cerevisiae G3PDH (Gpd2p)
4. SEQ ID NO: 4
   Proteinsequenz kodierend für Saccharomyces cerevisiae G3PDH (Gpd2p)
5. SEQ ID NO: 5
   Proteinsequenz kodierend für Saccharomyces cerevisiae G3PDH (Gpd2p) mit zweitem, alternativem Start-Codon
6. SEQ ID NO: 6
   Nukleinsäuresequenz kodierend für Schizosaccharomyces pombe G3PDH
7. SEQ ID NO: 7
   Proteinsequenz kodierend für Schizosaccharomyces pombe G3PDHD
8. SEQ ID NO: 8
   Nukleinsäuresequenz kodierend für Schizosaccharomyces pombe G3PDH
9. SEQ ID NO: 9
   Proteinsequenz kodierend für Schizosaccharomyces pombe G3PDH
10. SEQ ID NO: 10
   Nukleinsäuresequenz kodierend für Yarrowinia lipolytica G3PDH
11. SEQ ID NO: 11
   Proteinsequenz kodierend für Yarrowinia lipolytica G3PDH
12. SEQ ID NO: 12
   Proteinsequenz kodierend für Yarrowinia lipolytica G3PDH, mit zweitem, alternativem Start-Codon
13. SEQ ID NO: 13
   Nukleinsäuresequenz kodierend für Zygosaccharomyces rouxii G3PDH
14. SEQ ID NO: 14
   Proteinsequenz kodierend für Zygosaccharomyces rouxii G3PDH
15. SEQ ID NO: 15
   Nukleinsäuresequenz kodierend für Zygosaccharomyces rouxii G3PDH
16. SEQ ID NO: 16
   Proteinsequenz kodierend für Zygosaccharomyces rouxii G3PDH
17. SEQ ID NO: 16
   Expressionsvektor auf Basis von pSUN-USP für S.cerevisiae G3PDH (Gpdlp; Insert von bp 1017 - 2190)
18. SEQ ID NO: 18 Oligonukleotidprimer ONP1
   5'-ACTAGTATGTCTGCTGCTGCTGATAG-3'
19. SEQ ID NO: 19 Oligonukleotidprime ONP2
   5'-CTCGAGATCTTCATGTAGATCTAATT-3'
20. SEQ ID NO: 20 Oligonukleotidprime ONP3
   5'-GCGGCCGCCATGTCTGCTGCTGCTGATAG-3'
21. SEQ ID NO: 21 Oligonukleotidprime ONP4
   5'-GCGGCCGCATCTTCATGTAGATCTAATT-3'
22-35: SEQ ID NP 22 bis 35: Sequenzmotive für Hefe G3PDHs mit Angabe von möglichen Sequenzvariationen. Die Variationen eines einzelnen Motives können jeweils einzeln aber auch in den unterschiedlichen Kombinationen miteinander vorkommen.
36. SEQ ID NO: 36
   Expressionsvektor pGPTV-gpd1 auf Basis von pGPTV-Napin für S.cerevisiae G3PDH (Gpdlp; Insert gdp1 von bp 11962-13137; nos-Terminator: 13154-13408; Napin-Promotor: 10807-11951).
37. SEQ ID NO: 37
   Nukleinsäuresequenz kodierend für Emericella nidulans G3PDH
38. SEQ ID NO: 38
   Aminosäuresequenz kodierend für Emericella nidulans G3PDH
39. SEQ ID NO: 39
   Nukleinsäuresequenz kodierend für Debaryomyces hansenii G3PDH (partiell)
40. SEQ ID NO: 40
   Aminosäuresequenz kodierend für Debaryomyces hansenii G3PDH (partiell)

### Abbildungen

Fig. 1: Ölgehalt in transgenen GPD1p-Linien
Messung des TAG-Gehalts in T2 Samen von transgenen Arabidopsis-Linien mit dem Gpd1p-Gen aus Saccharomyces cerevisiae (G2 bis G30). Zum Vergleich ist der Gehalt in entsprechenden untransformierten (Wildtyp-Pflanzen; W1 bis W10) bestimmt worden. Es wurden 8 Arabidopsis-Linien mit einem signifikant gesteigerten Ölgehalt identifiziert. Die angegebenen Fehlerabweichungen ergeben sich aus jeweils 3 unabhängigen Messungen.

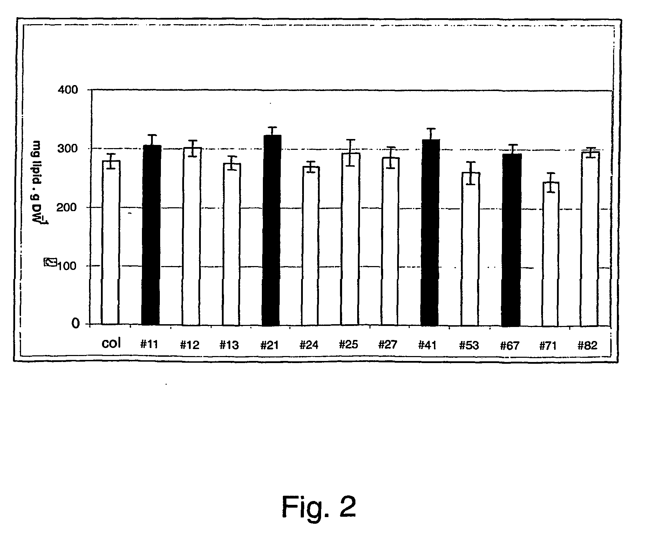

Fig. 2: Bestimmung des Ölgehalts in Samen der T3 Generation.
Wiedergegeben ist der Ölgehalt (in mg Lipid pro g Trockgewicht (DW)) einzelner Arabidopsis-Linien. Jede Säule stellt den Mittelwert aus 6 individuellen Pflanzen pro unabhängiger Linie dar. Von jeder Pflanze wurde eine dreifach Bestimmung durchgeführt. Die Fehlerbalken bezeichnen die Standardabweichung über alle Werte. Die Kontrollpflanzen sind mit 'col' bezeichnet. Die Einzelwerte sind numerisch zusätzlich in folgender Wertetabelle wiedergegeben (die Kontrolle wurde auf 100% Ölgehalt gesetzt):

| Linien | Ölgehalt (mg/g) | STD | Rel. Anstieg in % |
|---|---|---|---|
| col | 278,1 | 12,2 | 100 |
| #11 | 304,6 | 18,3 | 110 |
| #12 | 301,4 | 19,0 | 108 |
| #13 | 275,2 | 89,7 | 99 |
| #21 | 323,2 | 77,0 | 116 |
| #24 | 268,9 | 15,1 | 97 |
| #25 | 293,6 | 23,0 | 106 |
| #27 | 285,6 | 18,4 | 103 |
| #41 | 316,1 | 19,1 | 114 |
| #53 | 260,3 | 16,4 | 94 |
| #67 | 292,0 | 13,8 | 105 |
| #71 | 244,1 | 11,6 | 88 |
| #82 | 295,6 | 16,8 | 106 |

Linien mit einem statistisch signifikant erhöhtem Lipidgehalt (Linien #11, #21, #41 und #67) sind mit schwarzen Balken präsentiert.

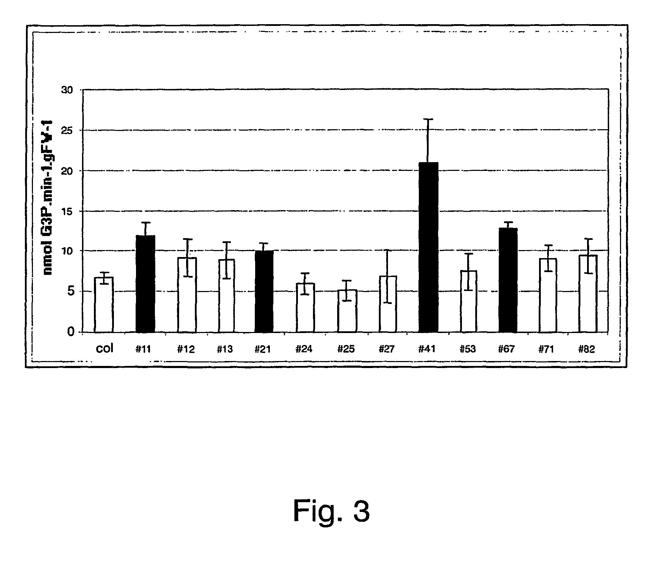

Fig. 3: Bestimmung der Aktivität von G3PDH in der Kontrolle (,col') und den gdp1 transformierten Pflanzen.
Die G3PDG Aktivität der einzelne Linien wurde wie in Beispiel 8 beschrieben bestimmt und ist in nmol G3P pro Minute und g Feuchtgewicht (FW) wiedergegeben.

| | G3PDH Aktivität | STD |
|---|---|---|
| col | 6,68337432 | 0,71785229 |
| #11 | 11, 8958635 | 1,67941604 |
| #12 | 9,14226124 | 2,25411878 |
| #13 | 8,8210768 | 2,19519777 |
| #21 | 9,88435444 | 1,04798566 |
| #24 | 5,89378595 | 1,26005769 |
| #25 | 5,14179348 | 1,22845409 |
| #27 | 6,77303725 | 3,22220935 |
| #41 | 20,8325636 | 5,42018531 |
| #53 | 7,45794947 | 2,25573816 |
| #67 | 12,7670015 | 0,74678353 |
| #71 | 9,04748534 | 1,59829185 |
| #82 | 9,37260033 | 2,1356558 |

Linien mit einer statistisch signifikant erhöhten G3PDH-Aktivität (Linien #11, #21, #41 und #67) sind mit schwarzen Balken präsentiert. Es ist erkennbar, dass eine erhöhte G3PDG Aktivität mit einem erhöhten Lipidgehalt korreliert.

### Beispiele

### Allgemeine Methoden:

Alle Chemikalien, wenn nicht anders erwähnt, stammen von den Firmen Fluka (Buchs), Merck (Darmstadt), Roth (Karlsruhe), Serva (Heidelberg) und Sigma (Deisenhofen). Restriktionsenzyme, DNAmodifizierende Enzyme und Molekularbiologie-Kits wurden von den Firmen Amersham-Pharmacia (Freiburg), Biometra (Göttingen), Roche (Mannheim), New England Biolabs (Schwalbach), Novagen (Madison, Wisconsin, USA), Perkin-Elmer (Weiterstadt), Qiagen (Hilden), Stratagen (Amsterdam, Niederlande), Invitrogen (Karlsruhe) und Ambion (Cambridgeshire, United Kingdom). Die verwendeten Reagenzien wurden entsprechend der Angaben des Herstellers eingesetzt.

Die chemische Synthese von Oligonukleotiden kann beispielsweise, in bekannter Weise, nach der Phosphoamiditmethode (Voet, Voet, 2. Auflage, Wiley Press New York, Seite 896-897) erfolgen. Die im Rahmen der vorliegenden Erfindung durchgeführten Klonierungsschritte wie z.B. Restriktionsspaltungen, Agarosegelelektrophorese, Reinigung von DNA-Fragmenten, Transfer von Nukleinsäuren auf Nitrozellulose und Nylonmembranen, Verknüpfen von DNA-Fragmenten, Transformation von E. coli Zellen, Anzucht von Bakterien, Vermehrung von Phagen und Sequenzanalyse rekombinanter DNA werden wie bei Sambrook et al. (1989) Cold Spring Harbor Laboratory Press; ISBN 0-87969-309-6 beschrieben durchgeführt. Die Sequenzierung rekombinanter DNA-Moleküle erfolgt mit einem Laserfluoreszenz-DNA-Sequenzierer der Firma ABI nach der Methode von Sanger (Sanger et al. (1977) Proc Natl Acad Sci USA 74:5463-5467).

### Beispiel 1: Allgemeine Verfahren

Die Pflanze Arabidopsis thaliana repräsentiert ein Mitglied der höheren Pflanzen (Samenpflanzen). Diese Pflanze ist eng verwandt mit anderen Pflanzenarten aus der Familie der Cruciferen wie z.B. Brassica napus, aber auch mit anderen Pflanzenfamilien der Dikotyledonen. Aufgrund des hohen Grades an Homologie ihrer DNA-Sequenzen bzw. Polypeptidsequenzen kann Arabidopsis thaliana als Modellpflanze für andere Pflanzenarten eingesetzt werden.

### a) Anzucht von Arabidopsis Pflanzen

Die Pflanzen werden entweder auf Murashige-Skoog Medium mit 0,5 % Saccharose (Ogas et al. (1997) Science 277:91-94) oder auf Erde gezogen (Focks & Benning (1998) Plant Physiol 118:91-101). Um einheitliche Keimungs- und Blühzeiten zu erreichen, werden die Samen nach Ausplattieren bzw. Ausstreuen auf Erde zwei Tage bei 4°C stratifiziert. Nach der Blüte werden die Schoten markiert. Entsprechend der Markierungen werden dann Schoten mit einem Alter von 6 bis 20 Tagen nach der Blüte geerntet.

### Beispiel 2: Klonierung des Gpd1-Gens aus Hefe

Genomische DNA aus Saccharomyces cerevisiae Stamm S288C (Mat alpha SUC2 mal mel ga12 CUP1 flo1 flo8-1; Invitrogen, Karlsruhe, Deutschland) wurde nach folgendem Protokoll isoliert:

Eine 100 ml Kultur wurde bis zu einer optischen Dichte von 1,0 bei 30°C angezogen. Davon wurden 60 ml abzentrifugiert bei 3000 xg für 3 min. Das Pellet wurde in 6 ml doppelt-destilliertem H₂O resuspendiert und auf 1,5 ml Gefäße verteilt, abzentrifugiert und der Überstand verworfen. Die Pellets wurden mit 200 µl Lösung A, 200 µL Phenol/Chloroform (1:1) und 0,3 g Glaskugeln durch Vortexen resuspendiert und lysiert. Nach Zugabe von 200 µl TE-Puffer pH 8,0 wurde für 5 min zentrifugiert. Der Überstand wurde einer Ethanolfällung mit 1 ml Ethanol unterzogen. Das erhaltene Pellet nach Fällung wurde in 400 µL TE-Puffer pH 8,0 + 30 µg/mL RnaseA gelöst. Nach Inkubation für 5 min bei 37°C wurden 18 µL 3 M Natriumacetat-Lösung pH 4,8 und 1 mL Ethanol zugegeben und die präzipitierte DNA durch Zentrifugation pelletiert. Das DNA-Pellet wurde in 25 µL doppelt-destilliertem H₂O gelöst. Die Konzentration der genomischen DNA wurde durch deren Absorption bei 260 nm bestimmt.

### Lösung A:

2 Trition-X100
1 % SDS
0,1 M NaCl
0,01 M Tris-HCl pH 8,0
0,001 M EDTA

Für die Klonierung des Gpd1-Gens wurde die isolierte Hefe-DNA in einer PCR-Reaktion mit den Oligonukleotidprimern ONP1 und ONP2 eingesetzt.
ONP1: 5'-ACTAGTATGTCTGCTGCTGCTGATAG-3' (SEQ ID NO: 18)
ONP2: 5'-CTCGAGATCTTCATGTAGATCTAATT-3' (SEQ ID NO: 19)

Zusammensetzung des PCR-Ansatzes (50 µL):
5,00 µL 5 µg genomische Hefe-DNA
5,00 µL 10x Puffer (Advantage-Polymerase)+ 25 mM MgCl₂
5,00 µL 2 mM dNTP
1,25 µL je Primer (10 pmol/µL)
0,50 µL Advantage-Polymerase

Die Advantage-Polymerase von Clontech wurden eingesetzt.

### PCR-Programm:

Anfangsdenaturierung für 2 min bei 95°C, dann 35 Zyklen mit 45 sec 95°C, 45 sec 55°C und 2 min 72°C. Abschließende Extension von 5 min bei 72°C.

Das PCR-Produkte wurde in den pCR2.1-TOPO Vektor (Invitrogen) gemäß Herstellerangaben kloniert, resultierend in dem Vektor pCR2.1-gpd1 und die Sequenz wurde durch Sequenzierung überprüft.

Für die Klonierung in den Agrotransformationsvektor pGPTV wurden 0,5 µg des Vektors pCR2.1-gpd1 mit den Restriktionsenzym XhoI (New England Biolabs) für 2 Stunde inkubiert und anschließend 15 min mit Klenow-Fragment (New England Biolabs) inkubiert. Nach Inkubation für 2 Stunden mit SpeI wurden die DNA-Fragmente per Gelelektorphorese aufgetrennt. Das neben dem Vektor (3,9 kb) 1185 bp große Fragment der gpd1-Sequenz wurde aus dem Gel ausgeschnitten und mit dem "Gelpurification"-Kit von Qiagen nach Herstellerangaben aufgereinigt und mit 50 µL Elutionspuffer eluiert. 0,1 µg des Vektors pGPTV wurde zuerst für 1 Stunde mit dem Restriktionsenzym SacI verdaut, dann für 15 min mit Klenow-Fragment (New England Biolabs) inkubiert. 10 µL des Eluates des gpd1-Fragments und 10 ng des behandelten Vektors pGPTV wurden über Nacht bei 16°C ligiert (T4 Ligase von New England Biolabs). Die Ligationsprodukte werden dann in TOP10 Zellen (Stratagene) nach Herstellerangaben transformiert und entsprechend selektioniert, resultierend in dem Vektor pGPTV-gpd1. Positive Klone werden mit den Primern ONP1 und ONP2 durch PCR und Sequenzierung verifiziert.

Für die Herstellung des Vektors pSUN-USP-gpd1 wurde eine PCR vom Vektor pCR2.1-gpd1 mit den Primern ONP3 und ONP4 durchgeführt.
ONP3: 5'-GCGGCCGCCATGTCTGCTGCTGCTGATAG-3' (SEQ ID NO: 20)
ONP4: 5`-GCGGCCGCATCTTCATGTAGATCTAATT-3' (SEQ ID NO: 21)

Zusammensetzung des PCR-Ansatzes (50 µL):
5 ng DNA Plasmid pCR2.1-gpd1
5,00 µL 10x Puffer (Advantage-Polymerase)+ 25 mM MgCl₂
5,00 µL 2 mM dNTP
1,25 µL je Primer (10 pmol/µL)
0,50 µL Advantage-Polymerase

Die Advantage-Polymerase von Clontech wurden eingesetzt.

### PCR-Programm:

Anfangsdenaturierung für 2 min bei 95°C, dann 35 Zyklen mit 45 sec 95°C, 45 sec 55°C und 2 min 72°C. Abschließende Extension von 5 min bei 72°C.

Das 1190 bp große PCR-Produkt wurde mit dem Restriktionsenzym NotI für 24 Stunden verdaut. Der Vektor pSUN-USP wurde für 2 Stunden mit NotI verdaut, dann 15 min mit alkalischer Phosphatase (New England Biolabs) inkubiert. 100 ng des vorbehandelten gpd1-Fragments und 10 ng des behandelten Vektors pGPTV wurden über Nacht bei 16°C ligiert (T4 Ligase von New England Biolabs). Die Ligationsprodukte werden dann in TOP10 Zellen (Stratagene) nach Herstellerangaben transformiert und entsprechend selektioniert, resultierend in dem Vektor pSUN-USP-gpd1. Positive Klone werden mit den Primern ONP3 und ONP4 durch PCR und Sequenzierung verifiziert.

### Beispiel 3: Plasmide für die Pflanzentransformation

Zur Pflanzentransformation können binäre Vektoren, wie pBinAR verwendet werden (Höfgen und Willmitzer (1990) Plant Science 66: 221-230). Die Konstruktion der binären Vektoren kann durch Ligation der cDNA in Sense- oder Antisense-Orientierung in T-DNA erfolgen. 5' der cDNA aktiviert ein Pflanzenpromotor die Transkription der cDNA. Eine Polyadenylierungssequenz befindet sich 3' von der cDNA.

Die gewebespezifische Expression lässt sich unter Verwendung eines gewebespezifischen Promotors erzielen. Beispielsweise kann die samenspezifische Expression erreicht werden, indem der Napin - oder der LeB4- oder der USP-Promotor 5' der cDNA einkloniert wird. Auch jedes andere samenspezifische Promotorelement kann verwendet werden. Zur konstitutiven Expression in der ganzen Pflanzen lässt sich der CaMV-35S-Promotor verwenden.

Ein weiteres Beispiel für binäre Vektoren ist der Vektor pSUN-USP und pGPTV-Napin in welche das Fragment aus Beispiel 2 kloniert wurde. Der Vektor pSUN-USP enthält den USP-Promotor sowie den OCS Terminator. Der Vektor pGPTV-Napin enthält einen verkürzte Version des Napin-Promotors und den nos-Terminator.

Die Fragmente aus Beispiel 2 wurden in die multiple Klonierungsstelle des Vektors pSUN-USP bzw. pGPTV-Napin kloniert, um die samenspezifische Expression des gdp1 Genes der Suppressionskonstrukte zu ermöglichen. Das entsprechende Konstrukt pSUN-USP-gpd1 ist mit der SEQ ID NO: 17 beschrieben, das Konstrukt von G3PDH in pGPTV-Napin (pGPTV-gpd1) ist durch SEQ ID NO: 36 beschrieben.

### Beispiel 4: Transformation von Agrobacterium

Die Agrobacterium-vermittelte Pflanzentransformation kann zum Beispiel unter Verwendung der Agrobacterium tumefaciens-Stämme GV3101 (pMP90) (Koncz und Schell (1986) Mol Gen Genet 204: 383-396) oder LBA4404 (Clontech) durchgeführt werden. Die Transformation kann durch Standard-Transformationstechniken durchgeführt werden (Deblaere et al. (1984) Nucl Acids Res 13:4777-4788).

### Beispiel 5: Pflanzentransformation

Die Agrobacterium-vermittelte Pflanzentransformation kann unter Verwendung von Standard-Transformations- und Regenerationstechniken durchgeführt werden (Gelvin SB, Schilperoort R, Plant Molecular Biology Manual, 2. Aufl., Dordrecht: Kluwer Academic Publ., 1995, in Sect., Ringbuc Zentrale Signatur: BT11-P ISBN 0-7923-2731-4; Glick BR, Thompson JE, Methods in Plant Molecular Biology and Biotechnology, Boca Raton: CRC Press, 1993, 360 S., ISBN 0-8493-5164-2).

Die Transformation mittels Agrobacterium von Arabidopsis thaliana wurde durch die Methode nach Bechthold et al., 1993 (C.R. Acad. Sci. Ser. III Sci. Vie., 316, 1194-1199) durchgeführt.

Beispielsweise kann Raps mittels Kotyledonen- oder Hypokotyltransformation transformiert werden (Moloney et al.(1989) Plant Cell Report 8:238-242; De Block et al.(1989) Plant Physiol 91: 694-701). Die Verwendung von Antibiotika für die Agrobacterium- und Pflanzenselektion hängt von dem für die Transformation verwendeten binären Vektor und Agrobacterium-Stamm ab. Die Rapsselektion wird gewöhnlich unter Verwendung von Kanamycin als selektierbarem Pflanzenmarker durchgeführt.

Der Agrobacterium-vermittelte Gentransfer in Lein (Linum usitatissimum) lässt sich unter Verwendung von beispielsweise einer von Mlynarova et al. (1994) Plant Cell Report 13:282-285 beschriebenen Technik durchführen. Die Transformation von Soja kann unter Verwendung von beispielsweise einer in EP-A-0 0424 047 (Pioneer Hi-Bred International) oder in EP-A-0 0397 687, US 5,376,543, US 5,169,770 (University Toledo) beschriebenen Technik durchgeführt werden.

Die Pflanzentransformation unter Verwendung von Teilchenbeschuss, Polyethylenglycol-vermittelter DNA-Aufnahme oder über die Siliziumcarbonatfaser-Technik ist beispielsweise beschrieben von Freeling und Walbot "The maize handbook" (1993) ISBN 3-540-97826-7, Springer Verlag New York).

### Beispiel 6: Untersuchung der Expression eines rekombinanten Genproduktes in einem transformierten Organismus

Die Aktivität eines rekombinanten Genproduktes im transformierten Wirtsorganismus wurde auf der Transkriptions- und/oder der Translationsebene gemessen.

Ein geeignetes Verfahren zur Bestimmung der Menge an Transkription des Gens (ein Hinweis auf die Menge an RNA, die für die Translation des Genproduktes zur Verfügung steht) ist die Durchführung eines Northern-Blots wie unten ausgeführt (als Bezugsstelle siehe Ausubel et al. (1988) Current Protocols in Molecular Biology, Wiley: New York, oder den oben erwähnten Beispielteil), wobei ein Primer, der so gestaltet ist, dass er an das Gen von Interesse bindet, mit einer nachweisbaren Markierung (gewöhnlich radioaktiv oder chemilumineszent) markiert wird, so dass, wenn die Gesamt-RNA einer Kultur des Organismus extrahiert, auf einem Gel aufgetrennt, auf eine stabile Matrix transferiert und mit dieser Sonde inkubiert wird, die Bindung und das Ausmaß der Bindung der Sonde das Vorliegen und auch die Menge der mRNA für dieses Gen anzeigt. Diese Information zeigt den Grad der Transkription des transformierten Gens an. Zelluläre Gesamt-RNA kann aus Zellen, Geweben oder Organen mit mehreren Verfahren, die alle im Fachgebiet bekannt sind, wie zum Beispiel das von Bormann, E.R., et al. (1992) Mol. Microbiol. 6:317-326 beschriebene, präpariert werden.

### Northern-Hybridisierung:

Für die RNA-Hybridisierung wurden 20 µg Gesamt-RNA oder 1 µg poly(A)⁺-RNA mittels Gelelektrophorese in Agarosegelen mit einer Stärke von 1,25 % unter Verwendung von Formaldehyd, wie beschrieben in Amasino (1986, Anal. Biochem. 152, 304) aufgetrennt, mittels Kapillaranziehung unter Verwendung von 10 x SSC auf positiv geladene Nylonmembranen (Hybond N+, Amersham, Braunschweig) übertragen, mittels UV-Licht immobilisiert und 3 Stunden bei 68°C unter Verwendung von Hybridisierungspuffer (10 % Dextransulfat Gew./Vol., 1 M NaCl, 1 % SDS, 100 mg Heringssperma-DNA) vorhybridisiert. Die Markierung der DNA-Sonde mit dem Highprime DNA labeling-Kit (Roche, Mannheim, Deutschland) erfolgte während der Vorhybridisierung unter Verwendung von alpha-³²P-dCTP (Amersham Pharmacia, Braunschweig, Deutschland). Die Hybridisierung wurde nach Zugabe der markierten DNA-Sonde im gleichen Puffer bei 68°C über Nacht durchgeführt. Die Waschschritte wurden zweimal für 15 min unter Verwendung von 2 X SSC und zweimal für 30 min unter Verwendung von 1 X SSC, 1 % SDS, bei 68°C durchgeführt. Die Exposition der verschlossenen Filter wurde bei -70°C für einen Zeitraum von 1 bis 14 T durchgeführt.

Zur Untersuchung des Vorliegens oder der relativen Menge an von dieser mRNA translatiertem Protein können Standardtechniken, wie ein Western-Blot, eingesetzt werden (siehe beispielsweise Ausubel et al. (1988) Current Protocols in Molecular Biology, Wiley: New York). Bei diesem Verfahren werden die zellulären Gesamt-Proteine extrahiert, mittels Gelelektrophorese aufgetrennt, auf eine Matrix, wie Nitrozellulose, übertragen und mit einer Sonde, wie einem Antikörper, der spezifisch an das gewünschte Protein bindet, inkubiert. Diese Sonde ist gewöhnlich mit einer chemilumineszenten oder kolorimetrischen Markierung versehen, die sich leicht nachweisen lässt. Das Vorliegen und die Menge der beobachteten Markierung zeigt das Vorliegen und die Menge des gewünschten, in der Zelle vorliegenden mutierten Proteins an.

### Beispiel 7: Analyse der Auswirkung der rekombinanten Proteine auf die Produktion des gewünschten Produktes

Die Auswirkung der genetischen Modifikation in Pflanzen, Pilzen, Algen, Ciliaten oder auf die Produktion einer gewünschten Verbindung (wie einer Fettsäure) kann bestimmt werden, indem die modifizierten Mikroorganismen oder die modifizierte Pflanze unter geeigneten Bedingungen (wie den vorstehend beschriebenen) gezüchtet werden und das Medium und/oder die zellulären Komponenten auf die erhöhte Produktion des gewünschten Produktes (d.h. von Lipiden oder einer Fettsäure) untersucht wird. Diese Analysetechniken sind dem Fachmann bekannt und umfassen Spektroskopie, Dünnschichtchromatographie, Färbeverfahren verschiedener Art, enzymatische und mikrobiologische Verfahren sowie analytische Chromatographie, wie Hochleistungs-Flüssigkeitschromatographie (siehe beispielsweise Ullman, Encyclopedia of Industrial Chemistry, Bd. A2, S. 89-90 und S. 443-613, VCH: Weinheim (1985); Fallon A et al. (1987) "Applications of HPLC in Biochemistry" in: Laboratory Techniques in Biochemistry and Molecular Biology, Bd. 17; Rehm et al. (1993) Biotechnology, Bd. 3, Kapitel III: "Product recovery and purification", S. 469-714, VCH: Weinheim; Belter PA et al. (1988) Bioseparations: downstream processing for Biotechnology, John Wiley and Sons; Kennedy JF und Cabral JMS (1992) Recovery processes for biological Materials, John Wiley and Sons; Shaeiwitz JA und Henry JD (1988) Biochemical Separations, in: Ullmann's Encyclopedia of Industrial Chemistry, Bd. B3; Kapitel 11, S. 1-27, VCH: Weinheim; und Dechow, F.J. (1989) Separation and purification techniques in biotechnology, Noyes Publications).

Neben den oben erwähnten Verfahren werden Pflanzenlipide aus Pflanzenmaterial wie von Cahoon et al. (1999) Proc. Natl. Acad. Sci. USA 96 (22):12935-12940, und Browse et al. (1986) Analytic Biochemistry 152:141-145, beschrieben extrahiert. Die qualitative und quantitative Lipid- oder Fettsäureanalyse ist beschrieben bei Christie, William W., Advances in Lipid Methodology, Ayr/Scotland: Oily Press (Oily Press Lipid Library; 2); Christie, William W., Gas Chromatography and Lipids. A Practical Guide - Ayr, Scotland: Oily Press, 1989, Repr. 1992, IX, 307 S. (Oily Press Lipid Library; 1); "Progress in Lipid Research, Oxford: Pergamon Press, 1 (1952) - 16 (1977) u.d.T.: Progress in the Chemistry of Fats and Other Lipids CODEN.

Zusätzlich zur Messung des Endproduktes der Fermentation ist es auch möglich, andere Komponenten der Stoffwechselwege zu analysieren, die zur Produktion der gewünschten Verbindung verwendet werden, wie Zwischen- und Nebenprodukte, um die Gesamteffizienz der Produktion der Verbindung zu bestimmen. Die Analyseverfahren umfassen Messungen der Nährstoffmengen im Medium (z.B. Zucker, Kohlenwasserstoffe, Stickstoffquellen, Phosphat und andere Ionen), Messungen der Biomassezusammensetzung und des Wachstums, Analyse der Produktion üblicher Metabolite von Biosynthesewegen und Messungen von Gasen, die während der Fermentation erzeugt werden. Standardverfahren für diese Messungen sind in Applied Microbial Physiology; A Practical Approach, P.M. Rhodes und P.F. Stanbury, Hrsgb., IRL Press, S. 103-129; 131-163 und 165-192 (ISBN: 0199635773) und darin angegebenen Literaturstellen beschrieben.

Ein Beispiel ist die Analyse von Fettsäuren (Abkürzungen: FAME, Fettsäuremethylester; GC-MS, Gas-Flüssigkeitschromatographie-Massenspektrometrie; TAG, Triacylglycerin; TLC, Dünnschichtchromatographie).

Der unzweideutige Nachweis für das Vorliegen von Fettsäureprodukten kann mittels Analyse rekombinanter Organismen nach Standard-Analyseverfahren erhalten werden: GC, GC-MS oder TLC, wie verschiedentlich beschrieben von Christie und den Literaturstellen darin (1997, in: Advances on Lipid Methodology, Vierte Aufl.: Christie, Oily Press, Dundee, 119-169; 1998, Gaschromatographie-Massenspektrometrie-Verfahren, Lipide 33:343-353).

Das zu analysierende Material kann durch Ultraschallbehandlung, Mahlen in der Glasmühle, flüssigen Stickstoff und Mahlen oder über andere anwendbare Verfahren aufgebrochen werden. Das Material muss nach dem Aufbrechen zentrifugiert werden. Das Sediment wird in Aqua dest. resuspendiert, 10 min bei 100°C erhitzt, auf Eis abgekühlt und erneut zentrifugiert, gefolgt von Extraktion in 0,5 M Schwefelsäure in Methanol mit 2 % Dimethoxypropan für 1 Std. bei 90°C, was zu hydrolysierten Öl - und Lipidverbindungen führt, die transmethylierte Lipide ergeben. Diese Fettsäuremethylester werden in Petrolether extrahiert und schließlich einer GC-Analyse unter Verwendung einer Kapillarsäule (Chrompack, WCOT Fused Silica, CP-Wax-52 CB, 25 mikrom, 0,32 mm) bei einem Temperaturgradienten zwischen 170°C und 240°C für 20 min und 5 min bei 240°C unterworfen. Die Identität der erhaltenen Fettsäuremethylester muss unter Verwendung von Standards, die aus kommerziellen Quellen erhältlich sind (d.h. Sigma), definiert werden.

Für die quantitative Öl-Analyse der mit dem Gpd1-Gen transformierten Arabidopsis Pflanzen wurde folgendes Protokoll angewendet:

Die Extraktion der Lipide aus Samen wird nach der Methode von Bligh & Dyer (1959) Can J Biochem Physiol 37:911 durchgeführt. Dazu werden 5 mg Arabidopsis Samen in 1,2 ml Qiagen-Microtubes (Qiagen, Hilden) auf einer Sartorius (Göttingen) Mikrowaage abgewogen. Das Samenmaterial wird mit 500 µL Chloroform/Methanol (2:1; enthält Mono-C17-glycerin von Sigma als internen Standard) in der Rätschmühle MM300 der Firma Retsch (Haan) homogenisiert und 20 min bei RT inkubiert. Nach Zugabe von 500 µL 50 mM Kaliumphosphatpuffer pH 7,5 erfolgt die Phasentrennung. Von der organischen Phase werden 50 µL abgenommen, mit 1500 µL Chloroform verdünnt und 5 µL auf die Kapillaren Chromarods SIII der Firma Iatroscan (SKS, Bechenheim) aufgetragen. Nach Auftrag der Proben werden diese für 15 min in einer Dünnschichtkammer, die gesättigt ist mit 6:2:2 Chloroform: Methanol: Toluol in einem ersten Schritt aufgetrennt. Nach Ablauf der Zeit werden die Kapillaren 4 min bei Raumtemperatur getrocknet und dann für 22 min in eine Dünnschichtkammer, die gesättigt ist mit 7:3 n-Hexan:Dieethylether gestellt. Nach einem weiteren Trocknungsschritt für 4 min bei Raumtemperatur werden die Proben in einem Iatroscan MK-5 (SKS, Bechenheim) entsprechend Fraser & Taggart, 1988 J. Chromatogr. 439:404 analysiert. Folgende Parameter wurden für die Messungen eingestellt: Slice width 50 msec, Treshold 20 mV, Noise 30, Skim ratio 0. Die Quantifizierung der Daten erfolgte anhand des internen Standards Mono-C17-glycerin (Sigma) sowie einer erstellten Eichkurve mit Tri-C17-glycerin (Sigma) mittels des Programms ChromStar (SKS, Beichenheim).

Für die quantitative Bestimmung der Ölgehalte wurden T2 Samen von mehreren, unabhängigen transgenen Linien mit den Konstrukten pSUN-USP-gpd1 bzw. pGPTV-gpd1 analysiert. Von den Samenpools jeder Linie wurden drei unabhängige Extraktionen durchgeführt und die Extrakte unabhängig gemessen. Aus den drei unabhängigen Messungen wurde der Mittelwert sowie die Standardabweichung berechnet.

Das Ergebnis der Messungen für die Linien mit dem Konstrukt pGPTV-gpd1 zeigte einen signifikant höheren Ölgehalt in mehreren (10) transgenen Linien (Fig. 1), verglichen mit den Messungen von 10 Wildtyp-Pflanzen. Für das Konstrukt pSUN-USP-gpd1 werden ähnliche Ölgehalte gemessen (nicht gezeigt).

Der durchschnittliche Ölgehalt der oben aufgeführten Linien beträgt 34,86 ± 1,56 %, während der Durchschnitt der Wildtyppflanzen bei 27,75 ± 2,64 % liegt. Dies entspricht einer Steigerung des Ölgehalts um absolut 7,1 % (relativ 25,6 %).

Zur Kontrolle der Vererbbarkeit des Effektes von gdp1 (Steigerung des Ölgehaltes) wurden T2 Samen von den Linien mit erhöhten Ölgehalten sowie von Linien mit unverändertem Ölgehalt angezogen. Jeweils 6 Pflanzen pro Linien wurden ausgebracht und die Samen auf Ölgehalt und Enzymaktivität überprüft. Die Bestimmung der Ölgehalte erfolgte entsprechend der oben ausgeführten Methodik. Die erhaltenen Werte sind in Fig. 2 dargestellt. Als Kontrollen dienen Col-0 und C24 Arabidopsis Ecotypen. C24 ist dabei ein Ecotyp, der sich durch einen höheren Ölgehalt als Col-0 auszeichnet. Es konnten Linien charakterisiert werden, die einen erhöhten Ölgehalt zu Col-0 zeigen. Damit konnte die Vererbbarkeit der Erhöhung des Ölgehaltes als Effekt der Expression des gdp1-Genes demonstriert werden.

### Beispiel 8: Bestimmung von Glycerol-3-phosphatdehydrogenaseaktivität

Ein weiteres Ziel war neben der Erhöhung des Ölgehalts auch die direkte Wirkung des Enzyms in den transgenen Pflanzen nachzuweisen. Zur Bestimmung der Glycerol-3-phosphatdehydrogenase-Aktivität wurde je Pflanze zwei Arabidopsis Samenschoten geerntet und nach Geigenberger und Stitt ((1993) Planta 189:329-339) extrahiert. Dazu wurden die Schoten in einem Mörser unter flüssigem Stickstoff zermalen und mit 200 µL 50 mM HEPES pH 7,4, 5 mM MgCl₂, 1 mM EDTA, 1 mM EGTA, 5 mM DTT, 0,1 % (w/w) Rinderserumalbumin, 2 mM Benzamidin, 2 mM Amino-n-capronsäure, 0.5 mM Phenylmethylsulfonyl, 0,1 % Trition X-100 und 10 % Glycerin (w/w) aufgenommen, 5 min zentrifugiert und der Überstand aliquotiert. Für die G3PDH Aktivität wurde die Produktion von G3P (Glycerin-3-phosphat) aus den Substraten DHAP (Dihydroxyacteonphosphat) und NADH gemessen. Dazu wurde die Oxidation von NADH bei 340 nm verfolgt.

Der Reaktionsmix zur Bestimmung der Aktivität enthielt 50 mM HEPES pH 7,4, 4 mM DHAP, 0,2 mM NADH und 10 µl des Extraktionsmixes in einem Endvolumen von 100 µL. Nach 30 min Inkubation bei Raumtemperatur wurde die Reaktion durch Erhitzen (20 min, 95°C) gestoppt. In der Kontrollreaktion wurde sofort durch Erhitzen inaktiviert.

Glycerol-3-phosphat "Cycling-Assay": 10 µl des Reaktionsgemisches wurden zu 45 µl einer Lösung enthaltend 200 mM Tricin, MgCl₂ 5 mM (pH 8,5) gegeben und erhitzt (20 min, 95°C), um verbleibendes DHAP zu zerstören. Der Überstand wurde in eine 96-Well Microplatte überführt, mit 45 µl eines Gemisches versetzt, welches 2 units G3Pox, 130 units Katalase, 0,4 unit G3PDH und 0,12 µmol NADH enthielt. Die Reaktion wurde bei 30°C durchgeführt und die resultierende Absorption bei 340 nm in einem Anthos htII Microplatten-Lesegerät verfolgt. Reaktionsgeschwindigkeiten wurden anhand der Absorptionsabnahme in (mOD*min-1) unter Verwendung der Biolise software berechnet (Gibon Y et al. (2002) Plant J 30(2):221-235).

Die transgenen Linien #11, #21, #41 und #67 zeigten dabei eine signifikant erhöhte Enzymaktivität im Vergleich zu Kontrollpflanzen (Fig. 3). Die Pflanzen mit erhöhtem Ölgehalt korrelieren mit den Pflanzen mit erhöhter Enzymaktivität. Damit konnte gezeigt werden, dass der erhöhte Ölgehalt auf den erhöhten Umsatz von DHAP zu G3P, der Vorstufe zur Ölsynthese, zurückzuführen ist.

## Patentansprüche

1. Verfahren zum Erhöhen des Gesamtölgehalt in einem pflanzlichen Organismus oder einem Gewebe, Organ, Teil, Zelle oder vermehrungsgut desselben, umfassend
a) transgene Expression einer Glycerol-3-phosphat Dehydrogenase aus einer Hefe in besagtem pflanzlichen Organismus oder einem Gewebe, Organ, Teil, Zelle oder Vermehrungsgut desselben und
b) Auswahl von pflanzlichen Organismen, bei denen - im Unterschied oder Vergleich zum Ausgangsorganismus - der Gesamtölgehalt in dem besagten pflanzlichen Organismus oder einem Gewebe, Organ, Teil, Zelle oder Vermehrungsgut desselben erhöht ist.

2. Verfahren nach Anspruch 1, wobei die Glycerol-3-phosphat Dehydrogenase aus einer Hefe stammt ausgewählt aus den Gattungen Cryptococcus, Torulopsis, Pityrosporum, Brettanomyces, Candida, Kloeckera, Trigonopsis, Trichosporon, Rhodotorul, Sporobolomyces, Bullera, Saccharomyces, Debaromyces, Lipomyces, Hansenula, Endomycopsis, Pichia und Hanseniaspora.

3. Verfahren nach einem der Ansprüche 1 oder 2, wobei die Glycerol-3-phosphat Dehydrogenase aus einer Hefe stammt ausgewählt aus den Arten Saccharomyces cerevisiae, Pichia pastoris, Hansenula polymorpha, Schizosaccharomyces pombe, Kluyveromyces lactis, Zygosaccharomyces rouxii, Yarrowia lipolitica, Emericella nidulans, Aspergillus nidulans, Debaryomyces hansenii und Torulaspora hansenii.

4. Verfahren nach einem der Ansprüche 1 bis 3, wobei die Glycerol-3-phosphat Dehydrogenase die Umsetzung von Dihydroxyacetonphosphat zu Glycerin-3-phosphat unter Verwendung von NADH als Cosubstrat bewirkt und eine Peptidsequenz aufweist, die mindestens ein Sequenzmotiv umfasst ausgewählt aus der Gruppe von Sequenzmotiven bestehend aus
i) GSGNWGT(A/T)IAK
ii) CG(V/A)LSGAN(L/I/V)AXE(V/I)A
iii) (L/V)FXRPYFXV

5. Verfahren nach einem der Ansprüche 1 bis 4, wobei die Glycerol-3-phosphat Dehydrogenase die Umsetzung von Dihydroxyacetonphosphat zu Glycerin-3-phosphat unter Verwendung von NADH als Cosubstrat bewirkt und eine Peptidsequenz aufweist, die mindestens ein Sequenzmotiv umfasst ausgewählt aus der Gruppe von Sequenzmotiven bestehend aus
iv) GSGNWGTTIAKV(V/I)AEN
v) NT(K/R)HQNVKYLP
vi) D(I/V)LVFN(I/V)PHQFL
vii) RA(I/V)SCLKGFE
viii) CGALSGANLA(P/T)EVA
ix) LFHRPYFHV
x) GLGEII(K/R)FG

6. Verfahren nach einem der Ansprüche 4 oder 5, wobei die Glycerol-3-phosphat Dehydrogenase zusätzlich mindestens ein Sequenzmotiv umfasst ausgewählt aus der Gruppe von Sequenzmotiven bestehend aus
xi) H(E/Q)NVKYL
xii) (D/N) (I/V) (L/I)V(F/W) (V/N) (L/I/V)PHQF(V/L/I)
xiii) (A/G)(I/V)SC(L/I)KG
xiv) G (L/M) (L/G) E (M/I) (I/Q) (R/K/N) F (G/S/A)

7. Verfahren nach einem der Ansprüche 1 bis 6,wobei die Glycerol-3-phosphat Dehydrogenase aus einer Hefe beschrieben ist durch
a) eine Sequenz mit der SEQ ID NO: 2, 4, 5, 7, 9, 11, 12, 14, 16, 38 oder 40, oder
b) ein funktionelles Äquivalent mit einer Identität von mindestens 80% auf Aminosäureebene zu der Sequenz SEQ iD NO:2

8. Verfahren nach einem der Ansprüche 1 bis 7, wobei die Pflanze eine Ölpflanze ist.

9. Verfahren nach einem der Ansprüche 1 bis 8, wobei der Gesamtölgehalt im Samen einer Pflanze erhöht wird.

10. Transgene Expressionskassette umfassend unter Kontrolle eines in einem pflanzlichen Organismus oder einem Gewebe, Organ, Teil oder Zelle desselben funktionellen Promotors eine Nukleinsäuresequenz kodierend für eine Glycerol-3-phosphat Dehydrogenase entsprechend
a) SEQ ID NO: 2, 4, 5, 7, 9, 11, 12, 14, 16, 38 oder 40 oder
b) einem funktionellen Äquivalent mit einer Identität auf Aminosäureebene von mindestens 80 % zu der Sequenz SEQ iD NO:2

11. Transgene Expressionskassette nach Anspruch 10, wobei die Nukleinsäuresequenz kodierend für eine Glycerol-3-phosphat Dehydrogenase beschrieben ist durch
a) eine Sequenz mit der SEQ ID NO: 1, 3, 6, 8, 10, 13, 15, 37 oder 39 oder
b) eine Sequenz, die sich entsprechend dem degenerierten genetischen Code von einer Sequenz mit der SEQ ID NO: 1, 3, 6, 8, 10, 13,15, 37 oder 39 ableitet, oder
c) eine Sequenz, die eine Identität von mindestens 60 % zu der Sequenz SEQ ID NO: 1 aufweist.

12. Transgene Expressionskassette nach einem der Ansprüche 10 oder 11, wobei der Promotor ein samenspezifischer Promotor ist.

13. Transgener Vektor enthaltend eine Expressionskassette nach einem der Ansprüche 10 bis 12.

14. Transgener pflanzlicher Organismus oder Gewebe, Organ, Teil, Zelle oder Vermehrungsgut desselben, enthaltend eine Glycerol-3-phosphat Dehydrogenase entsprechend
a) SEQ ID NO: 2 oder
b) einem funktionellen Äquivalent von a) mit einer Identität auf Aminosäureebene von mindestens 80%.

15. Transgener pflanzlicher Organismus nach Anspruch 14, wobei der pflanzliche Organismus ausgewählt ist aus der Gruppe der Ölpflanzen bestehend aus Borago officinalis, Brassica campestris, Brassica napus, Brassica rapa, Cannabis sativa, Curthamus tinctorius, Cocos nucifera, Crambe abyssinica, Cuphea Arten, Elaeis guinensis, Ekeis oleiferu, Glycine max, Gossypium hirsitum, Gossypium barbadense, Gossypium herbaceum, Helianthus annus, Linum usitatissimum, Oenethem biennis, Ozea europea, Oryza sativa, Ricinus communis, Sesamum indicum, Triticum Arten, Zea maize, Walnuss und Mandel.

16. Verwendung eines transgenen pflanzlichen Organismus oder Gewebe, Organ, Teil, Zelle oder Vermehrungsgut desselben nach einem der Ansprüche 14 oder 15 zur Herstellung von Ölen, Fetten, freien Fettsäuren oder Derivaten der vorgenannten.

## Claims

1. A method of increasing the total oil content in a plant organism
or a tissue, organ, part, cell or propagation material thereof, comprising
a) the transgenic expression of yeast glycerol-3-phosphate dehydrogenase in said plant organism or in a tissue, organ, part, cell or propagation material thereof, and
b) the selection of plant organisms in which - in contrast to or comparison with the starting organism - the total oil content in said plant organism or in a tissue, organ, part, cell or propagation material thereof is increased.

2. A method as claimed in claim 1, wherein the glycerol-3-phosphate dehydrogenase is derived from a yeast selected from the genera Cryptococcus, Torulopsis, Pityrosporum, Brettanomyces, Candida, Kloeckera, Trigonopsis, Trichosporon, Rhodotorul, Sporobolomyces, Bullera, Saccharomyces, Debaromyces, Lipomyces, Hansenula, Endomycopsis, Pichia and Hanseniaspora.

3. A method as claimed in claim 1 or 2, wherein the glycerol-3-phosphate dehydrogenase is derived from a yeast selected from the species Saccharomyces cerevisiae, Pichia pastoris, Hansenula polymorpha, Schizosaccharomyces pombe, Kluyveromyces lactis, Zygosaccharomyces rouxii, Yarrowia lipolitica, Emericella nidulans, Aspergillus nidulans, Debaryomyces hansenii and Torulaspora hansenii.

4. A method as claimed in any of claims 1 to 3, wherein the glycerol-3-phosphate dehydrogenase brings about the conversion of dihydroxyacetone phosphate to glycerol-3-phosphate using NADH as cosubstrate and has a peptide sequence comprising at least one sequence motif selected from the group of sequence motifs consisting of
i) GSGNNGT(A/T)IAK
ii) CG(V/A)LSGAN(L/I/V)AXE(V/I)A
iii) (L/V)FXRPYFXV

5. A method as claimed in any of claims 1 to 4, wherein the glycerol-3-phosphate dehydrogenase brings about the conversion of dihydroxyacetone phosphate to glycerol-3-phosphate using NADH as cosubstrate and has a peptide sequence encompassing at least one sequence motif selected from the group of sequence motifs consisting of
iv) GSGNWGTTIAKV(V/I)AEN
v) NT (K/R) HQNVKYLP
vi) D(I/V)LVFN(I/V)PHQFL
vii) RA(I/V)SCLKGFE
viii) CGALSGANLA(P/T)EVA
ix) LFHRPYFHV
x) GLGEII(K/R)FG

6. A method as claimed in claim 4 or 5, wherein the glycerol-3-phosphate dehydrogenase additionally encompasses at least one sequence motif selected from the group of sequence motifs consisting of
xi) H(E/Q)NVKYL
xii) (D/N) (I/V) (L/I)V(F/W) (V/N) (L/I/V) PHQF (V/L/I)
xiii) (A/G) (I/V) SC (L/I) KG
xiv) G(L/M)L/G)E(M/I)(I/Q)(R/K/N)F(G/S/A)

7. A method as claimed in any of claims 1 to 6, wherein the yeast glycerol-3-phosphate dehydrogenase is described by
a) a sequence with the SEQ ID NO: 2, 4, 5, 7, 9, 11, 12, 14, 16, 38 or 40, or
b) a functional equivalent with an identity of at least 80% at the amino acid level with the sequence SEQ ID NO: 2.

8. A method as claimed in any of claims 1 to 7, wherein the plant is an oil crop.

9. A method as claimed in any of claims 1 to 8, wherein the total oil content in the seed of a plant is increased.

10. A transgenic expression cassette comprising, under the control of a promoter which is functional in a plant organism or a tissue, organ, part or cell thereof, a nucleic acid sequence encoding a glycerol-3-phosphate dehydrogenase corresponding to
a) SEQ ID NO: 2, 4, 5, 7, 9, 11, 12, 14, 16, 38 or 40, or
b) a functional equivalent with an identity of at least 80% at the amino acid level with the sequence SEQ ID NO: 2.

11. A transgenic expression cassette as claimed in claim 10, wherein the nucleic acid sequence encoding a glycerol-3-phosphate dehydrogenase is described by
a) a sequence with the SEQ ID NO: 1, 3, 6, 8, 10, 13, 15, 37 or 39 or
b) a sequence derived from a sequence with the SEQ ID NO: 1, 3, 6, 8, 10, 13, 15, 37 or 39 in accordance with the degeneracy of the genetic code
c) a sequence which has at least 60% identity with the sequence SEQ ID NO: 1.

12. A transgenic expression cassette as claimed in claim 10 or 11, wherein the promoter is a seed-specific promotor.

13. A transgenic vector comprising an expression cassette as claimed in any of claims 10 to 12.

14. A transgenic plant organism or tissue, organ, part, cell or propagation material thereof, comprising a glycerol-3-phosphate dehydrogenase corresponding to
a) SEQ. ID NO: 1 or
b) a functional equivalent of a) with an identity of at least 80% at the amino acid level.

15. A transgenic plant organism as claimed in claim 14, wherein the plant organism is selected from the group of the oil crops consisting of Borvago officinalis, Brassica, campestris, Brassica napus, Brassica rapa, Cannabis sativa, Carthamus tinctorius, Cocos nucifera, Crambe abyssinica, Cuphea species, Elaeis guinensis, Elaeis oleifera, Glycine max, Gossypium hirsutum, Gossypium barbadense, Gossypium herbaceum, Helianthus annuus, Linum usitatissimum, Oenothera biennis, Olea europaea, Oryza sativa, Ricinus communis, Sesamum indicum, Triticum species, Zea mays, walnut and almond.

16. The use of a transgenic plant organism or tissue, organ, part, cell or propagation material thereof as claimed in claim 14 or 15 for the production of oils, fats, free fatty acids or derivatives of the above.

## Revendications

1. Procédé pour augmenter la teneur totale en huile dans un organisme végétal ou dans un tissu, un organe, une partie, une cellule ou du matériel de reproduction de celui-ci, comprenant
a) l'expression transgénique d'une glycérol-3-phosphate déshydrogénase d'une levure dans ledit organisme végétal ou dans un tissu, un organe, une partie, une cellule ou du matériel de reproduction de celui-ci et
b) la sélection des organismes végétaux dans lesquels - à la différence ou par rapport à l'organisme de départ - la teneur totale en huile dans ledit organisme végétal ou dans un tissu, un organe, une partie, une cellule ou du matériel de reproduction de celui-ci est augmentée.

2. Procédé selon la revendication 1, la glycérol-3-phosphate déshydrogénase provenant d'une levure choisie parmi les genres Cryptococcus, Torulopsis, Pityrosporum, Brettanomyces, Candida, Kloeckera, Trigonopsis, Trichosporon, Rhodotorul, Sporobolomyces, Bullera, Saccharomyces, Debaromyces, Lipomyces, Hansenula, Endomycopsis, Pichia et Hanseniaspora.

3. Procédé selon l'une quelconque des revendications 1 ou 2, la glycérol-3-phosphate déshydrogénase provenant d'une levure, choisie parmi les espèces Saccharomyces cerevisiae, Pichia pastoris, Hansenula polymorpha, Schizosaccharomyces pombe, Kluyveromyces lactis, Zygosaccharomyces rouxii, Yarrowia lipolitica, Emericella nidulans, Aspergillus nidulans, Debaryomyces hansenii et Torulaspora hansenii.

4. Procédé selon l'une quelconque des revendications 1 à 3, la glycérol-3-phosphate déshydrogénase provoquant la transformation de dihydroxyacétone-phosphate en glycérol-3-phosphate avec utilisation de NADH comme co-substrat et présentant une séquence peptidique qui comprend au moins un motif de séquence choisi dans le groupe des motifs de séquence constitué par
i) GSGNWGT(A/T)IAK
ii) CG(V/A)LSGAN(L/I/V)AXE(V/I)A
iii) (L/V)FXRPYFXV

5. Procédé selon l'une quelconque des revendications 1 à 4, la glycérol-3-phosphate déshydrogénase provoquant la transformation de dihydroxyacétone-phosphate en glycérol-3-phosphate avec utilisation de NADH comme co-substrat et présentant une séquence peptidique qui comprend au moins un motif de séquence choisi dans le groupe des motifs de séquence constitué par
iv) GSGNWGTTIAKV(V/I)AEN
v) NT(K/R)HQNVKYLP
vi) D(I/V)LVFN(I/V)PHQFL
vii) RA(I/V)SCLKGFE
viii) CGALSGANLA(P/T)EVA
ix) LFHRPYFHV
x) GLGEII(K/R)FG

6. Procédé selon l'une quelconque des revendications 4 ou 5, la glycérol-3-phosphate déshydrogénase comprenant en outre au moins un motif de séquence choisi dans le groupe de motifs de séquence constitué par
xi) H(E/Q)NVKYL
xii) (D/N)(I/V)(L/I)V(F/W) (V/N)(L/I/V)PHQF(V/L/I)
xiii) (A/G)(I/V)SC(L/I)KG
xiv) G(L/M)(L/G)E(M/I)(I/Q) (R/K/N)F(G/S/A)

7. Procédé selon l'une quelconque des revendications 1 à 6, la glycérol-3-phosphate déshydrogénase d'une levure étant décrite par
a) une des séquences SEQ ID NO : 2, 4, 5, 7, 9, 11, 12, 14, 16, 38 ou 40, ou
b) un équivalent fonctionnel présentant une identité d'au moins 80% sur le plan des acides aminés avec la séquence SEQ ID NO : 2.

8. Procédé selon l'une quelconque des revendications 1 à 7, le végétal étant une plante oléagineuse.

9. Procédé selon l'une quelconque des revendications 1 à 8, la teneur totale en huile dans les graines d'une plante étant augmentée.

10. Cassette d'expression transgénique comprenant, sous le contrôle d'un promoteur fonctionnel dans un organisme végétal ou dans un tissu, un organe, une partie ou une cellule de celui-ci, une séquence d'acide nucléique codant pour une glycérol-3-phosphate déshydrogénase, correspondant
a) aux séquences SEQ ID NO : 2, 4, 5, 7, 9, 11, 12, 14, 16, 38 ou 40 ou
b) à un équivalent fonctionnel présentant une identité d'au moins 80% sur le plan des acides aminés avec la séquence SEQ ID NO : 2.

11. Cassette d'expression transgénique selon la revendication 10, la séquence d'acide nucléique codant pour une glycérol-3-phosphate déshydrogénase étant décrite par
a) une des séquences SEQ ID NO : 1, 3, 6, 8, 10, 13, 15, 37 ou 39 ou
b) une séquence qui dérive, selon le code génétique dégénéré correspondant, d'une des séquences SEQ ID NO : 1, 3, 6, 8, 10, 13, 15, 37 ou 39 ou
c) une séquence présentant une identité d'au moins 60% avec la séquence SEQ ID NO : 1.

12. Cassette d'expression transgénique selon l'une quelconque des revendications 10 ou 11, le promoteur étant un promoteur spécifique des graines.

13. Vecteur transgénique contenant une cassette d'expression selon l'une quelconque des revendications 10 à 12.

14. Organisme végétal transgénique ou tissu, organe, partie, cellule ou matériel de reproduction de celui-ci, contenant une glycérol-3-phosphate déshydrogénase correspondant à
a) la séquence SEQ ID NO:2, ou
b) un équivalent fonctionnel de a) présentant une identité d'au moins 80% sur le plan des acides aminés.

15. Organisme végétal transgénique selon la revendication 14, l'organisme végétal étant choisi dans le groupe des plantes oléagineuses constitué par Borago officinalis, Brassica campestris, Brassica napus, Brassica rapa, Cannabis sativa, Curthamus tinctorius, Cocos nucifera, Crambe abyssinica, les espèces de Cuphea, Elaeis guinensis, Ekeis oleiferu, Glycine max, Gossypium hirsitum, Gossypium barbadense, Gossypium herbaceum, Helianthus annus, Linum usitatissimum, Oenéthem biennis, Ozea europea, Oryza sativa, Ricinus communis, Sesamum indicum, les espèces de Triticum, Zea maize, les noix et les amandes.

16. Utilisation d'un organisme végétal transgénique ou d'un tissu, d'un organe, d'une partie, d'une cellule ou de matériel de reproduction de celui-ci selon l'une quelconque des revendications 14 ou 15 pour la préparation d'huiles, de graisses, d'acides gras libres ou de dérivés des substances susmentionnées.
